# EUROPEAN PATENT SPECIFICATION

(11) **EP 3 390 642 B1**
(45) Date of publication and mention of the grant of the patent: **24.11.2021**
(21) Application number: 16876606.1
(22) Date of filing: 14.12.2016
(51) Int. Cl.: A61K 31/713, C12N 15/85, C12N 15/113, C12Q 1/68, A61P 27/02

(54) **COMPOSITIONS FOR TREATMENT OF RETINITIS PIGMENTOSA 13**
VERFAHREN ZUR BEHANDLUNG VON RETINITIS PIGMENTOSA 13
COMPOSITIONS ET PROCÉDÉS POUR LE TRAITEMENT DE RÉTINITE PIGMENTAIRE 13

(30) Priority: 14.12.2015 US 201562267261 P
(43) Date of publication of application: 24.10.2018
(73) Proprietor: COLD SPRING HARBOR LABORATORY, Cold Spring Harbor, NY 11724 (US); Stoke Therapeutics, Inc., Bedford, Massachusetts 01730 (US)
(72) Inventor: AZNAREZ, Isabel, Jamaica Plain, Massachusetts 02139 (US); NASH, Huw M., Lexington, Massachusetts 02421 (US); KRAINER, Adrian, East Northport, NY 11731 (US)
(74) Representative: Avidity IP
(86) International application number: PCT/US2016/066684
(87) International publication number: WO 2017/106364

(56) References cited:
- CN-A- 103 667 438
- LIU M M ET AL: "Alternative splicing and retinal degeneration", CLINICAL GENETICS, WILEY-BLACKWELL MUNKSGAARD, DK, vol. 84, no. 2, 1 August 2013 (2013-08-01) , pages 142-149, XP002746168, ISSN: 0009-9163, DOI: 10.1111/CGE.12181 [retrieved on 2013-06-05]
- A. B. MCKIE ET AL: "Mutations in the pre-mRNA splicing factor gene PRPC8 in autosomal dominant retinitis pigmentosa (RP13)", HUMAN MOLECULAR GENETICS, vol. 10, no. 15, 1 July 2001 (2001-07-01), pages 1555-1562, XP055589287, gb ISSN: 0964-6906, DOI: 10.1093/hmg/10.15.1555
- SUSAN F. MURRAY ET AL: "Allele-Specific Inhibition of Rhodopsin With an Antisense Oligonucleotide Slows Photoreceptor Cell Degeneration", INVESTIGATIVE OPTHALMOLOGY & VISUAL SCIENCE, vol. 56, no. 11, 5 October 2015 (2015-10-05), page 6362, XP055373895, US ISSN: 1552-5783, DOI: 10.1167/iovs.15-16400
- JANA KRALOVICOVA ET AL: "Optimal antisense target reducing INS intron 1 retention is adjacent to a parallel G quadruplex", NUCLEIC ACIDS RESEARCH, vol. 42, no. 12, 8 July 2014 (2014-07-08), pages 8161-8173, XP055211568, ISSN: 0305-1048, DOI: 10.1093/nar/gku507
- FRIEDMAN KENNETH J ET AL: "Correction of aberrant splicing of the cystic fibrosis transmembrane conductance regulator (CFTR) gene by antisense oligonucleotides", JOURNAL OF BIOLOGICAL CHEMISTRY, AMERICAN SOCIETY FOR BIOCHEMISTRY AND MOLECULAR BIOLOGY, US, vol. 274, no. 51, 17 December 1999 (1999-12-17), pages 36193-36199, XP002589149, ISSN: 0021-9258, DOI: 10.1074/JBC.274.51.36193
- S. CEREVISIAE: "OMIM Submission 607301. PRECURSOR mRNA-PROCESSING FACTOR 3", HOMOLOG OF ; PRPF3, 26 January 2015 (2015-01-26), XP055555042, Retrieved from the Internet: URL:http://omim.org/entry/607301 [retrieved on 2017-03-18]
- BRAVO-GIL et al.: "Improving the management of Inherited Retinal Dystrophies by targeted sequencing of a population-specific gene panel", Sci Rep, vol. 6, 1 April 2016 (2016-04-01), page 23910, XP055397372,
- GONZALEZ-SANTOS et al.: "Mutation in the splicing factor Hprp3p linked to retinitis pigmentosa impairs interactions within the U4/U6 snRNP complex", Hum Mol Genet, vol. 17, no. 2, 2008, pages 225-239, XP055397374,

### SEQUENCE LISTING

<110> COLD SPRING HARBOR LABORATORY STOKE THERAPEUTICS, INC.
<120> COMPOSITIONS AND METHODS FOR TREATMENT OF RETINITIS PIGMENTOSA 18 AND RETINITIS PIGMENTOSA 13
<130> 47991-709.601
<140>
   <141>
<150> 62/267,261
   <151> 2015-12-14
<160> 448
<170> PatentIn version 3.5
<210> 1
   <211> 31777
   <212> DNA
   <213> Homo sapiens
<400> 1
   gacagcggcg tggcggcggc ggtggcggta gcgacggcac gccgtagggc ggtcagaagg      60
   tttccggttc cggtgtaacg ttcgggctcc gtctcagggg ctgaagtttg tgaggtgagt     120
   agtggccccg ggcccacaga gggtaattcc agtccgcgcc actgtggccg gggactcagt     180
   ccaaggggag gaccgaagcg ggagggttca gcgggaagga gtgtgaaagg agtacctggt     240
   ttggtgggag aagaggttgg aatgagattg ttgagaggaa aagggcgggg gcgggaagga     300
   gaccgggttg ggagaccagc tctggggtga ggagtttagg tagaacaagg tagagtaaga     360
   gactggttgg agtgacggag agagtaaaaa tgaagattag cgggggagtg gggggctgca     420
   gagggaaact caggttgtag caaacaagag cacggagtaa ggaattcctt ctgaagactg     480
   gatttggatt ctgctgcctg ctcattgaga ggagagatca ttgtacctcg ctttccttcc     540
   gcacccccac tacccccgca cctcgcagtt attgttgagc atttgattag tgacaaggct     600
   aattaccgtt tgaaactttt ttatccattg gctgtttagt ttgcacgtca tcttatggag     660
   gtaacagtgg cacctcattt attgctttgg gctttcaaat tgcttatact ttctagccta     720
   tcattcaatt ctagtggaat ataagacccg ttaaggtgag gactcttgtt agttttgctc     780
   actgttttat gtatccaccg cacctagtac attgcttgac acttagtagt tactcaataa     840
   atatttattg aatgaattag gatgtaaata tggcagtact cattttgtaa gatggtaaac     900
   tgtggctcca aagaattact taacttgccc aagggcttgt aaatggtggg gctcagtgtc     960
   ccaactgttt ctgtgtgaac tcttgttttc actttctatg gcatctggat atgccgcttt    1020
   tgtgcaatac atgtgtaata tgtagaaatt ggtacgctgt atgtctactc attgtggggt    1080
   aatagaacca ccatagaatt actcacgtaa cttgtcactg aggcagaaaa tctttcagtc    1140
   ttagtttcat cacttcaatc tactagcttc ctttggatat accaaaagtg agtctatttg 1200
   gcagcaagtc ttatagactc tagtatagag gctaaactta ccaagaatgt agaagttaat 1260
   cagactggat tttttggaga aaagatgtct taaaacatct tgaaagatga ttcccagatc 1320
   tttgataagt ggcactgctg ggaatagctg agcttggaaa ttgaggtcag tcaatttatt 1380
   gactcccatt tagacacctt tttttttttt tccccgggac ggagtcttgc tctgtcgccc 1440
   aggctggagt gcagtggtgc gatctcggct cactgcaacc tccgccttcc atgttcaagg 1500
   gattctcctg tctcagcctc cagagtagct gggattacag gtgcaggtca ccacgcccag 1560
   ctaatatttg tatttttaat agggatgcgg tttcaccatg ttggccaggc tggtctcgaa 1620
   ctcctgacgt cagataatcc acccgccttg gcctcccaaa gtgttgggat tacaggcgtg 1680
   agccaccgca cctggctttt tttttttttt tttttttttt tttggagaca cagtttcact 1740
   ctgtcgctca ggctggagtg cacatgattt tggctcaccg caacctccgc ctcctgggtt 1800
   caagtgattt tcctgcctct gcctcataag tagctgggat tacaggtgcc caccacctca 1860
   cctggctaat ttttgtattt ttagtagaga caggtgcagt gggtcacgcc tgtaatccca 1920
   gaactttcgg aggtcaaggt gggcagatca cttgaggtaa ggcattacct ggccgatatg 1980
   gtgaaaccgt gtctttatta aaactacgaa attggccagg cgtggtggtg catgcctgta 2040
   gtcccagcta cttgggaggc tgaggcagga gaatcaagtg aacccaggag atggaggttg 2100
   cagtgaacca agattacgcc actgcactcc agcctgggcg acggagtgag actgtctcaa 2160
   aataaaaatg agggtattct tttctgagct gaaggatcat caagtcagct gatggagacc 2220
   ctgtagattg aacatgataa ttacctgtat gcagaattcc cactgggatg aatatgacca 2280
   cagagaaatg tgacagaaca ttcttttttt tttttttttt gagacggagt cttactctgt 2340
   tgcccaggct ggagtgcagt ggcgcgatct cggctcactg caacctctgc tgcctgggtt 2400
   caagtgattc tcttgtctca gcctcccgag tagctgggat tacaggcgcc tgccaccatg 2460
   cccagctaaa tttttttgta gtttttagta gagatggggt ttcaccatct tggccaggct 2520
   ggtcttgaac tcctgacctc gtgatccacc tgcctcagcc tcccaaagtg ctgggattac 2580
   aggcgtgagc caccgcgccc ggccagaaca ttcttaaaga tcatgagatt taatttaata 2640
   tctaagatga ctgacctgga taaaccctct tttacttgca taccttaaaa gaagggaatc 2700
   ctacataagt cagggttggg ttagggaatg cctgcctgga agtaggaaga atggaaaagc 2760
   tggcctctgg atgggctttt ttagttctgt tttagtgtta ctaggtttgc aagtccttgt 2820
   tgatatgaca ggatcaagaa agaagctaga actaatttct cagacttagc ttattcacag 2880
   agtttgttta ttccgcagat ttcattgatc aacttaagag ttagaattaa aaatcaaatg 2940
   ggatctcttt ctgctttgag atatggttct tggggaaaat atttgccttc aaatgagttg 3000
   gaaaaacttg tctagggatt gagaataggg ctggggaatg cattcctgat gtactaacca 3060
   tttccctgga tcactttagt cttttttttt tttttgagag agagtcttgc tttgtcgccc 3120
   aggctggagt gcagtggtgc gattttgact tgttgcaagc tctacctccc tggttattcc 3180
   cctgcctcag cctcccaagt agctgggatt acaggcacat gccaccacgc ccagctaatt 3240
   tttttgtatt tttagtagag gcagggtttc accatgttgg ccagactggt ctcgaactct 3300
   tgaccccagg cagtccatcc accttggcct cccaaagtgc tgggattaca ggcatgagcc 3360
   actgcaccca gcccacttta gtcttttctt attctctaac ttgtctcttt tttttttttt 3420
   tttaggtgta gtattgagtc ctgtttgagc tattgttctc tttttcctga aaaatggcac 3480
   tgtcaaagag ggagctggat gagctgaaac catggataga gaagacagtg aagagggtcc 3540
   tgggtttctc agagcctacg gtggtcacag cagcattgaa ctgtgtgggg aagggcatgg 3600
   acaagaagaa ggcagccggt atgtaccttt ctgcatcttt tatcttaaca tatagggtga 3660
   ccccaaacac tgctttgaac ttcctcaaaa ttctctctgt tgggaacatc taaaaaaatg 3720
   gcaggccata ttttattttc acctgtatta tagtgaaaat aggctaaata ataatttgct 3780
   ttttctctta attgcttatt ttttatgtta tttatgatag attttaaatc ttcgtttggc 3840
   taccatactg agtcacctaa gttagattta cttaggaagt atgcttaagt cagataggca 3900
   tattcagatt cagactgaac atctcaacat acaaaaatct ttatgaaata catgaaaaag 3960
   cttgaaggag gtagattttg ttaatggtag gcttctacaa cttgctattt gggaaaggtg 4020
   agggtggtta ttcatctcaa acttgatgtt caaggtaaac aggttgctct aactgcacca 4080
   cttcccacgc caaaaaatta taactatttt cacctcacag ggcaaggtca tttagtgtaa 4140
   gagagatggg aatcctacaa aaaacttaaa atttgtttca ctcctgggaa taaaattcca 4200
   gtgttggtac ctgttatagg cctagtatta gactgtgtac tcttaccttt ccaaccctac 4260
   caccgccttt cttcctgtca gatcatctga aaccttttct tgatgattct actctccgat 4320
   ttgtggacaa actgtttgag gctgtggagg aaggccgaag ctctaggcat tccaagtcta 4380
   gcagtgacag gagcagaaaa cgagagctaa aggtaggtta caatttactg tctaatgagc 4440
   tcaggactgt tttgaatggt aacagagttg ctgagcttac cagttcacac gttgatatcc 4500
   tctatttaga gcagcaaatg ttcaagagag gtacttagac attagagtag gagagatgaa 4560
   ttagtgtcat caccatctga actacctcta agttcatggg ttgaaatatc caaagaaata 4620
   attggcagct ttgtcttttt ttagataaca ggagttttga ttctcatgtt tctccatatt 4680
   tccttttttt ccttcttact gttacttata aactgattgc tctaagattt tatctttgat 4740
   ttatttgagg cctaataagt tgttttagaa tgtgactcca ctggtacctg agctgtacac 4800
   ctcattgttg cctctttgta gaagtttctc tgcaatgcac ttgtagatag aataccaaaa 4860
   aataactatt atttattata ttcttgagag cttaccattt gctctatggc atattgctgg 4920
   tcctctgctg ccatgagtga aacacttgga ggagttgtca gtaccccctt aactactaca 4980
   ctttttattc tgtagttgcc gactgatcct ggcctctttc agtattagca gtatcaagta 5040
   gcctattttt ctactcattt cccagtatct ctctttagga gaaaaaaaaa agttctgtga 5100
   ctcctatcaa accttccaaa gttcttctga taccttggct atcccattac ttttcctgag 5160
   ggaaaagagt gaataagtaa tgtctgcttg gtcccctggc caggctatgg aactaaagaa 5220
   aaaaagcaga gagatttgtg ccctttgtgt ttggagcatt gtagggtggt cagagaagga 5280
   gagatagtcc ttgccttaaa acaatttgta atttgtcagg cagtgtggca tgtgcctgta 5340
   atcctagcta cttgggaagc tgaggcaaga ggatctcttg agcccaggag ttacaattta 5400
   gaagaggcag gggtatcaca aaatatatat tcacgtatta taaaatctaa gtagcagttt 5460
   catgtaaatt ttagggttgg ttatagaatt tcagacctat gttgaatttt gttatattta 5520
   aggtggaatg attgatccac agtatacaca gatctgtaca gtgaagatta tgtcacgata 5580
   gggcatactt cttttttttt ttcctttttt ttttttcaag ggtgagtctc actctgtcgc 5640
   ccaggctgga gtacagtagc gcaatcttga ctcactgcaa cttccacctc ccaggttcaa 5700
   gtgattctcc tgcctcagcc tcctgagtag ctgggattac aggcacatgc caccatgcct 5760
   ggctaatttt tgtattttta gtagagacgg ggtttcatca tgttgatcag gttggtctcg 5820
   aactcctgac ctcaagatct gctcgcctcg gcctcccaaa gtgctggaat tacaggcatg 5880
   agccactgcg cctggccagt agggcatact tctgtatgag tgctgagggc atgctgtgaa 5940
   tgaagtgact acattgggtg ggattaataa aaatggcttc ttgaaggagg tgtctagata 6000
   gccttgggat acgggtccca tttttggtcc attcaggtcc ccaccccaca caatatatta 6060
   tgggagagtc ctttttcttg aactgtagga gttccaggaa gcgctcgccc ctggccgagg 6120
   atatgtcctt gcaaaacacg agccctccta gtgaagaggg gatgtcaccc aaagcctacc 6180
   accggtgtgt acatttgctt tttcccattg tcccttccag ttggccactc tcggtcctgt 6240
   taaattcacc ttgcaaatta gtcatcatat ttgagtttcc attgtttgat gaaaacaaag 6300
   tgttaatggg gattactgaa gaagaaagag aaagcccctg tcctggaaga gcttagactt 6360
   agttgggaaa actatactaa cattgaaata cttaacactt ggcaaacaga atatcaacaa 6420
   tgtaatttaa cctaatatga accaagctct tacacgctaa gagaatgtga aacaaaagtt 6480
   taatcagagt gggataggtt ataatgagga aaacttcttg gaggaggtga gttttaggca 6540
   ggatttggaa caggggttgt ggaaaagttg agagaaatca ttataaatgt gagcagtagc 6600
   atttgcaaag accttgagat gggaacattt ataagaacat gggaatgaaa tttgtttgac 6660
   tggattagga gaatgttgaa gttgggtgag atggagagtc tagtagaccc tgcttctggc 6720
   aggtggctat ttgcaaggtt gtgtcatatt cccctgggaa tagccagaaa atgctggtag 6780
   gggctaggat aattcttcct ttctccccac ggggagggat acagcttttc tttcatcttg 6840
   ggttccctta ggaggtgttt ggtgatgact ctgagatctc taaagaatca tcaggagtaa 6900
   agaagcgacg aataccccgt tttgaggagg tggaagaaga gccagaggtg atccctgggc 6960
   ctccatcaga gagccctggc atgctgacta agctccaggt tagtgattag ggactggaag 7020
   caaagtggtt ttgtgagttg aagaagcaaa aggtgcatgg gtctgtattt attgtggaat 7080
   tttttttttt tttttttttt tttttgaggt ggagttttgc tcttgtcacc caggctggaa 7140
   tacagtggcg cgatctcagc tcactgcaac ctccgcctcc cgggttcaag caaatctcct 7200
   gtctcagcct actgagtagc tgggattaga ggcacccacc accaagcctg gctaattttt 7260
   gtatttttag tagagatggg gtttcaccat gttggccagg ctggtcttga actcctgatg 7320
   tcaggtgatg cactcacctt ggcctctcag agtgctagaa ttacaggagc gagccactgc 7380
   gcccggctaa ttttttgtat ttcttttagt agagacaggg tttcaccatg ttgggcaggc 7440
   tggtcttcaa cttctgacct caggtcatct gcccaccttc gcctcccaga gtgctgggat 7500
   tataggcgtg agccagcgtg cctcgcctta ttgtgatctt gatttgaatt agtgtttagc 7560
   atttggggaa actttttttt tttttttttg agatagagtt tcactctgtt gcccatgctg 7620
   gagtgtagtg gcgtgatctc agctcacggc aacctccacc tcccaggttc aagcgattct 7680
   cctgcctaag cctccggagt agctgggact acatgcgaac accactacgc ctggctaatt 7740
   tttgtatttt tagtacagat ggtattttgt catgttggcc aggctgctct ggaactcctg 7800
   gcctcaagtg atctgccacc tcagccgccc aaatggctgg gattacaggt gtgagccacc 7860
   atgccaggcc agcatttggg aaaattttat ttcctgcttt atgttttagc gttctttgat 7920
   ttgaccagga gtattctagg taagggtatt gaaattgcca tttactgtgg caagcaaagt 7980
   gtgttatgta cattatttgc ctaattacct atgtcgttga gtcattgaga aaattaaatg 8040
   aaatgatcag aactcatctt aatgacagag gagactctcc aaacataatg caaagaagat 8100
   ataatccact gataaataat tttaagttga ctttattttt ttttaacagg aagtatttgt 8160
   gttttttcaa ttggcattgt taaataatac cacataagtg tttcttttac catttaatat 8220
   ttgagggggg taatagaaca tatacagaaa tatgggtggg tggatttgca tatatgtgat 8280
   atttacagaa ggaaagaaat ggattttttt tgtgtgtgtg tgtgagatgt tctctctgtc 8340
   actcaggctg gagcgcagtg gtgcgatctc ggttcactgc agcctctgcc tcccaggttc 8400
   aagtgattct cctgcctcag cctcctaagt agctgggatt ataggcatgt gccaccacgc 8460
   acggctaatt tttgtatttt tagtagagac agggtttcac catgttgacc aggctggtct 8520
   ggaactcctg acctcaagtg atctgcctac cttggcctcc caaattgctg ggattatagg 8580
   cgtgagccac cgtacctagc ctgtttttgt ttttcaggca aggtctcact tttttgtcca 8640
   ggctggagtt cagtggcgag atcttggctt actgcaacct ctgcctcccg ggcttgagtg 8700
   atcctcccac cttggcttcc caagtagctg ggactacagg tcaccatgcc tggctgattt 8760
   ttgtattttt tttaaagatt gagttccacc atgttgccca ggctggtctt gaactcctgg 8820
   gctgaaatga tcctcctgcc tcagcctccc aaagtgctgg acttacaggc gtgagctacc 8880
   acgcccagcc aattattttc ttttttgggg ggtgggggat ggagtcttgc tctgtcactt 8940
   aggctggagt acggtggcgc gttctcggct cactgcaacc tctgcctccc gggttcacgc 9000
   cattctcctg cctcagcctc ctgagtagct gggactacag gtatgtacca ccatgcccga 9060
   ctaatttttt gtatttttag tagagatggg gtttcaccat gttagccagg atggtcttga 9120
   tctcctgacc tcgtgatcca cccgcctcgg cctcccaaag tgctgggatt acaggcatga 9180
   gccaccgtgc caggcccaat gatgtttttt atggagaatg gggatggacg tattcacaac 9240
   atatcctttt gttatgtttc tttctttttt tttttttgag acggagtctc actctgtcgc 9300
   ccagagtgga gtgtaatggc gctatctcgg ctcactgcaa cgtccgcctc ccgggttcaa 9360
   gctattcttc tgcctcagtc ccttgagtag ctgggattac aggcacgcgc caccacgcct 9420
   agctaatttt tttgtatgtt tagtagagac ggggtttcac catgttggtc aggctggtct 9480
   cgaactcctg acctcgtgat ccgcccggct cggcctccca aagtgctggg attacagacg 9540
   tgagctgcca ggcccagcct gtaatttttc attttatttt attttatttt ttgagacgga 9600
   gtctcgctct tgcactgttg cctaggctgg agtgcaatgc cgtgatctca gctcactgca 9660
   acgtccgcct cctgggttcc agcagttctc ctgcctcagc ctcctgagta gctgagatta 9720
   cagaacgtgc atcaccatat ccagctaatt tttgtatttt tagtagagat ggggtttcac 9780
   cctgttagcc aggatggtct cgatctcctg acctcgtgat ccacctgcct cagcctccca 9840
   aagtattggg attacaggcg tgagccacca cgcccagccc tgtaattttt aattttttta 9900
   ttttctttct ttctttactt atttagagat ggagtctccc cctgttgtcc aggctggagt 9960
   gcagtggcat gatcttggct cactgtaacc tccaccttct ggattcaagc gcttctcctg 10020
   cctcagcctc ctgagtagct ggcattacag gcgcccacca ccacacccgg ctaatttttg 10080
   tatttttact agagacggtg tttcaccatg ttagccaggc tggtcttcag ctcctgacct 10140
   caagtgattc gcccacctca gcctctcaaa gtgctgggat tacaggcgtg agccaccatg 10200
   cctgaccctt ccgtaatttt tgtattttga atatatcacc tattcaagaa aaaatgttaa 10260
   agacaaatgt ttaaaaatat cttgaagcag tttaagaaca ggtctgtttt gcaaaatgta 10320
   agaggagttg tttggccata ccctcaaagc ttatcattct gaacttcttt aggacagaag 10380
   gggtttatag agactaggga gcaggtcttt gcaccatcgt ctcttgtttc ccaaactagt 10440
   agttcaagta ctaatatcat gattttttaa aaaaaattaa gtataggctg ggtgcggtgg 10500
   ctcacacctg taatcccagc actttgagag gccgaggtgg gcggatcacg aggtcaggag 10560
   atcgagacca tcctggctaa cagggtgaaa ccccatctct actaaaaata caaaaaatta 10620
   gccgggcgtg gtggtgggcg cctatagtcc cagctactcg ggatgctgag gcaggagaat 10680
   ggcatgaacc cgggaggcag agcttgcagt gagccaagat cgcaccactg cactccagcc 10740
   tgagtgacag agcgagactc catctcaaaa aaaaaaaaga aaaattaagt ataatatttt 10800
   gtgtaggttc tgttaggttt gcatgcatta tttaagtggt taacactaga cctataaggt 10860
   aggtactgtt ttcttccccc ttctagacac ttaggaggtt tagagactaa gttccttatc 10920
   caagataaca aaggcattaa gtggcagagt taagattcaa actcaggttt ctctgactcc 10980
   agagtctcaa tcatcacact atattactac ctaatatgct aatcttgctt taggaacatg 11040
   ggttgaaaaa ttccagtgct cacttatctt gactaaattt tttgcactgt ggtgcaggca 11100
   gaatgtggta tagtcattca aaacatttta agtgtctaga cctgagagcc ttgtgggttt 11160
   aaaaacctga atgggagaag taagaaggag aaattaacag tttttcaatt tttttcctgg 11220
   agagcagatc aaacagatga tggaggcagc aacacgacaa atcgaggaga ggaaaaaaca 11280
   gctgagcttc attagccccc ctacacctca ggtattgtgt ctagattact ctgaacagaa 11340
   taatctttgg cacagaattt cttgccatcc actcaaaatg agagactaga agaggtgact 11400
   acatgtttca cttcataatc atctgctaca gtctaccatg atgtgtgtgt atatctgtgt 11460
   gtatgtatgt gtgtttgtct gcctgtctta attcctctga tttctttttc tctatctcac 11520
   agccaaagac tccttcttcc tcccaaccag aacgacttcc tattggcaac actattcagc 11580
   cctcccaggc tgccactttc atgaatgatg ccattgagaa ggcaaggaaa gcagctgaac 11640
   tgcaagctcg aatccaagcc cagctggcac tgaagccagg actcatcggc aatgccaaca 11700
   tggtgggcct ggctaatctc catgccatgg gcattgctcc cccgtgagtg tctatttcat 11760
   ggaatacctt ttcatttctg aagtttgaga agcaataaat acctttgaat gttactgatc 11820
   tggctgggcg cagtgcctca ggcctgtaat cgtagcactt tgggaggctg aggtaggtgg 11880
   atcacctgag gtcaggagtc aggagttcga gaccagcctg gccaatatgg tgaaacccca 11940
   tctctactaa aaatacaaaa attagccagg cgtggtggtg catgcctgta atcccagcta 12000
   ctcgggaggc tgaggcagga gaatcgcttg aacacaggag gcagaggttg cagtgagctg 12060
   agatcgtgcc actgcgtggg tgacagaggg agactccggc tcaaagaaaa caacaaacaa 12120
   aatattactg atcctctttt tccacagaac ctagatcatt tctcacagtt tcatatttct 12180
   taggtgtgat tgattaatca gttgaatttt ttctcgtctt ccctgttgga gggtttatta 12240
   tcttcttatg atttatataa ctgagattaa ggttattcat ctaatgttgg agctaggagt 12300
   atagccaggt ttccttcctg gtttcaaact cagatttatc cattaaacca cattgtgtga 12360
   gactagatct gaggttcctc tgcaactggt caaagatgtt acattttttt tagcatctac 12420
   agttggtctg gcatcccatg agtgacataa gaattcaggg ataaggaaag ataggattat 12480
   aaattcatat aatttataaa ctcatttcat ttggttgtcc tgtatactga aatatccact 12540
   ttgatatttt taatcaagat actattggca gggcgcggtg gctcacacct gtaatcccag 12600
   cactttggga ggccgagact ggcagattgc ttgagctcag gagttcaaga ccagcctagg 12660
   caacatggca aaaccccatc tctctaaaat atacaaaaat tagccaggca tggtagagca 12720
   tgcgtgtagt tccagctact taggaggctg aggtgggaga atttctttgg gcctgggagg 12780
   ttgaggttgc aatgagctgt gattgtgcca ctgtacttta gcctgggcag cagagtgaga 12840
   ccatgtctca aaaaaaaaga tggtattgct cactgatgag attgttccct agaattattt 12900
   tattgtcctt tttgttgttg ttgttgttga gagggagtct cactctgtca cccaggctgg 12960
   agtgcagtga tctcggctcc ctgcaacctc tgcctcccag gttcagtagc tgggactaca 13020
   agtgcccgtc taaattttgt attttttgta gagatggggt ttcactgtgt tgccaaggct 13080
   ggtcttgaac tcctgggctc aagccatcca cccgcctcag cctcccaaag tgctgggatt 13140
   acagtgtgag ccaccacacc cgtccatcca aataactctt atagagaagg cactgcagcc 13200
   tcaacttcct gggctcaagt gatcctcctc aacctcctga gtaggtggga ccacaggtat 13260
   atgccaccgc acccagctaa ttttaaattt tttgttgaga tgcagtctcc ttgtgttggc 13320
   cagactggtc tttaactcct ggtctcaagt gatccctctg ccttggcctc ccaaaatgct 13380
   gtggttccag gcttgagcca ccacgcctgg ctttattttg ttttttgctt gccttaatgt 13440
   tccatacagg atttatttct ttgcgggcta tttttcagga aggtggagtt aaaagaccaa 13500
   acgaaaccta caccactgat cctggatgag caagggcgca ctgtagatgc aacaggcaag 13560
   gagattgagc tgacacaccg catgcctact ctgaaagcca atattcgtgc tgtgaagagg 13620
   gaacaattca agcaacaact aaaggaaaag ccatcagaag acatggaatc caataccttt 13680
   tttgaccccc gagtctccat tgccccttcc cagcgccaga gacgcacttt taaattccat 13740
   gacaagggca aatttgagaa gattgctcag cgattacgga caaaggtatc tggcttagag 13800
   tataacacag aatttggaaa aagttaagaa gctgacaaga aaacattgtt aaatctctgt 13860
   gttctaggaa attagataaa taggtcattt aaagcagcag tcctcaacct ttttggcacc 13920
   agggacttgt ttcatggaag ataatttttc ccctggagta aggatggttt cgtgatggtt 13980
   caagcacata gcatttattg tgtactttat tattattatt tttttcgaga tggagtgtcg 14040
   ctcttgttgc ccaggctgga gtgcaatggg gcgatctcgg cttgctgcaa cctttgcctc 14100
   ccaggttcaa gtgattctcc tgcctcagcc tcccgagtag ctgggatcac aggcatgtgc 14160
   caccacgcct ggctaatttt gtatttttgg tggagacggg gtttctccat gttggtcagg 14220
   ctggtctcga actcctgacc tcaggtgatc tgcccacttt gtcctcccaa agtgctggga 14280
   ttacaggcgt gagccaccgc ccccgccttt tttttttttt gggcggggag ggggatggag 14340
   tcttgcactg ttacctgggc tggagtgcag tggtacagtc tcagctcact gcaacctctg 14400
   cctcctgggt tcaagcaatt ctcctgcctc agcctcctga gtagctggga ctgcaggcat 14460
   gcgccgccat gcctgacgaa ttttttgtat ttttagtaga gacagggttt cactatgttg 14520
   gcccggctgg tcttaaactc ctgaccttgt gatctgcctg ccttgacctc ccaaagtgct 14580
   gggattatag gcgtgagcca ctgtgctggc ctatttctat tattatttta ttgtaatata 14640
   tgatgaaata attatataac tcaccataat ataaaatcac tgggagcctt gagcttgttt 14700
   tcctgctgaa atggtcccat ttgggtgtga tgggagacag tgacagatca tcaggcatta 14760
   gattctcata aggaatgtgc aacatagccc cctcacatgt acagttcaca atagggttcg 14820
   cgctcctatg agaatctcat gccaccgatg atctgacagg aggtggagct caggccataa 14880
   tgcaggtaat agggagcaat cgtaagtaca gatgagactg ttggcttgcc tgctgctcac 14940
   ctcctgttgt gcagcccagt tcctaaaaga ctgttgggtt ggggacccct gatttaaagc 15000
   aatgaatact ttgaaaaatg tgctggacaa gatggctcaa gcctgtaatc ccagcgcttt 15060
   gggaggccga ggtgggtgga tcacgaggtc aggagttcaa gaccagcctg cccaacatgg 15120
   tgaaaccccg tctctactaa aagtacaaaa attaaccgag cgtggtggtg ggtgcctgta 15180
   atcctagcta cttgggaggc tgagtcagga gaatcacttg aaactggaag gcagaggttg 15240
   cagtgagctg agatcgcacc actgcactcc agcatgggca aaagagcgaa actccgtctc 15300
   aaaaaatata tatatatatt tattaatata atacataatt tttttagcag attttacttc 15360
   ctaattcgtt tctacataag tggaaatatt atgaaattga gtttgaaaat tctccacata 15420
   caactaagtg gagggaaaca atttgaaaca ttctgagtga ctaaggaagg cttgcctgta 15480
   gttaaaattt aatgagacag tacacctgga gagttgaatt gattgtatat tgaatgacct 15540
   ttgccgcctt tttatcctct caaagtgagg ttttcataaa attccttttt tttttttttt 15600
   ttttgagacg gagtctcgct ctgtcaccca ggctgcagtg cagtggcacg atctcagctc 15660
   actgaaagct ccgccttcca ggttcatgcc attctcctgc ctcagcctgc cgagtagctg 15720
   ggactatagg cgcccgccac cacgcccggc taattttttt gtatttttag tagagacggg 15780
   gtttcaccgt gttagccagg atggtctcga tctcctgacc tcatgatcct cccgcctcgg 15840
   cctctcaaag tgctgggatt acaggcgtga gccaccacac ccggccataa aattctattt 15900
   ctgcgtattt actgcaggca tatactgtgg agtgcaggga ggggttgaag gtgagatcta 15960
   tgtatatgaa ctaaataaag gataaaataa agcagaacaa ctcctttcta agctttcaga 16020
   aattatgaac ttgttgcgtt aggtgtaaag agttcaacgg ggaagtgttt taaaatttgg 16080
   gattcttggc cgaggtgggc gaatcactag gtcaggagat cgagaccatc ctggctaaca 16140
   tggtgaaacc acttctctac taaaaataca aaaaaatgag ccgggcgtgg tgatgggcgc 16200
   ctgtagtccc agctactcgg gaggctgagt caggagaatg gcgtgaaccc gggaggtgga 16260
   gcttacactg cactgcactg cagcctgggt gacagagtga gactccgtct caaaaaaaaa 16320
   aaaaaaaaat ttgggattct ggccgggcac agtggctcat gcctgtaatc ccatcacttt 16380
   gggaggccga ggcgggcaga tcacctgagg tcgggagttt gagaccaggc tgagcaacgt 16440
   ggagaaaccc catctctact aaaaatacaa aattagctga gggtggtggc acatgcctgt 16500
   aatcccagct actgaggagg cttaggcagg agaatcgctt aaacctggaa ggtagaggtt 16560
   gctgttagcc aagaccatgc cattgcactc cagcctgggc aagaagaacg aaactccgtc 16620
   tcaaaaaaaa aaaaaaaatt gagattctac acattctgtt ttctatgact ccaatttatc 16680
   tttctgtctt ggattatctt gtttttaggc tcaactggag aagctacagg cagagatttc 16740
   acaagcagct cgaaaaacag gcatccatac ttcgactagg cttgccctca ttgctcctaa 16800
   gaaggagcta aaggaaggag atattcctga aattgagtgg tgggactctt acataatccc 16860
   caatggcttt gatctgtgag tttgttttag taccttttta aaaaaacagt ttttaatcag 16920
   ctgagtttaa gactcaagtt aatacctttt agtaaagcta tggttttagt gggaaggatg 16980
   gtactcattc attaattaaa atttttaaat ccaagagaga taagtaactg tacacaaggt 17040
   cctgtatttt tttttttttt tttttttgag agggagtctc gctctgtcac ccaggctgga 17100
   gtgcagtggc acgagctcag ctcactgcaa cctccacctc ccgggttcaa gagattctcc 17160
   ttgaattctc cttgaatctc agcccctcaa gtagctggga ctacaggtgt gtgccaccat 17220
   gcccatctgc tttttgtatt tttgatagag acagggtttt gccatgttgg ccaggctggt 17280
   ctcgaactcc tgacctcagg tgatccgccc accttagcct cccaaagtgc tgggattaca 17340
   ggcgtgagcc actgcacctg gccaagctcc tgcatttgta agtgatgtat catatatata 17400
   ggctgttttt agcttttggg gttaatgtag gataaccagg gacacaatct acaaaagcaa 17460
   agtctagaat tctttttgct ctgttacaca ttgttgcctg tttttagcat tattttcctt 17520
   aattacattc ttttgctacg tattataggt gtttacattt cctaaagatc tcattgtgat 17580
   agagcaagga ttattagttg gtaggtgaga taatagtaaa tatagcatat aaggctgggc 17640
   tcggtggctg acacctgtta ttccagcact ttgggaggct gagggctgaa ggattgcttg 17700
   agcccaggag ttaaagacca gcttgggcaa tatggtgaga cctcgtttct acaaagatta 17760
   aaaaaaaaaa aaaagtagct gggcatggtg gcttgtgcct gtagtcctgg ctacccagga 17820
   ggctgatgtg agagaatcac ttgagcccag gagggtctgt gctgcagtca gctatgatca 17880
   tgaccactgc actctatcct ggacaacagg gtgaaactct gtctcaaaaa aaaaaaggat 17940
   ataagacaca tttgaagagc tttaaatata tctgctccat ccattttttt tttttttttc 18000
   ctgagacgga gtttcgctct tgttgcccag gctggagtgc agtggagcaa tcttggttca 18060
   ccacagcctc cgcctcctgg gttcaagtga ttctcctgcc tcagcctccc aagtagctga 18120
   gattataggc atgcaccacc acgcctggct aatttttggt gtttttagta gagacgaggt 18180
   ttctccatgt tggtcaggct ggtcccaaat tcccgacctc aggtgatcca cccgcctcag 18240
   cctcccaaag ggctgggatt acaggcatga gccaccacgc ctggcgtttt tttttttttt 18300
   tttttttttt aagacagagt ctcactctgt tgcacaatct cggctcgctg caacctctgt 18360
   ctcctgggtt gaagcagttc ttctagctca gcctcataag tatccgggac tacaggcaaa 18420
   taccaccaca cctggccagt ttttatattt ttagtagaca agagtttcac tatttggcca 18480
   aactggtctc aaactcctga cctcaggtga tccacctgcc ttcatctccc aaagtgctgg 18540
   gattataggc ataagccaca tcacctggcc ctgctccatc attctgatac atggctttag 18600
   tagattttgt cttagccttt cgttgcccct tctttgtcag ccactgttcc agggacttat 18660
   tgtcactaat gagagaacat agcgtctgtc agctgtggca tggtagaaaa aaggttgaga 18720
   atgattgctg tggagagtgt gttcaaccat agagggactc agttttctca tcatgaaaat 18780
   tatgtgtaat ttaactatat attttaggta cctaaatgct attttaggta tatcaagcac 18840
   aagaacccat tatagagagt gggttttttc attgtatttc tgagactcct ctcttagaat 18900
   catctctacc cgcatatcgt gttttctttt cttcctacaa ttttagtaca gaggaaaatc 18960
   ccaagagaga agattatttt ggaatcacaa atcttgttga acatccagcc cagctcaatc 19020
   ctccaggtaa tgtatagatt cctgaatcaa gtctctagag cagcattacc cattggaaat 19080
   ttatacagtg aggaacatgt tctgtatcta tgctgttcaa tacaatagcc actacccatg 19140
   tgtaattctt ttttttaatt cagttttttt cctgacaata gtttgttgtt tgaccagtga 19200
   acatacattt taagtgcata ttcctgtgct tagaatttca tgagacagcc aagcatggtg 19260
   gctcatgcct ctaatccaaa cactttcgga ggccaaggca ggaggattgc ttgaggccaa 19320
   gaattcaaga ccagtctggg caacacagtg agacccatct caaggaaaaa aaatttggct 19380
   gggtatggtg gcttatgcct gtaatcactg cactttggga ggctgaggtg ggcagattgc 19440
   ttgagctcag gagttcgaga tctaggcaac atggcaaaac cccatctcta caaaaaataa 19500
   aaaatcagcc gggcatggtg gtgtgcatct gtagtcccag cctcttggga ggctgaggtg 19560
   tggaggatgg cttgagcctg ggaggtggaa gttgcagtga gctgagatgg tgctgctgca 19620
   ctccagcctg gatgatactg ccagaccttg tctcaaaaaa aaaaaaaaaa aaaaaagaag 19680
   aaagaaagaa agaaaaaaac ccaaaatttc atgaagcttt acatttacag ttccctggtt 19740
   gttcctagga aagagccctc agcttgctcc ttttatagaa ccacatgttg ttgtggcatc 19800
   cacagctaaa tgcaaatgca agagccaaac tcttgggttg gtgtgttatt agatgcgata 19860
   tgctccacta cccactgtga gggatgctaa ttccaaaggg ctttttgtta gtttcccaaa 19920
   gttgaaactg ggaaacaaag ttgcttctat tttttttttt tttttttttt tgaggaggag 19980
   tttcgctctt gtggtccagg ctgcagtgca acggcatgat ctcagctcac cacaacctct 20040
   gcctcctggg ttcaagcaat tctcctgcct cagcctcctg agtagctggg attacaggca 20100
   tgtgccacca cgcccagcta attttgtatt tttagtagag acagggtttc accatgtttg 20160
   gtcaggctgg tcttgaactc ctgacctcag gtgatctgcc agccttggcc tcccaaagtg 20220
   ctgggattat aggtgtgagc caccgcgccc ggcctttttt ttttttttga gttggagttt 20280
   cgcttttgtc acccaggctg gagtgcaatg gcactatctc cgttcactgc aactttcacc 20340
   cccctgggtt gaagctattc tccggcctta gcctccggag tagcttggat tataggtgcc 20400
   tgccaccaca cctggctagt ttttgaactt ttagtagaga cagggttttg ccatgttggt 20460
   catgcttgtc ttgaactcct gacctcaagt gatctgccca cctaggcttc ccaaagtgct 20520
   gggtttaccg gcgtgagcca ccgcacctgg ccctaatttt gttttctaca tttgccaggt 20580
   atcattgaat gagggggttt aacagcttaa aggaaaacaa ctaagaacac tacttaagaa 20640
   agataagagg ccaggcgagg tggctcatgc ctgtaatccc agcactttgg gaggccgagg 20700
   cgggcagatc acgaggtcaa gaaatcaaca ccatcctggc taacaaggtg aaaccccgtc 20760
   tctactaaaa atacaaaaat tagccagacg tggtggcggg tgcctgtagt cccagctact 20820
   caggaggctg aggcaggaga atggtgtgaa cccaggaggc agagcttgca gtgagctgag 20880
   attgcgccac tgcactccag cctgggcagc agagcgagac tccgtctcaa caaaaggaaa 20940
   gataagaatg ttttttaaaa acatagccat attgccgggc accaggcctg aactgatttt 21000
   aatcttactg atcccttatt aacgagtttt cttcattttc tatttaaagt ttatacacac 21060
   acacacacac acacatgttt tttgaaatgg agtctcgctg tgttgccagg ctagagtgca 21120
   gtggcaagtg gcacaatctc ggctcactgc aacctccacc tcccggattc aagtgattct 21180
   cctgcctcag cctcccgagt agctgggact acaggtgcgt gccaccacgc ccagctaatt 21240
   tttgtatttt tagtggagtc agggtttcac catgttggcc aggctggtct cgatctcttg 21300
   acctcgtgat ctgcccaccc gtgcctccca aagtgctggg attacaggtg tgatccaccg 21360
   cacctggcct caatatattt tttacataaa atgtttataa tagagttggg tctcacttgg 21420
   ttgtccaggc tggtctcgaa ctcttgggct cacgccatcc tcccaccttg gcctatcaaa 21480
   gtgctagaat tacaggcata agccactgtg tccagcctaa agtttctttt aatgatattg 21540
   atatttacct aggtttcttt aatattttta ttgctgtccg ggcctggtgg ctcatgcctg 21600
   gccaacatgg tgaaacccca tctctactaa aaatacaaac attagccagg catggtggcg 21660
   tgtgcctgta atcctagcta ctcgggaggc tgaggcagga gaattacttg aacccaggag 21720
   gcagaggcta cagtgagctg agatcgtgcc attgcactcc aacctgggtg acagagtgag 21780
   agagagaaaa aaaaaaaata tatatatata tatatgtatt cttactgctt tggtatacta 21840
   atatctctgc ctgacagttg acaatgacac accagttact ctgggagtat atcttaccaa 21900
   gaaggaacag aaaaaacttc ggagacaaac aaggagggaa gcacagaagg aactacaaga 21960
   aaaagtcagg ctgggcctga tgcctcctcc agaacccaaa ggtgcatttc tcagtggcct 22020
   cttctgttaa aaagagtggc aagtttatgt ctttaatcct ggaggaactt taacatctct 22080
   gtgtttgagt gatttctgtt tctaagaatt attcttgggg tcctttttct ccaagttttc 22140
   taccatcttt tcatattaag ataggtcaac acattcatta tagaggccat ttatggctta 22200
   tttttaagca tactgatcat acaacactct ggttttcctg gaagaataca ggtcttgaaa 22260
   ttcatggaga tagtagttgc agtatttgcg tagacttttt atgtacataa ttcttaatgt 22320
   tttaattttt acaatgaaag tacaggataa aaaacttgac cctgtagtta cattctcgtt 22380
   cagcagtatt ttgactgagc actattattt ataagctctt tcactagatg ctgtgatcag 22440
   tgtgaaaaat atgaaaggaa ttaagttcca cgtccttcat aaattttcag ataatgtaaa 22500
   tgcataccaa tcttcaactt aattgtgtga acttctgcaa atgctgaagt gattggatgg 22560
   gattttcaag ataggagtta ttggaggaag tgagtttaga gcagagattt gaaataggta 22620
   aataatatga attggtatgg aagaaataac aagttactcc agctggagaa gtgacttcaa 22680
   agactgattg ttgtcctctc ttcactgcag tgagaatttc taatttgatg cgagtattag 22740
   gaacagaagc tgttcaagac cccacgaagg tagaagccca cgtcagagct cagatggcaa 22800
   aaagacagaa gtaagtgcca tgggattggg tggaaacctc gggcaagtac ctttcccaaa 22860
   ctcttctggg gggtccatat ttggagggag gggagagttc tccgttgctc tctctttccc 22920
   tcagccctgc ctctgatctt caatgccttt ctcacttgtg gatgtccttc ctgtcacgac 22980
   agagcgcatg aagaggccaa cgctgcccga aaactcacag cagaacagag aaaggtcaag 23040
   aaaattaaaa agcttaaaga agacatttca cagggggtac acatatctgt atataggtag 23100
   gtgtccatac tgggccaaac ttccccttag aattactaaa acattttcca agtgcttgag 23160
   gcagaatcat tgtggcctaa aagcatacct aacaccagtt cctactctct tttaatgacc 23220
   ctagtgtgga tcaagagcta gtaaatcttg taaaatagcg agagttaatg acttatgttt 23280
   atggttaccc agttcttccc tctcaagtct tctttttttt tttaccagaa tattagttct 23340
   gaacaagtca atatacttgt tcaggccaag atgtgaacta aagattgtat gtcctttgag 23400
   aactataaaa tatcatccta tttctttttt ttttcatctt ttttttttta aatggtctga 23460
   gtttgcttcc aacatccatg gaagcattac aaattttctt tcctccgtag tcatatcctt 23520
   accctgatgt attgctgtgt ttcatcctca ctccaaaatg cattttatat gttgtaaata 23580
   tttttctacc tttgtgttga tttaatgttt ttattctttt atgttgagta catgtgtatc 23640
   ttctattttt aaaatgtgct tatttctatt aactagttct ttattataaa agtggtacat 23700
   tatcacaaaa atagtgaatg cttattaaga tttaaaaaat tgtctgacaa cacagaagaa 23760
   tacttactca aaatgtctcc tttctattct ctgatccctt agaaccactc tctttaggta 23820
   attgctatta acagtttttc acatatcctc ctagaaaatt ttcatgccta tatagtgatc 23880
   ctatatatat attttattta tttatttatt tattttgaga cggagtttca gtcttgttgc 23940
   ccaggcagga gtgcaatggc gcgatctcag cgcactgcaa cctctgcctc ccaagttcaa 24000
   gcaattctcc tgcctcagcc tcctgagtag ctaggattac aggcacgcac ccccacgccc 24060
   ggctaatttt tgtattttta gtagagatgg ggtttcacca tgttggccag gctggtctta 24120
   aactcctgac ctcgtgatct gcccactttg gcctcccaaa gtgctgggat tacaggcatg 24180
   agccaccgca cccagccaag taatcctata ttataagagt ctaagggtct agaggacaaa 24240
   aagtctgtag gtctttacat tgtcaagaac aatgagattg taagtatttt tggatgatag 24300
   taaatacctc tccctgactc ttagagtaaa ctttggttag taatgtaatg gttaatgtaa 24360
   agtgctagac tttgaagaaa tgtaagagtg tggaaatatt tctagactca tcaactctac 24420
   cttttaagca ttcatctatt tgtttactcc agaggccttt gtttatagag aggaaaccat 24480
   tttggattca tctttttact tgccactcca ttcaggcagc tgctgatgct aaaatggaag 24540
   acataactaa actttccctt ctccagagtt cgaaatttga gcaacccagc caagaagttc 24600
   aagattgaag ccaatgctgg gcaactgtac ctgacagggg tggtggtact gcacaaggat 24660
   gtcaacgtgg tagtagtgga agggggtgag tctgaaaaac ttgagggaga ctgtccccag 24720
   acaaccctag gcttagagtg attagcattg tggggagaaa ggagaaaaag atcgtatttt 24780
   agtgccatac ctgatataat atgctaggtc tctgcttctg tgtttttggg agtagaaggc 24840
   cctaagatgt ggttcctgag attgcttcaa ctaccacatt aatgtctgag ctcagaggtt 24900
   ctttctaccc catcatcttc cacagttctg gcaaaattat tcttctctgt ttccaggccc 24960
   caaggcccag aagaaattta agcgtcttat gctgcatcgg ataaagtggg atgaacagac 25020
   atctaacaca aagggagatg gtgaatgggg gttagagggg attaaggggg agagctatgg 25080
   gaggtgggga tggtgcatct gtccgttcat cttatttcct ccctgtgaca ctcttgataa 25140
   cactagatag tgttcaggaa ttttagaaag acccgtttag actttgagcc tcctagacat 25200
   tcatacaata tttactacat gattatatgc atgaatggta aagctatttt tttgcctatc 25260
   atttaaggct tttctaccag ctggtttctg tgtctgtcct ggatctgagt accggttaca 25320
   tgaatttatt cagtatttga aaattcagca agcagctagg catggtggct catgcctgta 25380
   atcctagcac tttgggaggc tgaggcgggc agattgcctg aggccaggag ttcgagacca 25440
   gcctgggcaa acaacgtggt gaaaccccat atctacaaaa agtacaaaaa ttagctggat 25500
   gtggtggcac acacctgtat tcccagctac tcaggaagct gaggtgggag gatcacttga 25560
   gcttggggag gtcgaggctg cagtaagctg tgatcgtgcc actgcaatcc agcctgggtg 25620
   acagaatgag accctgtctc aaaaaaaaga aaaagcaaat ctagtaagct atacattcat 25680
   gtgtacagtg tctatattat attattattt gatttttttt tttttaagga ttgaagccaa 25740
   aaagatcttc aggtcaaaaa agctctgctt tgatcaaact atttcttttg tctgatatat 25800
   atatatacac acatacacat acatacacac acacacacat acatacacac atgtacacac 25860
   acatacacat acacacaata cacacacata catgtacatg tacatactca cacatatgct 25920
   cttcaactta atgatggggt tagttccaat aaacctgaga taaattgaaa atattgttaa 25980
   gtcaaaaatg catttaaggc caggcacggt ggctcacact tataatctga gcactttggg 26040
   aggccgaggc gagcagatca tttgaggtca ggagttcgta acagcctggc caacatggtg 26100
   aaaccccgtc tctactgaaa acacaaaaat tcggggcgtg gtggtggacc tctgtaatcc 26160
   cagctactcg ggaggctgag gcaggagaat cgcttgaacc tggcaggcgg aggttgcagt 26220
   aagctgtgat cgcaccactg cactgcagcc taagcgacag agcaagactc catctcaaaa 26280
   aaaaaaaaat gcatttaata catctgacct acaggacatc ataacagcct agccttcctt 26340
   atactttctc agaatactta gattagccta cagttgggca aaatcatcta acacaaagcc 26400
   tatttttatc tcatgtaaga gtattttact gcatattgct agcccaggaa aatctcaaaa 26460
   tttaaaattt gaagtacatt ttctactaaa tatttatcac tttcacacta tactcaaggc 26520
   aaaaaattgt aagtcaagga ctgtctgtct atatttgaag gccagacatt cttctggcca 26580
   tcatactccc attagtgttc attcttctca tctttgaaca cctggattag catttaaaat 26640
   ctgcagagag gcctggtgtg atggcttatg cctgtaatcc cagcactttg ggaggccgag 26700
   gcgggtggat cacctgaggt caggagtttg agaccagcct gacatggtga aaccccgtct 26760
   ctactaaaaa atacaaaaat gagctgggcg tggcagcgtg cgcctgtaat cccagctact 26820
   cgggaggcct aggcaggaga atcacttgaa cccaggaagc ggttgcagtg agctgagatc 26880
   ataccacttc actccagcct gagtgacaga gcaagactcc gtctcaaaaa aataaaaata 26940
   taaataaaat ctgcagagag ttcatcttaa gatacctgag aataaaaaat attttgttct 27000
   acacgtgcat tttttttttt ttttttttga gatggagtct ttgctctttt gcccaggctg 27060
   gagtgcagtg gcaccatctt ggctcactgc agcctccgcc tcccaggttc aagtgattct 27120
   cctgcctcgg cctcctgata agctcggatt acaggcacct gccaccacac ctggctaatt 27180
   tttgtatttt tagtagagat gaggtttcgc catgttggct aggctggtct caaactcttg 27240
   acctcaggtg atccactcac ctcagcctcc caaagcgcta ggattacagg cgtgagccac 27300
   cacgctggcc gataattttg atttggtata tctgccctcc tcctccattg ggtgaaagtt 27360
   ttttgagagt aatatatgat gttgacatat gaagcctgcc tcccaaagtg cctaggacaa 27420
   gtttttattc ttgactgctt gtcttttatt tccttattcc cagtacctgg tggagctctt 27480
   gattcataac aagtgctatg taaatgctaa ttgtatcaat taatattact gcctacttgg 27540
   agcttcaagt ctaatttggg gaaaataaag agcaacagaa aagagaacac ttggtccaac 27600
   acataaaaag ggtgataata ttttagagag tttgggtaga cttgaatatt atttgtttag 27660
   aacctgaatc tcaagtctaa gtctgtaaca agatttctct tccagatgat gaggagtctg 27720
   atgaggaagc tgtgaagaaa accaacaaat gtgtactagt ctgggaggta ggtgatcctt 27780
   tgtaaaacat tgtaaaactt tagccaactc ctgaatattt atactatttg agtggagcaa 27840
   tggaatgtaa gatgtaatca gtgaataatg ttttaaataa tttatgtttc tttttggaat 27900
   ccatttttaa atggttggca gtaaattctt cctgatcatc tgtttaatta aattcgtgag 27960
   tatttagtat atgctatctg gggaagtgcg tggacagtgc tgctggtagc aggaatacaa 28020
   attctctacc gaaaattatt tatttcacag tcatgaatat gagacttgga aacgggaatg 28080
   aattgttgtt ggatttacct ttggatatag tgaaggaagt actagactta agaatataaa 28140
   gttaggaggc tgggcagtgc tgcttattag ctgtgcaatc tgggcaagac acttaatgtc 28200
   aaattctgtt gccttagcta ttgttctcta tattgtcaag tttttgagaa tgaaattaga 28260
   taattatgtg atagtgcttc ataaaatgtg actgaatcac tcttcaagtg gaatattttt 28320
   tatggttatc tagttcttta acccctgaag ccttcttaga gaaccagtat taaaaccagt 28380
   attaatgttt tcatgtttgc ttttaggtat agtacatgta tatctttttt tgggggcggg 28440
   ggggcggggg gcggtatgga gtttcgctct tgttgctctt gttgcccagg ctggagtgcg 28500
   atggcgtgat ctcggctcac cgcaacctct acctcccagg ttcaaacgat tctcctgcct 28560
   cagcctccca agtagctggg attacaggtg cacgccacca tgcccgacta atttttgtat 28620
   ttatagtaga gacagggttt ctccatgttg gtcaggctgg tcttgaactc ccaacctcag 28680
   gtgatccgcc tgcctcggcc tcccaaagtg ctgggattac aggcatgagc cactgtgccc 28740
   agcccctcca tttctttttt tttttttttg agagagtttt gcccttgtta cccaggctgg 28800
   agtgcaatgg tgcaatctca gctcaccgca accaccgcct cctgggttca agtgattctc 28860
   ctgcctcggc ctcccgagta gctggaatta caggcatgag ccaccacgcc tggctaattt 28920
   tgtattttta gtagagacgg ggtttcgcca tattggtcaa gctggtctcg aactcctgac 28980
   ctgaggtccg actgccttgg cctcccaaag tgctgggatt acaggcatga gtcactgcgc 29040
   ccggccatat atcttctatt tttaaaatgt gcttatttct agtatatgtg ctgctgaagc 29100
   gagcagtaaa atgtgcttat ttctattaat gaattcttta ttataaaagt ggtacattat 29160
   tacaaaagta gtaaatgttt attaagatta gaaacaaatt ctaattatac agaagagtac 29220
   ttactgagac ccagcacagt ggctcacgcc tataatccca gcactttggg aggatgaggt 29280
   ggttggatca cttgagctca ggagttcaag accagcctgg gcaatatggc gaaaccccgt 29340
   ctctacaaaa aatacaaaaa ttagcctggc ctggtggcgt ctgcctgtag tcccagctac 29400
   ttgagaggct gaggtgggaa gattgcttca gctttggagt ttgaagctgc agtgagctgt 29460
   gactatgcca ctgcactcca gcctgggcac cagagtgaaa ccctgtctta aaaaaaaaaa 29520
   agaaaaagtc tcaggccagg tgcagtggct catgcctata ctcccaacac tttgggaggc 29580
   tgaggtgggc ggattacgag gtcaggaatt cgagactaac ctggccaaca tggtgaaacc 29640
   cagtctctac taaaaataca aaaattagtg gggcgtggtg gccagtgcct ataatcccag 29700
   ctacttggga ggctgaggca ggagaatcgc ttgaatctgg gaagcggagg ttgcagtgag 29760
   ccgagatcgc accattgcac tccagcctgg gtgacagtgc gagactgtgt ctcaaaacaa 29820
   aagtctcttc ctattcgctg atctcttcag accagtcttt ataggtaatt gctattaata 29880
   gtttttcttc tgaaggagca atcaaaaaat aaaaattaaa aaaaaaggtt ttcatgtatc 29940
   ctgccttatc ccagattctt caatttatgc atacaaatta gatgataata accatagtat 30000
   ttgttctctg ttttataagt tcccacctgc atatttgtta tcttttttta atcaactttt 30060
   ttttttagag atagggtctt gctctattgc tcaggctggt gtgcagtggc gcagtcatag 30120
   ctcgctacag ccttgaactc ctagggacaa gcaatcctcc agtctcagct tctcaagtat 30180
   ctgagacctc aggtccacac cactgtgcct ggctaatttt tttttttttt tttttttttt 30240
   ttagaaatgg atctcactat gttgcctagg cttgtcttga actcttagcc ttgagtgatc 30300
   ctcccacctc agcctcccaa agtgctggga ttataggcat gagctactgt gcccagccct 30360
   accttcacat atctcagcac cctcatcttt caattttctt ctatttatct ctgaagagta 30420
   cttactgaaa aagtcccggc ttcttagaga accagtatta atgttgtagg aatttagttt 30480
   tcaggaattg tctctcatgt tcagaaataa gccaagttct gctccagcct ccttattgga 30540
   gtctactccc tgaacgcctc ttgaggtatt gctttatgac tgccattgct gctggtaact 30600
   ttggcaagaa tgccattctg cccagtactg tctttttctg ttgtaattct ctgctggtac 30660
   ctgctcttct tctgttcccc gcccccagtt gttctttgtt gctgggcccc aaagcctcct 30720
   gcttatataa atagtttctg ggcctttttt gaaggctcag atttagtcct taaggcctgg 30780
   taggagataa gtttatctca ttccaccttc catagttctt cctgacttgc tgttccaccc 30840
   aggcaccaga gcaaataatt cctcacttca tttcaaaaaa ctaaaagcct tatgcatatg 30900
   aattcgtaaa atctcataca aatatccaag gaggccgggc gcggtggctc acgcctataa 30960
   tcccagcact ttgggaggct gaggcgggag gatcacgagg tcaggagatc gagaccatcc 31020
   tggctaacac ggtgaaaccc cgtctctact agaaatacaa aaaatcagct gggcgtggta 31080
   gcgggcgcct gtagtcccag ctactccaga ggctgaggca ggagaatggt gtgaacccgg 31140
   gaggcggagc ttgcagtgag ccgagatcgt gccactgcac tccagcctgg gcgacagagc 31200
   gagactctgt ctcagaaaaa cacaaatatc caaggaaatg gaggtcacaa ttttttttta 31260
   accataaaat cacgttcagc tgggcacatg tctcacaaat gttactaggt cttcttataa 31320
   aagaatatta tctgatttaa ataagaaatt gaagtgtttt tattatatat ctcttttcta 31380
   gggtacagcc aaagaccgga gctttggaga gatgaagttt aaacagtgtc ctacagagaa 31440
   catggctcgt gagcatttca aaaagcatgg ggctgaacac tactgggacc ttgcgctgag 31500
   tgaatctgtg ttagagtcca ctgattgaga ctactgcaag cccttgcctc tcctcccttg 31560
   cctttgtctc ttcagtcctc tcacttattc tatttcccaa ccccctccca cttgtttgtg 31620
   tgatctcaga actgtgccaa gcagacactg ggacaaaggg agaatatctt gctcccctcc 31680
   tgagtcagcc tggtgttgcc ctttattccc cttatgtgca tatgattaaa gagttatttt 31740
   taaacttggt gtgatatttt tcacacattc gtaagta 31777
<210> 2
   <211> 34254
   <212> DNA
   <213> Homo sapiens
<400> 2
   gaactcgggt tgcgcacttc ccggcgctgg gaacgcggag cggacgcagt ctggccgcca      60
   ttgcgctgcg gggaaagcgg cctcttgtgt gagggcctgt ggggtgcgtg gcaggcgttt     120
   aaggccgggc tgcgggaggc gtggtcgggt cgggccgagt gaccgaaggg gttaggccgg     180
   gcggggctgg atgcactgtg tggcgggact gcaggccggg gtccccggac gcctttctgc     240
   tgcgcttctt gtgggcccgg gcctgtggtt agtgaatttt tctatcccgc cgctccccag     300
   attctccgga tatggccgga gtgtttcctt atcgagggcc gggtaacccg gtgcctggcc     360
   ctctagcccc gctaccggac tacatgtcgg aggagaagct gcaggagaaa ggtgaggcga     420
   gagtgtggag cggcgcgcgg gccggaggcg gggcgggtgt gcgcgcgcgc aggcgggtgg     480
   ggaccctccc cgtttgggca gctcctgagg tgtcagctcc cttgttattt ccagttaatt     540
   tttcatcaag tcattttaag cgcgtattat ttgcaagcgc tgatccagcg ggggtacagt    600
   agtgacggag acaaaattcc tgtccttacg gggtttaaat tacagcggga gagacagaga      660
   ataacaaaac cttgaagctc tgtaataaaa atgtcaggcc gggcgcggtg gctcacggtt     720
   gtaatcccag cactttggga ggcccaggcg ggcggatcac ctgaggtcag gagttcgaga     780
   ccagcctggc caacacagtg aaacctcgtc tctacgaaaa atacaaaaat gagtcgtgtg     840
   tggtggcgcg tgtctataat cccagctact ggggaggctg aggcaggaga atggcttgaa     900
   cccaggaggc ggaggttgca gtgagccgac atcgcaccat tgcactccag cctgggagac     960
   agagtgaaac aacctctcaa aaaaaaaaaa aaaagtcaga tagtgatcaa tgtgatgaaa    1020
   ggaaaaacag agtaaaatgg taagggaatt ggcaaggggt gcttcttcct gctggtgcct    1080
   gcttctccct cacactcact gacaggagct taccctaggg caagaggtga agaggattag    1140
   ggggagggtg tgcaggcctt taaactgagg tgcctttcca gctcgaaaat ggcagcaatt    1200
   gcaggccaag cgctatgcag aaaagcggaa gtttgggttt gtggatgccc agaaggaaga    1260
   catgccccca gaacatgtca ggaagatcat tcgagaccat ggagacatga ccaacaggaa 1320
   gttccgccat gacaaaaggg tttacttggg gtaaggaaca tcctgaaata atttcatttg 1380
   gaatttggcc cttttccctt ctccctgtcc cacagtcttg gattgatgct ttgacagctc 1440
   atcttggtgg atataagaga aaggagaaaa ccagtaggag tctctgataa tttctacatc 1500
   ttccactttt gcagtctgtt tcccctctct tcccgatctg ggactggtgc tttaatttgt 1560
   cctgttgatg atattataag atgtccctgg ccaggcgcgg tggctcatgc ctgtaatccc 1620
   agcactttgg gaggccgagg cgggtggatc atgaggtcag gagatcgaga ccatcctggc 1680
   taacacggtg aaaccccgtc tctactaaaa atacacaaaa ttagctgggc gtggtggcag 1740
   gtgtctgtag tcccagctac ttgggaggct gaggcaggag aatggcgtga acctgggagg 1800
   cggaacttgt agtgagccga gatcacacca ctgcactctg gcctgggcga aagtacgaga 1860
   ctccgtctca aaaaaaaaaa aaaaatgaaa aataagatgt ccctatggtt gagctggttg 1920
   aagtccagag tgggatactg tgcatagtgg cagcctgtcg ctcctcaggg acctgaattc 1980
   tgtgtttggg gtatttaatt tcagaggttt ccctactcct gttttttctg tggctctaaa 2040
   ctgggctagt acaggtacag atgtcttagc agattcctgt tctatttgga cacaaaaatg 2100
   tttgccatgt aatgaactgg aagttttgtt tatttcaaaa aatacttaga gaactgtgct 2160
   ggctgctggg gatatagtag cgagcagagg gtaacaatct ccgcctttct gcagtgtgcg 2220
   gtctagtggc ctgacagaca tgagacaact ggtgatagtg aagtatggtg aatactttat 2280
   tatgatttat gggaagatgc ggagggaata ggatgtgaga gagccgcaaa gctgtctcca 2340
   gagggatagc cgttgattta gaggagctga taccagcatt gtgggattat gacgtttaag 2400
   aagagtggtg caaggtgaag ttggagaggg agaggcaggg tttctggtta ggaagggcct 2460
   gtgggctaag tttgggagtt tggattgtaa ggtaggactg tggaacatga acatgtggct 2520
   tctctgcatg ggacaggtag cagaacacct cgtcatttct gatagccttc tctttttcta 2580
   atgtgccagt gccctaaagt acatgcccca cgcagtcctc aaactcctgg agaacatgcc 2640
   tatgccttgg gagcagattc gggatgtgcc tgtgctgtac cacatcactg gagccatttc 2700
   cttcgtcaat gagattccct gggtcattga acctgtctac atctcccagt gggggtgaga 2760
   aaggctgaaa tatggtgggt ggtggtggtg gtggtggttg ggaggcagtg ggtagaaatg 2820
   gttgtgtgat gtcttgcctt ccaggtcaat gtggattatg atgcgccgag aaaaaagaga 2880
   taggaggcat ttcaagagga tgcgttttcc cccttttgat gatgaggagc cgcccttgga 2940
   ctatgctgac aacatcctag atgttgagcc actggaggcc attcagctag agctggaccc 3000
   tgaggaggac gcccctgtgt tggactggtt ctatgaccac cagccgttga gggacagcag 3060
   gaagtgagtg atagatggag atactacacc tgggaggagt tctggaaatg cagaaggagt 3120
   cctgggagat cagtaggtgg tatgggtttg tctagctgct tacttgaggt gggcctttca 3180
   ttttttacct aggtatgtaa atggctccac ttaccagcgc tggcagttca cactacctat 3240
   gatgtcgact ctctaccgcc tggctaatca gctcctgaca gacttggtgg atgacaacta 3300
   cttctacctg tttgatttga aggccttctt tacgtccaag gcactcaata tggccattcc 3360
   tggaggcccc aaatttgaac ctcttgttcg agacatcaac ctacagtgag ttggagagat 3420
   tagggatttt agacattttg agttgaatga aacgtaatct tgatccttaa agtcctgata 3480
   taacttccaa attagtctgt ttacaccagg tgcagtggct cacgctgtaa tctcagcact 3540
   ttgggaagct gaggcaggag gattgcttga gcccagaagt ttgagaccaa tctgggcaaa 3600
   atggcaaaac cctatcttta ccaaaaaatg tagctgggcg tggtgacact cacagcttcg 3660
   tgtagtctca gttacttggg aagctgagct gtgggaggat tgtttgagcc caggaggtca 3720
   cggctgcagt gaacgatgat catgccattg cattccagct tgggtgataa gagtgagacc 3780
   ctgtctcaaa aaaaaaaaag tctgcttatt cttgatgttg taccctaggg atgaagactg 3840
   gaatgaattc aatgatatta acaagattat catccggcag cctatccgca ctgagtacaa 3900
   gattgctttt ccttacttgt acaacaatct tccacaccat gtccacctca cctggtaaga 3960
   gacctggaac acaacatttg gaaaggagaa ggctgctgtt taggttgggc cagggcggga 4020
   aaactttggc tgactcttcc tttcatttta ggtaccatac tcccaatgtt gtattcatca 4080
   aaactgagga tcctgacttg ccagctttct actttgaccc tttgatcaac ccaatctccc 4140
   ataggcactc agtcaaggtg aggaagggaa gggatgcttt ccctcagctc ttctaggaga 4200
   aatgcgtgag gttggctgat gttactaaac gctcaggtgg agggagatgt gttttccgtc 4260
   ttgctaagtg ttccccttga gctataagcg aatatgagaa ttcagcattt gctttaaaac 4320
   atgtcattgg tataatcctg ttttctgtga ttatcctttt ggactttacg gttcttttct 4380
   tgggtgctct agataatctg tcccggtctg tctctattgt gtttgtttgt tttagccttt 4440
   atattgagat gtagttaaca taccatacaa gttatccatt taaagtgtgt aatttgatgg 4500
   tttttagctt attctcagag ttgtcccaac tgtcacgaca tgcaatttga gaatattttt 4560
   attacttgag aaagaaaccc ttagcagtta ctcgccattt ctccccaact tccttacgca 4620
   accactaatc tactttctgt ctatagattt ggctattcta cacatttcat atgatagaat 4680
   gaaatagaat cgtacactat gtagtcttca gtgactggct tcttaagctt accataatgt 4740
   ttttgatgct catctgtgtt ccgttgtgtc caagcccagt gtttggagct tgggtgggag 4800
   atgattgcaa tgttgacctc ctgtgatgtt ggggctccat tgggaggctt tctggtggtc 4860
   agccagttga ggtgtgagaa gtgacgcttg ctttcctggt ggccctgtct tgttctttgc 4920
   tgtcttggtt tcctttgtgc tggcacttca gggctgcttt tgtcagacag cccagtttcc 4980
   atagcagata caggctttgt gcttggcccc agagaatttc cctcatctta agtctgtgcc 5040
   tagttcagca ggagctaaac ccttagaggg attccttttt cagagccagg aaccattgcc 5100
   ggatgatgat gaggaatttg agctcccgga gtttgtggag cccttcctga aggacacacc 5160
   cctctataca gacaatacag ccaatggcat tgccctgctc tgggccccgc ggcccttcaa 5220
   cctacgctct ggtcgcaccc gtcgggccct ggacataccc cttgtcaaga actggtaagg 5280
   tttttccctg tggcaggggg aatgatagtg gatgtgtagg agaaaagact ccaactaggt 5340
   gggcatgtag cctttttttt aaattctttt tttttttttc ccataaaaat ggtagacaac 5400
   ccaaggcttg cagcctttag cagagtgtct ggaagaagag atggccaaac tctggtgggc 5460
   ttatttttcc aggtatcggg agcattgtcc tgccgggcag cctgtgaaag tgagggtctc 5520
   ctaccagaag ctgcttaagt actatgtgct gaatgccctg aagcatcggc cccctaaggc 5580
   tcaaaagaag aggtaaggtg ccccaggggc cctgcgcaga cttctgttcc agagatatca 5640
   gtaggctgac tcttactctc catgttccca cctcaggtat ttgttccgct ccttcaaagc 5700
   caccaaattc tttcagtcca caaagctgga ctgggtggag gttgggctcc aggtttgccg 5760
   ccagggctac aacatgctca accttctcat tcaccgcaaa aacctcaact acctgcacct 5820
   ggactacaac ttcaacctca agcctgtgaa aacgctcacc accaaggtgc ctgcattgga 5880
   ccctaggggc ctcctgggct tcaaggacgg acagttgttt tcctctggtt actgatgaag 5940
   gcatacatca gttgggtcca ttgctcattc acgttctgtc tgttttttgc atcttctcca 6000
   ggaaagaaag aaatctcgtt ttgggaatgc tttccacctg tgtcgggaag ttctgcgttt 6060
   gactaagctg gtggtggata gtcacgtgca gtatcggctg ggcaatgtgg atgccttcca 6120
   ggtaaggtta ggtattccca acccctgcct gggtgaggac gctggaggaa gcctgtgctg 6180
   accgtggagc tggtttaagc tccaggtctg atggggcaat tgtattgcag ctggcagatg 6240
   gattgcagta tatatttgcc catgttgggc agttgacggg catgtatcga tacaaataca 6300
   agctgatgcg acagattcgc atgtgcaagg acctgaagca tctcatctat tatcgtttca 6360
   acacggtgag accttgactt tactgacttt cttttttttg agacagagtc ttactcttgt 6420
   ctcttgggct ggtgtgcaat ggcacgatct tggctcactg caacctccgc ctcctgagtt 6480
   caagcaattc tcctgcctca gcctcccgag tagctgggat tacaggtgcc cgccaccacg 6540
   cctggcaatt ttttttttgt tttgttttga aactgagtct ttctctgttg ccccagctgg 6600
   agtacaatgg cacgatcttg gctcaccaca gcctccgcct cccgggttga agtgattctc 6660
   ctgcttcagc ctcccaagta gctgggacta caggcgcgca ccaccatgcc tggctagttt 6720
   ttgaaatttt agtagagatg gggtttcact gtattggcca ggctggtctt gaactcctga 6780
   cctcaggtga tccacactcc tcggcctccc aaagtactgg gattaccagc gtgagtcacc 6840
   gtgcccagcc tcactggact taactttctc agatcttttt tcatgtatac atttagacag 6900
   ggctctttcc agggacctat ttggagagca gctatgactg tgtccttact gctcctttaa 6960
   attcacatgg gcgatcttag ttcctggttt ttgccttaag gtttctcatt tacccttttg 7020
   ttttagatgt tgaggtccca gagttttttt ttctttttac ccctctactg ccatacctac 7080
   attcctctgt atgtatatta tacatatatg ccccacctct gcccaggttt ctattgttgt 7140
   ttaaatgcat tgctaacttg tattcttaac ttctgcttgg gatcgtaggg ccctgtaggg 7200
   aagggtcctg gctgtggctt ctgggctgcc ggttggcgag tctggctctt tttcatgcgt 7260
   ggcattaccc ctttattaga gcgatggctt ggcaacctcc tggcccggca gtttgaaggt 7320
   gagtggtttt ctctgtgtcc aacttctcct gttattgctt caaaagtacc tgcccctgtt 7380
   tgagtctgtt ccctgctctt gtatgagcac tcttcctact ggattgcctt taggtccagt 7440
   tctgaaatga gtctgaccta gtattctatt tcttgggctt ccttctgggg ccttgagagg 7500
   cagtcatgca tttgcaggag tggaaattcc tcagaagcat agttgtgaat gtttaaggta 7560
   gctcgtcttt ttgcatatgc tgggcacagg gacgctttgt taatacctaa ttttattaat 7620
   aaccataatg agtgaggagc atgaaggtag agacccttat tgaaagcaag gaatcttcct 7680
   tgtaatcccc ttgggacact ctacttccag gtcgacactc aaagggggtg gcaaagacag 7740
   taacaaagca gcgagtggag tcacattttg accttgagct gcgggcagct gtgatgcatg 7800
   atattctgga catgatgcct gaggggatca agcagaacaa ggcccggaca atcctgcagc 7860
   acctcagtga agcctggcgc tgctggaaag ccaacattcc ctggaaggtg ggtgtctctt 7920
   tatttccttg gaggcgtaaa catccgtctt tagggtagga ttaccacttc gggaatcatt 7980
   agggggctat ctggctccaa tctcttttct tttccctttt agacagggaa aagaagcacc 8040
   atgttgctta ggctggcctc aagctcctga gctcaagcga tcctccttag cctcctgagt 8100
   agccgggact acagatgtgt gccacagtgc ctggctggat cctggcttga ttgtgctttt 8160
   tggactttta ggtccctggg ctgccgacgc ccatagagaa tatgatcctt cgatacgtga 8220
   aggccaaggc tgactggtgg accaacactg cccactacaa ccgagaacgg atccgccgag 8280
   gggccactgt ggacaagact gtttgtaaaa agaatctggg ccgcctcacc cggctctatc 8340
   tgaaggcaga acaggagcgg cagcacaact acctgaaggt tgggcagagt aggctgggct 8400
   gggtgtgagt ggtgggggag tgggcaagag ggaggactgt agagttgtga ctgaaccttg 8460
   ttctggcagg atcgggctgt agcgggaggc taatttcatg tccaccttac aggacgggcc 8520
   ttacatcaca gcggaggaag cagtggcagt atataccacc acagtgcatt ggttggaaag 8580
   ccgcaggttt tcacccatcc cattcccccc actctcctat aagcatgaca ccaagttgct 8640
   catcttggca ttggagcggc tcaaggaagc ttataggtga gtagtagggt agataggttc 8700
   ctgctgggaa ggtgacagca gggaatggcg tcatcctcag agtccaaaga aagaaggtgt 8760
   agccccagtc tttcctggtt ttacttcctc ctagttttcc cattcaccct tcccattgct 8820
   tttgtcagtg tgaagtctcg gttgaaccag tctcagaggg aggagctagg tctgatcgag 8880
   caggcctacg ataaccccca cgaggcgctg tcccgcatca agcgtcacct cctcacacag 8940
   agagccttca aagaggtgag tgaggcctcc caagttttct ttttggttct accaaaggta 9000
   gcagttgcct ttggttctac caaaggtagc agttggtgga ttggtgtgaa ccagcctcct 9060
   tgtcccacag gtgggcattg agttcatgga tctgtatagc cacctcgttc cagtatatga 9120
   tgttgagccc ctggagaaga taactgatgc ttacctggac cagtacctgt ggtatgaagc 9180
   cgacaagcgc cgcctgttcc caccctggat taagcctgca gacacagaac cacctccgct 9240
   gcttgtttac aagtggtgtc aaggtagaac tttcatgaga tcgccctgtg aggaacgggg 9300
   caggaattta gttctacgtc aaaaatctca tctgggtagc atgattggtt tactctcttg 9360
   atgcgtggta gtaaagggaa gtagtgttcg tgttaaactg aggaaaagcc catagtcaaa 9420
   gcaatccaaa tgaatacgtg gaatcatagt gggattggtt atggaaggta atgatagttc 9480
   ctgtggggct agtcttgcaa gaggatttct aacctgctgg agacctgcct tgctactcct 9540
   caggcatcaa taacctgcag gacgtgtggg agacgagtga aggcgagtgc aatgtcatgc 9600
   tggaatcccg ctttgagaag atgtatgaga agatcgactt gactctgctc aacaggctgc 9660
   tgcgcctcat cgtggaccac aacatagccg actacatgac agccaagaac aacgtcgtca 9720
   tcaactataa ggtgcgtctc aggcgcagta gcatggaatt gtgtgtggag tgcttgtctg 9780
   gcccactgac tcagaatttg ctgaggagga ggctgggtgc ggtggctcat gcctgtaatc 9840
   ccagcacttt gggaggctga ggcgggcaga tcacgaggtc aggagttcga gaccagtctg 9900
   gccaacatgg tgaaaccccg tctctactta aaaaaataca aaaattagtt gggtgtggtg 9960
   gtgggcgcct gtaatcccag ctacttggga ggctgaggca ggagaatcac ttgaacccgg 10020
   gaagtggagg ttgcagtgag ctgagattgc gccagtgcac tctcagcctg ggtgactgag 10080
   cacaactccg cctcgggagt ggaaaaaagt agaatttgct gaggggagaa caaaagaggt 10140
   taactctgga gaatcctggg gcatggctca cattcttaag aattgtagtt ctttctttct 10200
   aggacatgaa ccatacgaat tcatatggga tcatcagagg cctgcagttt gcctcattca 10260
   tcgtgcagta ttatggcctg gtgatggatt tgcttgtatt gggattgcac cgggccagtg 10320
   agatggctgg gccccctcag atgccaaatg actttctcag tttccaggac atagccactg 10380
   aggctgccca ccccatccgt ctcttctgca gatacattga tcgcatccat atttttttca 10440
   ggtgagggct ttcctggatt cctagccttg cagggtactg aggcatgtgc tttgcctctg 10500
   aaatcagcta ttgtacctga gttgacttaa ctgctgttgg ggctttaaaa tggaatgaag 10560
   cggccgggtg cggtagctca cgcctgtaat cccagcactt tgggaggccg aggcaggcgg 10620
   atcacgaggt caggagatcg agaccatcct ggctaacaca gtgaaacccc gtctctacta 10680
   aaaatacaaa aaattagctg ggcgtggtgg cgggtgcctg tagtcccagc tactccggag 10740
   gctgaggctg gagaagggcg tgaacctggg aggcggagct tgcagtgagc cgggattgcg 10800
   ccgctgcgct ccagcctggg gaaagagcga gactgtctca aaaaataaat aaataaaaat 10860
   aaaatggaat gaagcccggg gagggtgggg gtaggatggg gtggggtctc cgtctattga 10920
   gctggaggac ttgtgggtgt cccaaatctc agtgtactgg ggcctcagca gtgtacctgg 10980
   tctggtgtag gttcacagca gatgaggctc gggacctgat tcaacgttac ctgacagagc 11040
   accctgaccc caataatgaa aacatcgttg gctataataa caagaagtgc tggccccgag 11100
   atgcccgcat gcgcctcatg aaacatgatg ttaacttgtg agtctaggtt ggggctgtgt 11160
   gagctggaag gacataagct aagggcccag agagggcata cctgaggctg cgtcctgggt 11220
   gggagaggcg tggctgtgtc aggatgtcat cagttggcct ttgaagggaa gttccttcca 11280
   agaaactgct gtgaccatgt gttctctgtg ggcagaggcc gggcggtatt ctgggacatc 11340
   aagaaccgct tgccacggtc agtgactaca gttcagtggg agaacagctt cgtgtctgtg 11400
   tacagtaagg acaaccccaa cctgctgttc aacatgtgtg gcttcgagtg ccgcatcctg 11460
   cctaagtgcc gcaccagcta tgaggagttc acccacaagg acggggtctg gaacctgcag 11520
   aatgaggtac agcctgggcg gggctgggag tgacatgaac gcgcctgcac ttcctgggtc 11580
   agtcaccgga gtcttgttgg gtggaaagac ttgagtggtg ggcacagttg gctcttgctt 11640
   ctgtgggagt tcctcggaca tgttaacctt gactttgtgg tttaggttac taaggagcgc 11700
   acagctcagt gtttcctgcg tgtggacgat gagtcaatgc agcgcttcca caaccgcgtg 11760
   cgtcagattc tcatggcctc tgggtccacc accttcacca aggtacaggg ccgtcatgcc 11820
   cagcttccct cttgtcctct gctgtcctct gctcatttca cacccctttc acttcgtccc 11880
   ctgctggctg cttccttctc ttctttgctt agtgtgccag tttccatgct ctgtgccata 11940
   gtggcttata gtatgtcagg tcacttcatc gttattttct gagcgttcct tgtagaaatt 12000
   gtgtagcctc cttcttgtgg acttgtgatg attctaaatt aaggcaagag taaaagttgg 12060
   atttattatt ttgaacagaa aagcattatt tttgcatttt ctcaggtgct tatctaagtc 12120
   atgttaacca gtggcataga gaacctgtga aaaagcagcc ctcataggca tctcggggtt 12180
   cgtttctcct tcacttacct tcagaggccc agccgaaggc aaccggaatt gagaaagaaa 12240
   aatctgagga attttgaact acgtatattt aaggttttgc aaaatttaaa aagttacctc 12300
   ggccgggcac ggtggctcac gcctgtaatc ccagcacttt gggaggccga ggctggtgga 12360
   tcacctgagg tcaggagttt gagaccagcc tgtccagtgt ggttctgaaa cctcgtctct 12420
   gctaaaaata caaaaattaa ccgggcctgg tggccggcgc ctgtaatcgc agctacttgg 12480
   gaggctcagg caggagaatt gcttgaaccc aggaggcgga cgttgctgtg agtcgagatc 12540
   gtaccattgc actctagcct gggcgacaga gcaaaaactc tatctcaaaa tacaaacaaa 12600
   caaaaaaaag ttaccttact ctggtttcca ttagagtaac tcttagctat aacatttttc 12660
   ttgcattccg accttcatat ctctaaatgt gcggcatttg catgcgcaga atgttttctg 12720
   ttttttagtt aagtctgtta aggatacgtt ctgttatgtt ttagaactat tgcattacgg 12780
   ttgagtttcc tttatccaaa atgcttggga ccagaaatgt tttggatttc agattttctt 12840
   ttttaatatt ttcattattt ttattagaga cagggtctca ctgtcactta ggctgaaggg 12900
   cagtggcgct atcatagctc actgcagctc aagtgatcct cacacctcag cctcccaagt 12960
   atctgggacc acaggtgcat acgccaccat gcctggctaa tttttaattt tttttttttt 13020
   tttttttttt ttgagatgga gtcttgctct gtcacccagg ctgcagtgca gtggcgcagt 13080
   cttggctcac cgcaagctcc gcctcccagg ttcacgccat tctcctgcct cagcctcctg 13140
   agtagctggg actacaggcg cctgccacca cgcccggcta agtttttgta tttttggtag 13200
   agaaggggtt tcaccgtgtt agccaggatg gtctcgaact cctgaccttg tgatccgcct 13260
   gcctcagcct cccaaagtac tgggattaca ggcgtgagcc accacacccg gcctgatttt 13320
   taaatttttt gtagagctgg gatctcactc tcttgtccag gctggtctca gactctggat 13380
   tcaagcagtc ctcctgcctt agcctctcta atgctggatt ataggtgtga gtcaccgtgc 13440
   ccagccttga aatattttca ttatacttac aggttcagca tccctaatcc aaaatttgaa 13500
   atgccccaat gagcatttcc tttgagcgtc atgttggtgc tcagaatgtt gggactcaga 13560
   aagtttcagc tttttgtgag cattttgggt tttagattaa cctgtatgtg ggaatctgtg 13620
   agagcactag agttagtgat tgctctagcc cagtctggaa gtggaagagg aaaggattcc 13680
   tgaaagaagt agcatgtgat gagacaggtg agggagagca ttcctttagc cagaagtgtg 13740
   gcgatgtaac tagcttgtgt acatgggacc atggaaagtt caaggtttgc tggagtaaat 13800
   tgtggaattg ggaatgttaa gagatgaggc tggataaata gtggagagcc atatcatgga 13860
   gggcatcgca caccttagga agaatcatat aagggtctta agcacagagt gactttgtca 13920
   gatgtgtttt tcttcttttt tttttttggc ttcccacact acagatgcag actttgtcaa 13980
   atttctgtgt tagatgattc actcttgcag ctgtgagaaa gatggacctg aaaggatagg 14040
   atgggagacc aggtttgggg accactgaag aagtccagat aaaaatgatg agagcaggcc 14100
   aggcgcgggg gctcacgcct gtaatcccag cactttggga ggcccaggca ggtggatcac 14160
   ctgaggtcag gagtttgaga ccagcctgac caacatgggg aaaccccatc tctacttaaa 14220
   atatgaaaat tagccgggcg tggtggtgca cacctgtaat cccagctaca caggaggctg 14280
   aggcaggaga atcgctggaa cccgggaggc ggaggttgca gtgagccgag atcccaccag 14340
   tgcactccag cctggcaaca gggtgagact ctgtctccaa aaaaatacat aaatcaataa 14400
   aaatgatgag agcactgaaa gtagaggtag gaagagggga acagatttga taagaagtta 14460
   gcagataaaa tcagcagaac tttgtggtcc tgggatgctc aaagaaaagt caaaaatgat 14520
   gtgcagattt tggcatgggt gactaggtgg gtgttagtgc caccaactgg aagaccacat 14580
   gaacaagaac aggtttagga tcgaagataa agaaatctag tttaggatat ataaagtttg 14640
   aagttcctga gaacattcag atggagattt ccccaggtat ttatttatac cttatttctt 14700
   cagttccaag atacatattt aagtacatat atacacatgt gtagattcct gattccttct 14760
   gctcccttag tggtgcctct ttaattgatg atctgttaga tttgatttaa aaagtatgag 14820
   tgagacattc cagggagaga aatgaaacta gaaaggtaag agcagtcagg gatagcttag 14880
   aggttcctag gtgagaagca atctgaagaa acatggagaa caggtctgga ttaaagatgt 14940
   agatttcgga gcctatccac aaaaggtcaa aagctcaaga atgagtgaca tcacccaggg 15000
   acaatgtgga gtgagaagag gagagggagg aatttggaaa tgtggggaat actaagatga 15060
   agtgtggtca gaacctgtgg gagggtgccg ggtcttggaa ccaaggcctt caggaacacg 15120
   tgtccagaat tggggtgatg ctgagcagtg tcagttgcct aagagcattt cagttagatg 15180
   aggactgaaa agtatccatt ggatttgaca gttaggacgt cattggtgac cttagcagga 15240
   gctgttacag aaatagtagg gatggaagcc agatggcagt gggtgaggct tcatgggaag 15300
   tgagtgagtg gagacagcaa ggacaatctg tttgtcagtt gaagagaagg aaggacatca 15360
   gatgccacct agagggagct gtaagtcagt gcaggtgttt ttgaatgaca gtgacttgag 15420
   cgtgttttaa ttaaggagcc cagtagcagt tgagtgtaca agagaaaagt gtggaattgg 15480
   ctggacacag tggctcacgc ccgtaatccc agcactttgg gaggccaagg tgggtgaatc 15540
   accaagtcag gagttcgaga ccagcctggc caacatggtg aaaccccgtc tctactaaaa 15600
   atacaaaaat tagccaggcg cggtggcggg tgcctgtaat ctcagctact cgggaggctg 15660
   aggcaggaga attgcttgaa cccgggaggt agaggttgca gtgagccaag atcgtgccac 15720
   tgcaccccag cctcggcgac agagcaagac tccgtctcga agaaaagaaa agtaggtaat 15780
   tgatgagcag aggtgcagag ggtctcccta gggatagaat aaagagcaag cagaggaaaa 15840
   atcctctgaa cctgacagga aagaggtctg gaaagatgtg gatatagatg aggttagtgg 15900
   gaagctataa caggattttg agtttttcag cttttcttgg gctgcagtta ggggaaagag 15960
   ttgtgcatag tgaatttcat ggtattaggg tatgatgatt gctgaagaaa ctgagaggga 16020
   ttggaaggtg tgttgggagc tcagctgggt tttgggaaga caactctgag catttgtttt 16080
   tcccagaatc acccagctac tcagtgacag ggatgcagaa ggtggataga ttacactggt 16140
   gattaaggaa tagagctctg ttagaagaca gttgtagagt ggtaaggcat tgaatgcaag 16200
   ggaatcaaga gtgcgggacc cagccggccg cggtggctca tacctgtaat cccagcactt 16260
   tgggaggccg aggcgggtgg atcacgagtt caggagattg agaccatcct ggctaacgcg 16320
   gtgaaacccc gtctctacta aaaatacaaa aaattagctg gatgtcgtgg cgggcgcctg 16380
   tggtcccagc tactcagggg ggctgatgca ggagaatggc aggaacctgg gaggcagagc 16440
   ttgcagtgaa ccgagatcgc gacactgcac tccagcctgg gtgacagagc aagactctgt 16500
   ctcaaaaaaa aaaaaaaaaa aaaaaaaaga atgctagacc ccattgagat actggaagag 16560
   gggagaggag agagagcaga agaggccatt ggcctgggaa agaattgtag gcatcaggga 16620
   actggatatc agaagttaga ggctgaggag tggaggagtg tctgagaggg taaggatgca 16680
   gttgactcta gtggttagaa gcacaggctc tggagtcaga tggggttcac atcatatctt 16740
   tgtcatatta ctagttggga tcttgggaat attgcccatg ctgggcctca attccctcat 16800
   ctgtaaaata gggatactga taggacctag gtcagacggc ttttgtgaga attaaatgaa 16860
   acggggtatt tagtaagcca tattacttgg taaattgcat gataaagctg ggcatggtgg 16920
   cttatgcctg caatcccagc acttttggaa gccgaggtgg gaggactgct tgagcccagg 16980
   aatttgagac cagcttaggc aacatagcaa gatcctgttt atatttaaaa ataaataaat 17040
   aggccgggtg cggtggctca cgcctgtaat cccagcactt tgggaggccg aggtaggtgg 17100
   atcacgaggt caggagttca ggaccagcct ggccaatatg gtgaaacccc atctctacta 17160
   aaaatacaaa aattagccag gcgtggtggc acatgtctgt agtcccagct actctggaga 17220
   ctgaagcagg agaaccactt gagccccgga ggtggaggtt gcggtgagct gagattgcgc 17280
   cactgcactc cagcctgggt gacaaagtga gactccatct caaaaaaaaa aaaaaaaaaa 17340
   aaaagctatg tcatgggcaa gttctataac ttgtttttta tgaagacata agaggtataa 17400
   catatataaa tttcttacca tggtacctag tacacgtaga cctgagtaaa tattattttc 17460
   cttcttgcct ccccttatcc cccaggaatt tctgctattt agtaaaggaa cactgggctc 17520
   ctcaggaatt tatgcctcct ccccttcagg gatagagcac tgttaactac aacagaaata 17580
   ctgtgttcca ttacacacat cagcaattcc aagtgcagtc caagaattta ctaaacctaa 17640
   tgggaatcat ccacttgcct ttaccccagt atctagccta tcctggcccc tggccctcct 17700
   tttttttttt gagagggagt cttgctctat tgcccaggct ggagtgcagt ggcacaattt 17760
   cagctcactg cgacccccgc ctcccgggtt taagtaattc tcctgcctca gcctcctgag 17820
   tagctgggat tacaggtgcc tgccaccatg cccggctaat ttttgtattt ttagtagaga 17880
   cagggtttca ccatgttggt caggctggtc tcaaactcct gacctcatga tccaaccgcc 17940
   tcggcctccc aaagtgctgg gattacaggc atgagccacc gcacccggcc cccctccttt 18000
   ctgtagatct gcctggttgt ctcatcctgt ggacatgcga gatgctcagt ctagctttca 18060
   tagtagggtt tgagctgtta tctcacctgc agtcttttct ccttgcttgt ctgaatacca 18120
   tttgctcaag gcagattact atagtaactc cagccaacgc actctttcac ctcaataatt 18180
   gttcagaaga cattttttgc cttaaagtct tgactatatc atttggtatt ttgtggtttg 18240
   ctatcttgtt ttattcagct tttcaaagaa acagctttgg tttcattaat ttttatcgtt 18300
   tttctctttt ttgttgcatt gatttttttt ttttttgaga tggggtctct atgacccagg 18360
   ttggtgtgca gtgtcgcagt ctcagctcac tgtaacctct gcctcccagg ctcaggagac 18420
   cctcccacct tagcctccca agtagctggg accacaggca catgccacca tgcctggctg 18480
   atttttcttt gtattttgga tacagacagt ttcgccatgt tgcccaggct ggtcttgaat 18540
   tcctgagctc aggtaatcca cccccctggg ctcccaaagt gctgggattg taggtgtgag 18600
   ccaccacgcc tggccttgtt gcattgattt cttctcttat cattgccatc cctttgctag 18660
   tttgttaagg taaaaggtta gattatcaat tttggacctt tctctttttt tttttttgag 18720
   acagagtctc gccctgtcac tcaggttgga gtgcagtggc gcgatctctg ctcactgcaa 18780
   gctccgtctc ccgggttcac gccattctcc tgcttcagct cccaagtagc tgggactaca 18840
   ggtgcccacc accacacccg gctaatattt ttttttgtat ttttactacg acgcggtttc 18900
   accgtgtttg cgaggatggt ctcgatctcc tgaccaccac ctgatcagct tgcctcggcc 18960
   tcccaaagtg ttgggattat aggtgtgagc caccgcacct ggcctggacc tttctctttt 19020
   tctttttttt tttttttgag atggagtctc actctctcac ccaggctgga gtgcagtggc 19080
   gcgatctcag ctcactgcaa cctccgccct ctgagttcaa gcgattctcc tgcctcagtc 19140
   tcccgagtag ctaggattac agatgcctgc caccgcaccc ggctaatttt tttgtgtgtt 19200
   tttagtagag acagggtttc accatcttgg ccaggctggt cttgaactct tgacctcgtg 19260
   atccacctgc ctcgtcctcc caaagtgctg ggattacagg cgtgagccac cgtgcccagc 19320
   tgacctttct cgtttttttt tgtttgagac ggagtcttgc tctgtcgccc aggctggagt 19380
   gcagtggcgc aatcttggct cactgcaact tccgcctcct gggtttgtgt cattctcctg 19440
   cctcagcctc ctgagtagct gggactatag tcgcctgcca ccatgctcag ctaatttttt 19500
   gtatttgtag tagagacggg tttcaccgtg ttagctggga tggtcttgat ctcctgacct 19560
   cgtgatccac ccacctcagc ctcccaaagt gctgggatta caggggtgag ccaccgtgcc 19620
   tggccaacct ttctcttttt ctaatagtat tattcacttc tgtaaatttt cctctcagca 19680
   ttgctttagc tgcattcctc cctttttttt tttttttttt tttgaggcag agtctcacat 19740
   tttcgcccgg gctagagtgc agtggcacga tctcggctca ctgcaacctc cgcctaccct 19800
   gttcaagcga ttctcctgcc tcagcctccc aagtagctgg gattacaggt gcccgccacc 19860
   acacccagct aatttttttt tgtattttta gtagagacag gtttttacca tgttggccag 19920
   gctggtcttg aactcgtgac ctcgtgattc acccgccttg gcttcccaaa gtgctgggct 19980
   tacagatatg agccatcgtg cccggcccca ttcctcacat tttaatatgt tgtgttttca 20040
   tttttattta gttaaaagta tattctaatt tcccttatga gttcatattt caccttaggt 20100
   tgtttcaaat tatgctgttt acatatttgg gggtttccct tattagtttc tgctttgatt 20160
   ccattatgat taggaaacat gttgtatatg atcttagttt taaaaaatgt atcaagattt 20220
   tttttttaat aattaattca tttagaggtg ggatctcact cttgcctagg ttggacccaa 20280
   actcctgggc gcaagcgatc ctcctgcctc agccttccaa agagttggga ttacgggcat 20340
   gagctactgc acctggctga ggctgacttt ttgatgtcct agcgtacagt ctctcttggt 20400
   gcctgtccta tgtgtgctgg aaaatatctg ttaatctgta ttctgctatt gttaggttga 20460
   gtgttttaca aacattaggt ccagtcagtt ggttacattg cttaagtctt ctatatcctt 20520
   agtgatattt tttgtatact tctatcgatt actgaggagt attagaaatc tctagtaatc 20580
   ccagcagttt gggaggctga ggctggtgga ttgcttgagc acaggagttc gagaccagcc 20640
   tgggcaacat agcgaaaccc catttctaca aaaaaaatac aaaaattagc tgggtgtggt 20700
   ggtacacgcc tgtagtccca ggtgcttggg aggctgaggc gggagagtct cctgagcctg 20760
   ggaggtggag gctgcaatga gtggagatgg tgccattgca ctctagcctg ggcaacagag 20820
   caagaccctg tcttgcttga gagaaagatc caactacagc tgtgagttta ttgtctttca 20880
   ggtccatcat ttttgaaatt ctgttattag ttacataatg tatttaggat tattaggtct 20940
   tctgaacgag ttgactactt tatcgtatca aatatccctc tgtattcctg ataacattcc 21000
   atggtctgaa gtctttgtct taatcagtag tgccactcca gctttgtttt gtgtttgcat 21060
   gggtgtattt ttccatcttt ttactgttaa tctgtgtctt tgtaactaaa gtttatttcc 21120
   tatagacagc atatagttgg ctcttttttt tttttttttt tttttttttt ttgagatgga 21180
   gtctcttcct tggttgccca ggctggagtg cagtggcatg atcttggctc agtgcaacct 21240
   ctgcctcccg agttcaagcg attcttctac ctcagcctcc caagaaactg agactacagg 21300
   cgcgtgccac cacgctcagc taatttttgt atttttagta gagacggggg gtctcaccat 21360
   gctgtccagg ctggtttcga actcctgacc ccgaggcccg cctcagcctc ccagagtgct 21420
   gggattacag gtgtgagcca ccatgcccgg cctttttatt taaatccaat ttgacaatgt 21480
   ttgccttaca aatggtctaa atacctcaat taaggttaat gtaactatca atgtggcaat 21540
   gtagctcagt ttaaatctac cgtcttactc tttttttttt aaattatttt ttctgctttt 21600
   ctcttgtcct gccttcttct gaatttagtt gttttgtgac tttatctcca cttttcactt 21660
   atttggctac atggtttttt tcttattgtt tgctctaagg tttacagtat atacatagct 21720
   cacttactgt gatctacttt gaccattagt ctactacgga tcacttcaca tatagtacaa 21780
   gaaccttaga ataatacact ttaagttcca ctcccactct tagtagtatt tttgtcataa 21840
   cattttaatt gtacatgtat tataagtgct ataatacttt gttactcttt ttgctttgaa 21900
   cagcctttat cttttgtaga aattgcaaaa tgagaaaaaa tatttttttg cattttaccc 21960
   acatacttac catttctggt gttcttcact agtttgtata gctccatctg gtgtgctttt 22020
   ttttttcttt ttcttttctt tttttttttt ttgagacaca gtcttgctct gtctcccagg 22080
   ctggagtgca gtggtgcgat ctcagctcac tgcaacctct gcctcctggg ttcaatttct 22140
   gtatttttag tagagacggt ttcatgttgg ccaggctggt cttgaactcc tgacctcagg 22200
   tgatccaccc acctcggcct cccaaagtgc tggcatgaca ggcgtgagcc accacacctg 22260
   gcccagggga accatttttt tttttttttc ccctgagatg gagtctcgct gtgttgccca 22320
   ggctggaatg cagtggcaca atctcagctc actgcaacct ccacctccca ggttcaagcg 22380
   attctcttgc ctcagccttc tgagtagctg ggattacagg cgtctgccac cacacccggc 22440
   taattcttgt atttttagta gaaacagggt tttgccatgt tggccaggct ggtctccaac 22500
   tcccgggctc aagcagtcct cccaccttgg cctcccaaag tgctgggatt ataggcatga 22560
   gccatcgcgc ccagcctcat tgtgttttgc tttctatttc cctagtgact aatgatgttg 22620
   aactcaaatc tttctttagt agtagggata aaactaactg catcaagtag tctccaccct 22680
   ccccgcccct ggctcatctg taatttactt ttttttctac tacctttaga ttgtgaataa 22740
   gtggaataca gctctcattg gccttatgac atactttcgg gaggctgtgg tgaacaccca 22800
   agagctcttg gacttactgg tgaagtgtga gaacaaaatc cagacacgta tcaagattgg 22860
   actcaactcc aagatgccaa gtcggttccc cccggttgtg ttctacaccc ctaaggagtt 22920
   gggtggactc ggcatgctct caatgggcca tgtgctcatc ccccaatccg acctcaggta 22980
   cagcctggtt caacgtattt ctaaacccta ttgctcaggc cctaagggaa gtgggtattt 23040
   ctttagtttt aggctttatt ttttctgtaa tcttggttgt acacccaaac aggtggtcca 23100
   aacagacaga tgtaggtatc acacactttc gttcaggaat gagccatgaa gaagaccagc 23160
   tcattcccaa cttgtaccgc tacatacagc catgggagag cgagttcatt gattctcagc 23220
   gggtctgggc tgagtacgca ctcaagaggc aagaggccat tgctcagaac aggtgggcac 23280
   ctaggagggc atgccaaccc tggggcaggg gcagtggtgg cctttgaacc gtgtggagtt 23340
   cagggtcaga gcatgccatg gttgtatgga ggtgagaagg ctggttctac tgaccctaat 23400
   tatttttggc caggaaggag tgctgagctc ctcatatgag atcacgtttt gacttgaatc 23460
   tttctttttt tggaagacgc ctgactttag aagacctaga agattcatgg gatcgtggca 23520
   ttcctcgaat caataccctc ttccagaagg accggcacac actggcttat gataagggct 23580
   ggcgtgtcag aactgacttt aagcagtatc aggtatgtag agggagcaga ttttccctga 23640
   gtcaggaaaa tccatggcta tccccgaaac ttggggcaat aggcacccag aagcttgacc 23700
   gtgcctgcct tgccatctag gttttgaagc agaatccgtt ctggtggaca caccagcggc 23760
   atgatgggaa gctctggaac ctgaacaact accgtacaga catgatccag gccctgggcg 23820
   gtgtggaagg cattctggaa cacacactct ttaagggcac ttacttccct acctgggagg 23880
   ggcttttctg gtgaggattc tcctctctta gagggacctg tcacaaccct ttgagctcat 23940
   ctcttgtatg cctctgtgat ttcctgtggt gtttcccaga cagccacctc caggaggtta 24000
   gcagtgtgct gggaaatggc aggatatcac ctcacctgct tctcttgctc cccagggaga 24060
   aggccagtgg ctttgaggaa tctatgaagt ggaagaagct aactaatgct cagcgatcag 24120
   gactgaacca gattcccaat cgtagattca ccctctggtg gtccccgacc attaatcgag 24180
   ccaatgtgag tgtgattgac actggagagg ggaagctaaa gacagttgct gcttcttgcc 24240
   ttggttatgt cgtgagcgcc cgagtctctt gatctctaat gtcacattgt cgttttcctg 24300
   gcaggtatat gtaggctttc aggtgcagct agacctgacg ggtatcttca tgcacggcaa 24360
   gatccccacg ctgaagatct ctctcatcca gatcttccga gctcacttgt ggcagaagat 24420
   ccatgagagc attgttatgg acttatgtca ggtgggctgg aatcgagggg agagggtact 24480
   gtagagagtc agctcaggtg gaattgaggg gagagggtac tgtacagagt cagctcaggt 24540
   ggaatcgagg ggagagggta ctgtacagag tcagctcagg tggaatcgtg gggagagggt 24600
   actgtagaga gtcagctcag gtggaatcgc agggagaggg tgctgtacag agtcagctta 24660
   ggtggaatcg cggggagagg gtgctgtaca gagtcagctc aggtggaatc gcggggagag 24720
   cgtgctgtac agagtcagct cagcttgctc ttttgcatat gggaaatcca ccctggaagc 24780
   tctgaggatg agactcctat tgcctcatta tcttattgta caaactctga ttttaattgg 24840
   gcttccttaa caagtcttaa tctctccatg ttttccttca ggtgtttgac caggaacttg 24900
   atgcactgga aattgagaca gtacaaaagg agacaatcca tccccgaaag tcatataaga 24960
   tgaactcttc ctgtgcagat atcctgctct ttgcctccta taagtggaat gtctcccggc 25020
   cctcattgct ggctgactcc aagtaagtgc ctcaggaccc agccctaggc agccaggaca 25080
   ctttcgtttt cctgttcttc tagccctgca actttaggaa ttgtcctgtc tgcctttgtt 25140
   tcaaacttgg agccagtgct acgcttggag cctgtcaaca cccttagtca gatctgctga 25200
   ttctctgggg tcctgctgac ctggaacaag ttggtggagt gggtgggatg gttttgggat 25260
   ttaagtggtt ctggttctgg ggacattggt tatgcccatg gtttcttaga agcttgaacc 25320
   ctcttcatcc tcagggatgt gatggacagc accaccaccc agaaatactg gattgacatc 25380
   cagttgcgct ggggggacta tgattcccac gacattgagc gctacgcccg ggccaagttc     25440
   ctggactaca ccaccgacaa catgagtatc tacccttcgc ccacaggtgt actcatcgcc     25500
   attgacctgg cctataactt gcacaggtga gttgaggctc aggaccatgt attttcaggc 25560
   caggcgcagt ggctcacacc tgtaatccca gcactttggg aggccgcggt gggcagattg 25620
   tctgagctca ggagtttgag accagtctgg gcaacatggt gaaaccctgt ctctaccaaa 25680
   atacaaaaaa ttagctgggc gtggtggtcc acgcctgtag tcccagctac tcagaaggtt 25740
   gaggtgggag aattacttga acccgggagg cggagcttgc agtgaaccga gattgcgcca 25800
   ctgcactcca gcctgggcga cagagtgaga ctccatctca aagaaaaata ataataataa 25860
   taaataaata aataatccgt gtattttcca ttagctccat actttaacgt agtcatactt 25920
   ttttacttta cctaagtgag aggaagtggc ccattcctcc tatgggtcac tgattctctt 25980
   ctccagcctg tatgtacaca tgagcagagt gttgtcagcc tggcgaacag tctttcttcc 26040
   tctgtagagt tggcagcttg tcttgtttct ggggctgtca ttctcgtctt aacttttctt 26100
   gaccctttta tcctcacagt gcctatggaa actggttccc aggcagcaag cctctcatac 26160
   aacaggccat ggccaagatc atgaaggcaa accctgccct gtatgtgtta cgtgaacgga 26220
   tccgcaaggg gctacagctc tattcatctg aacccactga gccttatttg tcttctcaga 26280
   actatggtga gctcttctcc aaccagatta tctggtttgt ggatgacacc aacgtctaca 26340
   gagtgactat tcacaaggtg agtgttagca gcagtgtata ggtgcgggac tctgtacatg 26400
   gctgtgggag agtaatgggc taagtgtgta cacatgctgg gaagaaggca ggatggggat 26460
   gtgttgtgta ggccatgcta acgaatgccg tcctcctcta gacctttgaa gggaacttga 26520
   caaccaagcc catcaacgga gccatcttca tcttcaaccc acgcacaggg cagctgttcc 26580
   tcaagataat ccacacgtcc gtgtgggcgg gacagaagcg tttggggcag gtgagcaggt 26640
   ttaaagatga agaggctgta gaaccatgtt gcctcttatt cttggggtgg acttggtaat 26700
   atttactggt ttattagatg aaaggctgag aacatctcat ttgaacttat ccccaaaata 26760
   ccttgggaag tattctgtgg gaggtccaag agtgtatgag gtgcttcgtt cccttccagc 26820
   tggtctgttt cctgaatttc taaccttgaa cagggctatc aaaaatcatt tttccaaagg 26880
   caacgcatac tctttttttt tttttttttt tttagccgga gtctcgctgt gtggcccagg 26940
   ctggagcaca gtggctcaat ctcggttcac tgcaagctcc gcctcccggg ttcacgccat 27000
   tctcctgcct tagcctcccg agtagctggg actacaggcg cccgccacca cgcctggcta 27060
   attttttttg tattttttag tagagacagg gtttcaacgt gttagccagg atggtctcga 27120
   tctcctgact ttgtgatctg cctgcctctg cctctgcctc ccaaagtgct cggattacag 27180
   gtgtgagcca ctgctcctgg ccagcaatgc atattctctt tttttttttt tttttttgag 27240
   acggagtctt gctctgttgc ccaggctgga atgcagtggc ctgatttcag ctcactgcaa 27300
   gctccgcctc ccaggttcac gccattctcc tgcctcagcc tcccgagtag ctgggactac 27360
   aggtgcccgc caccacgccc ggctaaattt tttgtatttt tagtagagac ggggtttcac 27420
   catgttggcc aggatggtct caatctcctg acctcgtgat ccgcccgcct tggcctctca 27480
   aagtgctggg attacaggct tgagccaccg tgcccggccc agcaacgcat attcttgaaa 27540
   catccacttt gaccaaatct cttctctacc tcacaactct tgctgaatta actcttcttt 27600
   agcttcctag acgttcaaca tctggctggg ctgtatctgt ccagccagtt tctcttcctt 27660
   tacatttata gcatgtttca tcttacattc agtccaggat cttagtagcc caaacccctg 27720
   atgctcatct ccaaatctgt gttttgcttg tatttttctt tctagaatga actcccctct 27780
   ttgctctgtt aatcacagtc cctccttaag acacgttttt ttgtgcagct tttttctgat 27840
   cctagtagcc ttaatttctt ttgttcctga actctgtaaa acagtgtata gtttagcaca 27900
   tactcatgtg tcagcttgtg gtattgtctt gttgaaaggg gctgtcagcc tggtcattgg 27960
   acacggccat ttgatactct ttgtaccttc taagtgccta acatttaaca tcagtgttca 28020
   atagaaacgt agttggatta tttcaaaact aggatattct gttctagttg atctgggagg 28080
   gtagagacta aatttccatt ttcttctctc ttgcttttaa gttggctaag tggaagacag 28140
   ctgaggaggt ggccgccctg atccgatctc tgcctgtgga ggagcagccc aagcagatca 28200
   ttgtcaccag gaagggcatg ctggacccac tggaggtaag agggtggggt ggggtaggag 28260
   aaaagagacc ttgagacctt caggctgttt ctcatcttgg acttgatctt gtcttaggtg 28320
   cacttactgg acttccccaa tattgtcatc aaaggatcgg agctccaact ccctttccag 28380
   gcgtgtctca aggtggaaaa attcggggat ctcatcctta aagccactga gccccagatg 28440
   gttctcttca acctctatga cgactggctc aagactattt catcttacac ggtatgaacc 28500
   tcgggaagag aagatcgctg gaggaggagc gggtttaggg ttaggagaag atcctgtgtc 28560
   aggtttttgc cattttcaca tttttagcct atagattggg tgggaactct gttgactttc 28620
   atgagagttt aagaccttaa agttaaaaat caactcttgg ggccgggcgt ggtggctcac 28680
   gcccgtaatc ccagcacttt gggaggctga gttgggtgaa tcacctgagg tcaggagttt 28740
   gagatcaggc tggccaacat gacgagaccc catctctact aaaaatacaa caaaattagc 28800
   cgagtgtgct gggatgcacc tgtagtccca gctactcggg aggctgaggc accagaatca 28860
   ctggaaccca ggaggcagag gttgcagtga gccgagatgg caccattgca ctccagcctg 28920
   ggcgacagag taagactgtc taaaaaaaaa aaaaaaaagg ccgggcgcag tggctcacgc 28980
   ctgtaatccc agcactttgg gaggccgagg cggcggatca cgaggtcagg agattgagac 29040
   catcctggct aacacagtga aaccccatct ctactaaaaa tacaaaaaat tagccgggcg 29100
   tggtggcagg tgcctgtcat cccagctatt cgggaggctg aggcaggaga atggtatgaa 29160
   cctgggaggc ggagcttgca gtgagccgag atcatgccac tgcactccag cctgggcgac 29220
   agagcaagac tctgtctcaa aagaaaaaaa aaaaattctt gggtttcatg aaaggttagg 29280
   attaaagtgg gagagcctca agcagctgac ctaccccact cttccaaatc ttttcccagg 29340
   ccttctcccg tctcatcctg attctgcgtg ccctacatgt gaacaacgat cgggcaaaag 29400
   tgatcctgaa gccagacaag actactatta cagaaccaca ccacatctgg cccactctga 29460
   ctgacgaaga atggatcaag gtcgaggtgc agctcaagga tctgatcttg gctgactacg 29520
   gcaagaaaaa caagtgagca gtgctggtgg ggacacagac aggaggtcta tatacggttt 29580
   tctggggttt tttggaagca cttaggctta gaagtttctc aagaaggcca ggcgcggtgg 29640
   ctcatgcctg taatcccagc actttgggag gccaaggtgg gtagatcatt tgaggtcagg 29700
   agtttgaaac catcctggcc aacgtggtga aactccgtct ctactaaaaa tacaaaagtt 29760
   agccgggtgc gatggcgcct acctgtaatc ccagctactc gggaggctga ggcaggagaa 29820
   ttgcttgaac ctgggggcag aggttgcagt gagccgagat catgccactg cactccagcc 29880
   tgggcgacaa gagtgagact gtctccaaaa aaaaaaaaaa aaaagacgtt tctcaagaaa 29940
   ttatcttgtc ttagccaggc ttgagtgctc atgctagtaa taccagcact ttgggaggcc 30000
   aaggtgggag gattgcatga gccaggagtt gaaaccagcc tgatcaacaa gagactgacg 30060
   ccatctctac caaaaaaaaa aaatttaaaa caggtgtggt ggtacacgct tgtagtccca 30120
   gcttcttgga ggctgaggca ggaggcttgc ttgagcccgg gggtttgagg ctgcagtgag 30180
   ccatgatgat gccactgtac tccagcctgg gtaacagagc gagactcttg tcttgaaaac 30240
   aaggaaagaa attatcttac agagtctcga ggaagagaga tacagcagtg tcttcgaata 30300
   gtatgggaag catccctgtt ttagggcttc agtctgactc ttggccattg tttctcactg 30360
   ttgccatttc aaacagggca tttctttact gtccatacat gggaagaatt ttgaacatcc 30420
   gagaccctaa gtatccgaga ctgctgccaa cacacacaca caccttcctc ccctcgtctc 30480
   cctccctgtc atcgtggcaa ccaaaattat ccatagggtg acggacaata ccacctctga 30540
   ttaagaacca gtattctagg gtttctgggg tttccatttc tgagaacagt tccatgccag 30600
   agcattgttt tggtcaagga agcgtagggt ttatggatgc taaacagtgg gaaggtgcac 30660
   acgcagtgtg ctgtcccgct tggatctgac gaatcttgga agtgttagtg cacctccgtt 30720
   tcacacttcc tgtagaagca gctcttgtgg attgtctggg gcgtgagtat aggctgtcct 30780
   gtcctaccaa gttacaccct ttccattgag gcagaagtga ccaaggggaa gggatccttg 30840
   taatataacc cacaccatcc ccacagtgtg aacgtggcat cactgacaca atcagaaatt 30900
   cgagacatca tcctgggtat ggagatctcg gcaccgtcac agcagcggca gcagatcgct 30960
   gagatcgaga agcagaccaa ggaacaatcg cagctgacgg caacacagac tcgcactgtc 31020
   aacaagcatg gcgatgagat catcacctcc accaccagca actatgagac ccagactttc 31080
   tcatccaaga ctgagtggag ggtcaggtac tgtcgggcaa gagggtgggg atggaaaggc 31140
   cggctgcact acctaaggtg ctgggctgag cgatgctgac actccctgcc tgtttttagg 31200
   gccatctctg ctgccaacct gcacctaagg accaatcaca tctatgtttc atctgacgac 31260
   atcaaggaga ctggctacac ctacatcctt cccaagaatg tgcttaagaa gttcatctgc 31320
   atatctgacc ttcgggccca agtgagtaag tggactcagc taggccacag tgtgtgccca 31380
   actcattttg tgcctaaaac tcagacctga gattgtctgg aacttgagat gctggtttca 31440
   agattcatgg atgagtaatt atacaaggat agccaaaaca acgaggtggg ttttggcccc 31500
   atgagatagc aaaagctgtg gcagctgaga gaaggtagta attgtagtat tggcctgata 31560
   gtatttggaa gagaacagat atggtcagaa acaaattcct gaccaggtgt gcgtgctggc 31620
   tcatgcctgt aatcccaaca ctcggctggg cacagtggct aatgcctata atcccagcac 31680
   tttgggaggc ctaggtgggt ggatcacctg aggtcagggg tttgagacca gcctgaccaa 31740
   tatggtgaaa ccctgtctct actaaaaata caaaaaatta gccaggcatg gtggcatgcg 31800
   cccgtagttg cagctactag ggaggttgag acaggagaat tgcttgaacc cgggaggtga 31860
   ggtggagctt gcagtgagcc aagattgcat cactgcactc cagcctgggc aacagagcaa 31920
   gaccccgtct aaaaaaacaa aaccaaaaaa aacgtggctg tagtcccagc tactcaggag 31980
   gatgaggttg cttgaacgca agcagtgagc tttgatgacc ccactgcact ccaggctggg 32040
   cacagtggct catgactgta atcccagcac tgtgggaggc cgaggtgggc agatcttttg 32100
   agcccaggag ttcgagacca gcctgggcaa catgacgaaa tggagtctct acaaaaaaaa 32160
   aaaaaaaaaa ctagccaggc atggtggcat acacctgtaa tctcagctac tcaggaggct 32220
   gaggtgggag gatcacctga tcctggaggg tggtgagtgt agtgagctga gatcatgtca 32280
   ctgcctgcta gcctgggtga cagagtgata ccctgcttca aaaaagagaa aagatgcata 32340
   actttactag tgtatcaggg atattatagt ttataaaaca tcattttggg ccaaggtggg 32400
   tgaatcactt gaggtcagga gttcaagacc agcttggcca acgtggcgaa accccgtctc 32460
   tcttaaatat atgaaaatta gcggggcatg gtggcatgtg cctgtagtcc cagctactca 32520
   ggaagctgag gcaggagaat tgcttgaacc caggaggcgg aggtcgcagt gagctgagat 32580
   cacaccactg cactccagcc ttgctgacag agtgagactc catctcaaac ccactattta 32640
   gtggctgtca ggttggcaaa tttgaaaaga ttgttaatat cctgtgtggg tgagtttata 32700
   gtaaacagat cctctttctt tcttggtgat aatgtaaaac ttaaaatagc accaagtgct 32760
   ggttttccag atgttttcag cttgtgaaaa tacactgagc tgcatgtgtg ctcatttgct 32820
   tgtgaatact atgcttaata atcagctggg gagcttgtga gcagtgcaga ttcctggacc 32880
   ccactcagga gattctgatg tggtaagtcc gcgatgaggt ccatgtctaa cattttgaac 32940
   aagcacccca gctgattctg atacaggggg ttctgatcac gttttaagaa actctgtcct 33000
   cgttcttggg ggaaggaagg aaacagaggg ctggcagctc caactctgac ctggtactaa 33060
   gaagagtttt ggttcctgac ccctttgtct ccagattgca ggatacctat atggggtgag 33120
   cccaccagat aacccccagg tgaaggagat ccgctgcatt gtgatggtgc cgcagtgggg 33180
   cactcaccag accgtgcacc tgcctggcca gctgccccag catgagtacc tcaaggtaat 33240
   ggggagtgat ggagcctggg agtgtggaaa agctggtgcg attcctgtct gtggatggga 33300
   ttcagctctg tactcctctt tacctatagg agatggaacc cttaggttgg atccacactc 33360
   agcccaatga gtccccgcag ttatcacccc aggatgtcac cacccatgcc aagatcatgg    33420
   ctgacaaccc atcttgggat ggcgagaaga ccattatcat cacatgcagg tgggcctggg    33480
   ctccttggga ggaagtatgg tggggcaggg attgcaggcc agggcccaga acagtggcct    33540
   gagctgttac tctgtcctcg ttcctccccc agcttcacgc caggctcctg tacactgacg    33600
   gcctacaagc tgacccccag tggctacgaa tggggccgcc agaacacaga caagggcaac    33660
   aaccccaagg gctacctgcc ttcacactat gagagggtgc agatgctgct gtcggaccgt    33720
   ttccttggct tcttcatggt ccctgcccag tcctcgtgga actacaactt catgggtaag    33780
   tgggaggagc ctggggatgt ggggatagca gtagggataa ggtgaggcca tcgccctttg    33840
   cacttggggc ctggctggct tggaggtggc gcaggctgca tgaggcagga gccctgttaa    33900
   cattggctgt ttccttctcc ccgaaggtgt tcggcatgac cccaacatga aatatgagct    33960
   acagctggcg aaccccaaag agttctacca cgaggtgcac aggccctctc acttcctcaa    34020
   ctttgctctc ctgcaggagg gggaggttta ctctgcggat cgggaggacc tgtatgcctg    34080
   accgtttccc tgcctcctgc ttcagcctcc cgaggccgaa gcctcagccc ctccagacag    34140
   gccgctgaca ttcagcagtt tggcctcttt ccctctgtct gtgcttgtgt tgttgacctc    34200
   ctgatggctt gtcatcctga ataaaatata ataataaatt ttgtataaat agga          34254
<210> 3
   <211> 31777
   <212> RNA
   <213> Homo sapiens
<400> 3
<210> 4
   <211> 34254
   <212> RNA
   <213> Homo sapiens
<400> 4
<210> 5
   <211> 18
   <212> RNA
   <213> Artificial Sequence
<220>
   <223> Description of Artificial Sequence: Synthetic oligonucleotide
<400> 5
   gcguuggccu cuucaugc 18
<210> 6
   <211> 18
   <212> RNA
   <213> Artificial Sequence
<220>
   <223> Description of Artificial Sequence: Synthetic oligonucleotide
<400> 6
   gggcagcguu ggccucuu 18
<210> 7
   <211> 18
   <212> RNA
   <213> Artificial Sequence
<220>
   <223> Description of Artificial Sequence: Synthetic oligonucleotide
<400> 7
   uuuucgggca gcguuggc 18
<210> 8
   <211> 18
   <212> RNA
   <213> Artificial Sequence
<220>
   <223> Description of Artificial Sequence: Synthetic oligonucleotide
<400> 8
   gugaguuuuc gggcagcg 18
<210> 9
   <211> 18
   <212> RNA
   <213> Artificial Sequence
<220>
   <223> Description of Artificial Sequence: Synthetic oligonucleotide
<400> 9
   cugcugugag uuuucggg 18
<210> 10
   <211> 18
   <212> RNA
   <213> Artificial Sequence
<220>
   <223> Description of Artificial Sequence: Synthetic oligonucleotide
<400> 10
   cuguucugcu gugaguuu 18
<210> 11
   <211> 18
   <212> RNA
   <213> Artificial Sequence
<220>
   <223> Description of Artificial Sequence: Synthetic oligonucleotide
<400> 11
   uuucucuguu cugcugug 18
<210> 12
   <211> 18
   <212> RNA
   <213> Artificial Sequence
<220>
   <223> Description of Artificial Sequence: Synthetic oligonucleotide
<400> 12
   ugaccuuucu cuguucug 18
<210> 13
   <211> 18
   <212> RNA
   <213> Artificial Sequence
<220>
   <223> Description of Artificial Sequence: Synthetic oligonucleotide
<400> 13
   uuucuugacc uuucucug 18
<210> 14
   <211> 18
   <212> RNA
   <213> Artificial Sequence
<220>
   <223> Description of Artificial Sequence: Synthetic oligonucleotide
<400> 14
   uuaauuuucu ugaccuuu 18
<210> 15
   <211> 18
   <212> RNA
   <213> Artificial Sequence
<220>
   <223> Description of Artificial Sequence: Synthetic oligonucleotide
<400> 15
   gcuuuuuaau uuucuuga 18
<210> 16
   <211> 18
   <212> RNA
   <213> Artificial Sequence
<220>
   <223> Description of Artificial Sequence: Synthetic oligonucleotide
<400> 16
   uuuaagcuuu uuaauuuu 18
<210> 17
   <211> 18
   <212> RNA
   <213> Artificial Sequence
<220>
   <223> Description of Artificial Sequence: Synthetic oligonucleotide
<400> 17
   ucuucuuuaa gcuuuuua 18
<210> 18
   <211> 18
   <212> RNA
   <213> Artificial Sequence
<220>
   <223> Description of Artificial Sequence: Synthetic oligonucleotide
<400> 18
   aaaugucuuc uuuaagcu 18
<210> 19
   <211> 18
   <212> RNA
   <213> Artificial Sequence
<220>
   <223> Description of Artificial Sequence: Synthetic oligonucleotide
<400> 19
   cugugaaaug ucuucuuu 18
<210> 20
   <211> 18
   <212> RNA
   <213> Artificial Sequence
<220>
   <223> Description of Artificial Sequence: Synthetic oligonucleotide
<400> 20
   acccccugug aaaugucu 18
<210> 21
   <211> 18
   <212> RNA
   <213> Artificial Sequence
<220>
   <223> Description of Artificial Sequence: Synthetic oligonucleotide
<400> 21
   uguguacccc cugugaaa 18
<210> 22
   <211> 18
   <212> RNA
   <213> Artificial Sequence
<220>
   <223> Description of Artificial Sequence: Synthetic oligonucleotide
<400> 22
   agauaugugu acccccug 18
<210> 23
   <211> 18
   <212> RNA
   <213> Artificial Sequence
<220>
   <223> Description of Artificial Sequence: Synthetic oligonucleotide
<400> 23
   uauacagaua uguguacc 18
<210> 24
   <211> 18
   <212> RNA
   <213> Artificial Sequence
<220>
   <223> Description of Artificial Sequence: Synthetic oligonucleotide
<400> 24
   uuuggcccag uauggaca 18
<210> 25
   <211> 18
   <212> RNA
   <213> Artificial Sequence
<220>
   <223> Description of Artificial Sequence: Synthetic oligonucleotide
<400> 25
   ggaaguuugg cccaguau 18
<210> 26
   <211> 18
   <212> RNA
   <213> Artificial Sequence
<220>
   <223> Description of Artificial Sequence: Synthetic oligonucleotide
<400> 26
   gggaaguuug gcccagua 18
<210> 27
   <211> 18
   <212> RNA
   <213> Artificial Sequence
<220>
   <223> Description of Artificial Sequence: Synthetic oligonucleotide
<400> 27
   uuuuaguaau ucuaaggg 18
<210> 28
   <211> 18
   <212> RNA
   <213> Artificial Sequence
<220>
   <223> Description of Artificial Sequence: Synthetic oligonucleotide
<400> 28
   aaauguuuua guaauucu 18
<210> 29
   <211> 18
   <212> RNA
   <213> Artificial Sequence
<220>
   <223> Description of Artificial Sequence: Synthetic oligonucleotide
<400> 29
   uuggaaaaug uuuuagua 18
<210> 30
   <211> 18
   <212> RNA
   <213> Artificial Sequence
<220>
   <223> Description of Artificial Sequence: Synthetic oligonucleotide
<400> 30
   agcacuugga aaauguuu 18
<210> 31
   <211> 18
   <212> RNA
   <213> Artificial Sequence
<220>
   <223> Description of Artificial Sequence: Synthetic oligonucleotide
<400> 31
   ccucaagcac uuggaaaa 18
<210> 32
   <211> 18
   <212> RNA
   <213> Artificial Sequence
<220>
   <223> Description of Artificial Sequence: Synthetic oligonucleotide
<400> 32
   uucugccuca agcacuug 18
<210> 33
   <211> 18
   <212> RNA
   <213> Artificial Sequence
<220>
   <223> Description of Artificial Sequence: Synthetic oligonucleotide
<400> 33
   aaugauucug ccucaagc 18
<210> 34
   <211> 18
   <212> RNA
   <213> Artificial Sequence
<220>
   <223> Description of Artificial Sequence: Synthetic oligonucleotide
<400> 34
   gccacaauga uucugccu 18
<210> 35
   <211> 18
   <212> RNA
   <213> Artificial Sequence
<220>
   <223> Description of Artificial Sequence: Synthetic oligonucleotide
<400> 35
   uuuaggccac aaugauuc 18
<210> 36
   <211> 18
   <212> RNA
   <213> Artificial Sequence
<220>
   <223> Description of Artificial Sequence: Synthetic oligonucleotide
<400> 36
   augcuuuuag gccacaau 18
<210> 37
   <211> 18
   <212> RNA
   <213> Artificial Sequence
<220>
   <223> Description of Artificial Sequence: Synthetic oligonucleotide
<400> 37
   uagguaugcu uuuaggcc 18
<210> 38
   <211> 18
   <212> RNA
   <213> Artificial Sequence
<220>
   <223> Description of Artificial Sequence: Synthetic oligonucleotide
<400> 38
   gguguuaggu augcuuuu 18
<210> 39
   <211> 18
   <212> RNA
   <213> Artificial Sequence
<220>
   <223> Description of Artificial Sequence: Synthetic oligonucleotide
<400> 39
   gaacuggugu uagguaug 18
<210> 40
   <211> 18
   <212> RNA
   <213> Artificial Sequence
<220>
   <223> Description of Artificial Sequence: Synthetic oligonucleotide
<400> 40
   aguaggaacu gguguuag 18
<210> 41
   <211> 18
   <212> RNA
   <213> Artificial Sequence
<220>
   <223> Description of Artificial Sequence: Synthetic oligonucleotide
<400> 41
   aagagaguag gaacuggu 18
<210> 42
   <211> 18
   <212> RNA
   <213> Artificial Sequence
<220>
   <223> Description of Artificial Sequence: Synthetic oligonucleotide
<400> 42
   auuaaaagag aguaggaa 18
<210> 43
   <211> 18
   <212> RNA
   <213> Artificial Sequence
<220>
   <223> Description of Artificial Sequence: Synthetic oligonucleotide
<400> 43
   gggucauuaa aagagagu 18
<210> 44
   <211> 18
   <212> RNA
   <213> Artificial Sequence
<220>
   <223> Description of Artificial Sequence: Synthetic oligonucleotide
<400> 44
   cacuaggguc auuaaaag 18
<210> 45
   <211> 18
   <212> RNA
   <213> Artificial Sequence
<220>
   <223> Description of Artificial Sequence: Synthetic oligonucleotide
<400> 45
   auccacacua gggucauu 18
<210> 46
   <211> 18
   <212> RNA
   <213> Artificial Sequence
<220>
   <223> Description of Artificial Sequence: Synthetic oligonucleotide
<400> 46
   ucuugaucca cacuaggg 18
<210> 47
   <211> 18
   <212> RNA
   <213> Artificial Sequence
<220>
   <223> Description of Artificial Sequence: Synthetic oligonucleotide
<400> 47
   cuagcucuug auccacac 18
<210> 48
   <211> 18
   <212> RNA
   <213> Artificial Sequence
<220>
   <223> Description of Artificial Sequence: Synthetic oligonucleotide
<400> 48
   auuuacuagc ucuugauc 18
<210> 49
   <211> 18
   <212> RNA
   <213> Artificial Sequence
<220>
   <223> Description of Artificial Sequence: Synthetic oligonucleotide
<400> 49
   acaagauuua cuagcucu 18
<210> 50
   <211> 18
   <212> RNA
   <213> Artificial Sequence
<220>
   <223> Description of Artificial Sequence: Synthetic oligonucleotide
<400> 50
   auuuuacaag auuuacua 18
<210> 51
   <211> 18
   <212> RNA
   <213> Artificial Sequence
<220>
   <223> Description of Artificial Sequence: Synthetic oligonucleotide
<400> 51
   ucgcuauuuu acaagauu 18
<210> 52
   <211> 18
   <212> RNA
   <213> Artificial Sequence
<220>
   <223> Description of Artificial Sequence: Synthetic oligonucleotide
<400> 52
   aacucucgcu auuuuaca 18
<210> 53
   <211> 18
   <212> RNA
   <213> Artificial Sequence
<220>
   <223> Description of Artificial Sequence: Synthetic oligonucleotide
<400> 53
   ucauuaacuc ucgcuauu 18
<210> 54
   <211> 18
   <212> RNA
   <213> Artificial Sequence
<220>
   <223> Description of Artificial Sequence: Synthetic oligonucleotide
<400> 54
   auaagucauu aacucucg 18
<210> 55
   <211> 18
   <212> RNA
   <213> Artificial Sequence
<220>
   <223> Description of Artificial Sequence: Synthetic oligonucleotide
<400> 55
   uaaacauaag ucauuaac 18
<210> 56
   <211> 18
   <212> RNA
   <213> Artificial Sequence
<220>
   <223> Description of Artificial Sequence: Synthetic oligonucleotide
<400> 56
   aaccauaaac auaaguca 18
<210> 57
   <211> 18
   <212> RNA
   <213> Artificial Sequence
<220>
   <223> Description of Artificial Sequence: Synthetic oligonucleotide
<400> 57
   uggguaacca uaaacaua 18
<210> 58
   <211> 18
   <212> RNA
   <213> Artificial Sequence
<220>
   <223> Description of Artificial Sequence: Synthetic oligonucleotide
<400> 58
   agaacugggu aaccauaa 18
<210> 59
   <211> 18
   <212> RNA
   <213> Artificial Sequence
<220>
   <223> Description of Artificial Sequence: Synthetic oligonucleotide
<400> 59
   agggaagaac uggguaac 18
<210> 60
   <211> 18
   <212> RNA
   <213> Artificial Sequence
<220>
   <223> Description of Artificial Sequence: Synthetic oligonucleotide
<400> 60
   uugagaggga agaacugg 18
<210> 61
   <211> 18
   <212> RNA
   <213> Artificial Sequence
<220>
   <223> Description of Artificial Sequence: Synthetic oligonucleotide
<400> 61
   aagacuugag agggaaga 18
<210> 62
   <211> 18
   <212> RNA
   <213> Artificial Sequence
<220>
   <223> Description of Artificial Sequence: Synthetic oligonucleotide
<400> 62
   aaaagaagac uugagagg 18
<210> 63
   <211> 18
   <212> RNA
   <213> Artificial Sequence
<220>
   <223> Description of Artificial Sequence: Synthetic oligonucleotide
<400> 63
   aaaaaaaaag aagacuug 18
<210> 64
   <211> 18
   <212> RNA
   <213> Artificial Sequence
<220>
   <223> Description of Artificial Sequence: Synthetic oligonucleotide
<400> 64
   gguaaaaaaa aaaagaag 18
<210> 65
   <211> 18
   <212> RNA
   <213> Artificial Sequence
<220>
   <223> Description of Artificial Sequence: Synthetic oligonucleotide
<400> 65
   auucugguaa aaaaaaaa 18
<210> 66
   <211> 18
   <212> RNA
   <213> Artificial Sequence
<220>
   <223> Description of Artificial Sequence: Synthetic oligonucleotide
<400> 66
   cuaauauucu gguaaaaa 18
<210> 67
   <211> 18
   <212> RNA
   <213> Artificial Sequence
<220>
   <223> Description of Artificial Sequence: Synthetic oligonucleotide
<400> 67
   cagaacuaau auucuggu 18
<210> 68
   <211> 18
   <212> RNA
   <213> Artificial Sequence
<220>
   <223> Description of Artificial Sequence: Synthetic oligonucleotide
<400> 68
   uuguucagaa cuaauauu 18
<210> 69
   <211> 18
   <212> RNA
   <213> Artificial Sequence
<220>
   <223> Description of Artificial Sequence: Synthetic oligonucleotide
<400> 69
   uugacuuguu cagaacua 18
<210> 70
   <211> 18
   <212> RNA
   <213> Artificial Sequence
<220>
   <223> Description of Artificial Sequence: Synthetic oligonucleotide
<400> 70
   guauauugac uuguucag 18
<210> 71
   <211> 18
   <212> RNA
   <213> Artificial Sequence
<220>
   <223> Description of Artificial Sequence: Synthetic oligonucleotide
<400> 71
   aacaaguaua uugacuug 18
<210> 72
   <211> 18
   <212> RNA
   <213> Artificial Sequence
<220>
   <223> Description of Artificial Sequence: Synthetic oligonucleotide
<400> 72
   gccugaacaa guauauug 18
<210> 73
   <211> 18
   <212> RNA
   <213> Artificial Sequence
<220>
   <223> Description of Artificial Sequence: Synthetic oligonucleotide
<400> 73
   ucuuggccug aacaagua 18
<210> 74
   <211> 18
   <212> RNA
   <213> Artificial Sequence
<220>
   <223> Description of Artificial Sequence: Synthetic oligonucleotide
<400> 74
   ucacaucuug gccugaac 18
<210> 75
   <211> 18
   <212> RNA
   <213> Artificial Sequence
<220>
   <223> Description of Artificial Sequence: Synthetic oligonucleotide
<400> 75
   uuaguucaca ucuuggcc 18
<210> 76
   <211> 18
   <212> RNA
   <213> Artificial Sequence
<220>
   <223> Description of Artificial Sequence: Synthetic oligonucleotide
<400> 76
   aaucuuuagu ucacaucu 18
<210> 77
   <211> 18
   <212> RNA
   <213> Artificial Sequence
<220>
   <223> Description of Artificial Sequence: Synthetic oligonucleotide
<400> 77
   cauacaaucu uuaguuca 18
<210> 78
   <211> 18
   <212> RNA
   <213> Artificial Sequence
<220>
   <223> Description of Artificial Sequence: Synthetic oligonucleotide
<400> 78
   aaggacauac aaucuuua 18
<210> 79
   <211> 18
   <212> RNA
   <213> Artificial Sequence
<220>
   <223> Description of Artificial Sequence: Synthetic oligonucleotide
<400> 79
   ucucaaagga cauacaau 18
<210> 80
   <211> 18
   <212> RNA
   <213> Artificial Sequence
<220>
   <223> Description of Artificial Sequence: Synthetic oligonucleotide
<400> 80
   auaguucuca aaggacau 18
<210> 81
   <211> 18
   <212> RNA
   <213> Artificial Sequence
<220>
   <223> Description of Artificial Sequence: Synthetic oligonucleotide
<400> 81
   auuuuauagu ucucaaag 18
<210> 82
   <211> 18
   <212> RNA
   <213> Artificial Sequence
<220>
   <223> Description of Artificial Sequence: Synthetic oligonucleotide
<400> 82
   augauauuuu auaguucu 18
<210> 83
   <211> 18
   <212> RNA
   <213> Artificial Sequence
<220>
   <223> Description of Artificial Sequence: Synthetic oligonucleotide
<400> 83
   auaggaugau auuuuaua 18
<210> 84
   <211> 18
   <212> RNA
   <213> Artificial Sequence
<220>
   <223> Description of Artificial Sequence: Synthetic oligonucleotide
<400> 84
   aagaaauagg augauauu 18
<210> 85
   <211> 18
   <212> RNA
   <213> Artificial Sequence
<220>
   <223> Description of Artificial Sequence: Synthetic oligonucleotide
<400> 85
   aaaaaaagaa auaggaug 18
<210> 86
   <211> 18
   <212> RNA
   <213> Artificial Sequence
<220>
   <223> Description of Artificial Sequence: Synthetic oligonucleotide
<400> 86
   ugaaaaaaaa aagaaaua 18
<210> 87
   <211> 18
   <212> RNA
   <213> Artificial Sequence
<220>
   <223> Description of Artificial Sequence: Synthetic oligonucleotide
<400> 87
   aaagaugaaa aaaaaaag 18
<210> 88
   <211> 18
   <212> RNA
   <213> Artificial Sequence
<220>
   <223> Description of Artificial Sequence: Synthetic oligonucleotide
<400> 88
   aaaaaaaaga ugaaaaaa 18
<210> 89
   <211> 18
   <212> RNA
   <213> Artificial Sequence
<220>
   <223> Description of Artificial Sequence: Synthetic oligonucleotide
<400> 89
   uuaaaaaaaa aaagauga 18
<210> 90
   <211> 18
   <212> RNA
   <213> Artificial Sequence
<220>
   <223> Description of Artificial Sequence: Synthetic oligonucleotide
<400> 90
   accauuuaaa aaaaaaaa 18
<210> 91
   <211> 18
   <212> RNA
   <213> Artificial Sequence
<220>
   <223> Description of Artificial Sequence: Synthetic oligonucleotide
<400> 91
   cucagaccau uuaaaaaa 18
<210> 92
   <211> 18
   <212> RNA
   <213> Artificial Sequence
<220>
   <223> Description of Artificial Sequence: Synthetic oligonucleotide
<400> 92
   gcaaacucag accauuua 18
<210> 93
   <211> 18
   <212> RNA
   <213> Artificial Sequence
<220>
   <223> Description of Artificial Sequence: Synthetic oligonucleotide
<400> 93
   uggaagcaaa cucagacc 18
<210> 94
   <211> 18
   <212> RNA
   <213> Artificial Sequence
<220>
   <223> Description of Artificial Sequence: Synthetic oligonucleotide
<400> 94
   gauguuggaa gcaaacuc 18
<210> 95
   <211> 18
   <212> RNA
   <213> Artificial Sequence
<220>
   <223> Description of Artificial Sequence: Synthetic oligonucleotide
<400> 95
   ccauggaugu uggaagca 18
<210> 96
   <211> 18
   <212> RNA
   <213> Artificial Sequence
<220>
   <223> Description of Artificial Sequence: Synthetic oligonucleotide
<400> 96
   ugcuuccaug gauguugg 18
<210> 97
   <211> 18
   <212> RNA
   <213> Artificial Sequence
<220>
   <223> Description of Artificial Sequence: Synthetic oligonucleotide
<400> 97
   uguaaugcuu ccauggau 18
<210> 98
   <211> 18
   <212> RNA
   <213> Artificial Sequence
<220>
   <223> Description of Artificial Sequence: Synthetic oligonucleotide
<400> 98
   aaauuuguaa ugcuucca 18
<210> 99
   <211> 18
   <212> RNA
   <213> Artificial Sequence
<220>
   <223> Description of Artificial Sequence: Synthetic oligonucleotide
<400> 99
   aaagaaaauu uguaaugc 18
<210> 100
   <211> 18
   <212> RNA
   <213> Artificial Sequence
<220>
   <223> Description of Artificial Sequence: Synthetic oligonucleotide
<400> 100
   ggaggaaaga aaauuugu 18
<210> 101
   <211> 18
   <212> RNA
   <213> Artificial Sequence
<220>
   <223> Description of Artificial Sequence: Synthetic oligonucleotide
<400> 101
   acuacggagg aaagaaaa 18
<210> 102
   <211> 18
   <212> RNA
   <213> Artificial Sequence
<220>
   <223> Description of Artificial Sequence: Synthetic oligonucleotide
<400> 102
   auaugacuac ggaggaaa 18
<210> 103
   <211> 18
   <212> RNA
   <213> Artificial Sequence
<220>
   <223> Description of Artificial Sequence: Synthetic oligonucleotide
<400> 103
   uaaggauaug acuacgga 18
<210> 104
   <211> 18
   <212> RNA
   <213> Artificial Sequence
<220>
   <223> Description of Artificial Sequence: Synthetic oligonucleotide
<400> 104
   caggguaagg auaugacu 18
<210> 105
   <211> 18
   <212> RNA
   <213> Artificial Sequence
<220>
   <223> Description of Artificial Sequence: Synthetic oligonucleotide
<400> 105
   uacaucaggg uaaggaua 18
<210> 106
   <211> 18
   <212> RNA
   <213> Artificial Sequence
<220>
   <223> Description of Artificial Sequence: Synthetic oligonucleotide
<400> 106
   agcaauacau caggguaa 18
<210> 107
   <211> 18
   <212> RNA
   <213> Artificial Sequence
<220>
   <223> Description of Artificial Sequence: Synthetic oligonucleotide
<400> 107
   aacacagcaa uacaucag 18
<210> 108
   <211> 18
   <212> RNA
   <213> Artificial Sequence
<220>
   <223> Description of Artificial Sequence: Synthetic oligonucleotide
<400> 108
   gaugaaacac agcaauac 18
<210> 109
   <211> 18
   <212> RNA
   <213> Artificial Sequence
<220>
   <223> Description of Artificial Sequence: Synthetic oligonucleotide
<400> 109
   gugaggauga aacacagc 18
<210> 110
   <211> 18
   <212> RNA
   <213> Artificial Sequence
<220>
   <223> Description of Artificial Sequence: Synthetic oligonucleotide
<400> 110
   uuggagugag gaugaaac 18
<210> 111
   <211> 18
   <212> RNA
   <213> Artificial Sequence
<220>
   <223> Description of Artificial Sequence: Synthetic oligonucleotide
<400> 111
   gcauuuugga gugaggau 18
<210> 112
   <211> 18
   <212> RNA
   <213> Artificial Sequence
<220>
   <223> Description of Artificial Sequence: Synthetic oligonucleotide
<400> 112
   aaaaugcauu uuggagug 18
<210> 113
   <211> 18
   <212> RNA
   <213> Artificial Sequence
<220>
   <223> Description of Artificial Sequence: Synthetic oligonucleotide
<400> 113
   cauauaaaau gcauuuug 18
<210> 114
   <211> 18
   <212> RNA
   <213> Artificial Sequence
<220>
   <223> Description of Artificial Sequence: Synthetic oligonucleotide
<400> 114
   uacaacauau aaaaugca 18
<210> 115
   <211> 18
   <212> RNA
   <213> Artificial Sequence
<220>
   <223> Description of Artificial Sequence: Synthetic oligonucleotide
<400> 115
   auauuuacaa cauauaaa 18
<210> 116
   <211> 18
   <212> RNA
   <213> Artificial Sequence
<220>
   <223> Description of Artificial Sequence: Synthetic oligonucleotide
<400> 116
   gaaaaauauu uacaacau 18
<210> 117
   <211> 18
   <212> RNA
   <213> Artificial Sequence
<220>
   <223> Description of Artificial Sequence: Synthetic oligonucleotide
<400> 117
   agguagaaaa auauuuac 18
<210> 118
   <211> 18
   <212> RNA
   <213> Artificial Sequence
<220>
   <223> Description of Artificial Sequence: Synthetic oligonucleotide
<400> 118
   cacaaaggua gaaaaaua 18
<210> 119
   <211> 18
   <212> RNA
   <213> Artificial Sequence
<220>
   <223> Description of Artificial Sequence: Synthetic oligonucleotide
<400> 119
   aucaacacaa agguagaa 18
<210> 120
   <211> 18
   <212> RNA
   <213> Artificial Sequence
<220>
   <223> Description of Artificial Sequence: Synthetic oligonucleotide
<400> 120
   auuaaaucaa cacaaagg 18
<210> 121
   <211> 18
   <212> RNA
   <213> Artificial Sequence
<220>
   <223> Description of Artificial Sequence: Synthetic oligonucleotide
<400> 121
   aaaacauuaa aucaacac 18
<210> 122
   <211> 18
   <212> RNA
   <213> Artificial Sequence
<220>
   <223> Description of Artificial Sequence: Synthetic oligonucleotide
<400> 122
   aaaaauuagc cgggcgug 18
<210> 123
   <211> 18
   <212> RNA
   <213> Artificial Sequence
<220>
   <223> Description of Artificial Sequence: Synthetic oligonucleotide
<400> 123
   aauacaaaaa uuagccgg 18
<210> 124
   <211> 18
   <212> RNA
   <213> Artificial Sequence
<220>
   <223> Description of Artificial Sequence: Synthetic oligonucleotide
<400> 124
   cuaaaaauac aaaaauua 18
<210> 125
   <211> 18
   <212> RNA
   <213> Artificial Sequence
<220>
   <223> Description of Artificial Sequence: Synthetic oligonucleotide
<400> 125
   cucuacuaaa aauacaaa 18
<210> 126
   <211> 18
   <212> RNA
   <213> Artificial Sequence
<220>
   <223> Description of Artificial Sequence: Synthetic oligonucleotide
<400> 126
   cccaucucua cuaaaaau 18
<210> 127
   <211> 18
   <212> RNA
   <213> Artificial Sequence
<220>
   <223> Description of Artificial Sequence: Synthetic oligonucleotide
<400> 127
   gaaaccccau cucuacua 18
<210> 128
   <211> 18
   <212> RNA
   <213> Artificial Sequence
<220>
   <223> Description of Artificial Sequence: Synthetic oligonucleotide
<400> 128
   auggugaaac cccaucuc 18
<210> 129
   <211> 18
   <212> RNA
   <213> Artificial Sequence
<220>
   <223> Description of Artificial Sequence: Synthetic oligonucleotide
<400> 129
   ccaacauggu gaaacccc 18
<210> 130
   <211> 18
   <212> RNA
   <213> Artificial Sequence
<220>
   <223> Description of Artificial Sequence: Synthetic oligonucleotide
<400> 130
   ccuggccaac auggugaa 18
<210> 131
   <211> 18
   <212> RNA
   <213> Artificial Sequence
<220>
   <223> Description of Artificial Sequence: Synthetic oligonucleotide
<400> 131
   accagccugg ccaacaug 18
<210> 132
   <211> 18
   <212> RNA
   <213> Artificial Sequence
<220>
   <223> Description of Artificial Sequence: Synthetic oligonucleotide
<400> 132
   uuaagaccag ccuggcca 18
<210> 133
   <211> 18
   <212> RNA
   <213> Artificial Sequence
<220>
   <223> Description of Artificial Sequence: Synthetic oligonucleotide
<400> 133
   ggaguuuaag accagccu 18
<210> 134
   <211> 18
   <212> RNA
   <213> Artificial Sequence
<220>
   <223> Description of Artificial Sequence: Synthetic oligonucleotide
<400> 134
   ggucaggagu uuaagacc 18
<210> 135
   <211> 18
   <212> RNA
   <213> Artificial Sequence
<220>
   <223> Description of Artificial Sequence: Synthetic oligonucleotide
<400> 135
   cacgagguca ggaguuua 18
<210> 136
   <211> 18
   <212> RNA
   <213> Artificial Sequence
<220>
   <223> Description of Artificial Sequence: Synthetic oligonucleotide
<400> 136
   cagaucacga ggucagga 18
<210> 137
   <211> 18
   <212> RNA
   <213> Artificial Sequence
<220>
   <223> Description of Artificial Sequence: Synthetic oligonucleotide
<400> 137
   gugggcagau cacgaggu 18
<210> 138
   <211> 18
   <212> RNA
   <213> Artificial Sequence
<220>
   <223> Description of Artificial Sequence: Synthetic oligonucleotide
<400> 138
   ccaaaguggg cagaucac 18
<210> 139
   <211> 18
   <212> RNA
   <213> Artificial Sequence
<220>
   <223> Description of Artificial Sequence: Synthetic oligonucleotide
<400> 139
   ggaggccaaa gugggcag 18
<210> 140
   <211> 18
   <212> RNA
   <213> Artificial Sequence
<220>
   <223> Description of Artificial Sequence: Synthetic oligonucleotide
<400> 140
   cuuugggagg ccaaagug 18
<210> 141
   <211> 18
   <212> RNA
   <213> Artificial Sequence
<220>
   <223> Description of Artificial Sequence: Synthetic oligonucleotide
<400> 141
   cagcacuuug ggaggcca 18
<210> 142
   <211> 18
   <212> RNA
   <213> Artificial Sequence
<220>
   <223> Description of Artificial Sequence: Synthetic oligonucleotide
<400> 142
   aaucccagca cuuuggga 18
<210> 143
   <211> 18
   <212> RNA
   <213> Artificial Sequence
<220>
   <223> Description of Artificial Sequence: Synthetic oligonucleotide
<400> 143
   ccuguaaucc cagcacuu 18
<210> 144
   <211> 18
   <212> RNA
   <213> Artificial Sequence
<220>
   <223> Description of Artificial Sequence: Synthetic oligonucleotide
<400> 144
   ucaugccugu aaucccag 18
<210> 145
   <211> 18
   <212> RNA
   <213> Artificial Sequence
<220>
   <223> Description of Artificial Sequence: Synthetic oligonucleotide
<400> 145
   guggcucaug ccuguaau 18
<210> 146
   <211> 18
   <212> RNA
   <213> Artificial Sequence
<220>
   <223> Description of Artificial Sequence: Synthetic oligonucleotide
<400> 146
   gugcgguggc ucaugccu 18
<210> 147
   <211> 18
   <212> RNA
   <213> Artificial Sequence
<220>
   <223> Description of Artificial Sequence: Synthetic oligonucleotide
<400> 147
   gcugggugcg guggcuca 18
<210> 148
   <211> 18
   <212> RNA
   <213> Artificial Sequence
<220>
   <223> Description of Artificial Sequence: Synthetic oligonucleotide
<400> 148
   acuuggcugg gugcggug 18
<210> 149
   <211> 18
   <212> RNA
   <213> Artificial Sequence
<220>
   <223> Description of Artificial Sequence: Synthetic oligonucleotide
<400> 149
   ggauuacuug gcugggug 18
<210> 150
   <211> 18
   <212> RNA
   <213> Artificial Sequence
<220>
   <223> Description of Artificial Sequence: Synthetic oligonucleotide
<400> 150
   auauaggauu acuuggcu 18
<210> 151
   <211> 18
   <212> RNA
   <213> Artificial Sequence
<220>
   <223> Description of Artificial Sequence: Synthetic oligonucleotide
<400> 151
   uuauaauaua ggauuacu 18
<210> 152
   <211> 18
   <212> RNA
   <213> Artificial Sequence
<220>
   <223> Description of Artificial Sequence: Synthetic oligonucleotide
<400> 152
   gacucuuaua auauagga 18
<210> 153
   <211> 18
   <212> RNA
   <213> Artificial Sequence
<220>
   <223> Description of Artificial Sequence: Synthetic oligonucleotide
<400> 153
   ccuuagacuc uuauaaua 18
<210> 154
   <211> 18
   <212> RNA
   <213> Artificial Sequence
<220>
   <223> Description of Artificial Sequence: Synthetic oligonucleotide
<400> 154
   uagacccuua gacucuua 18
<210> 155
   <211> 18
   <212> RNA
   <213> Artificial Sequence
<220>
   <223> Description of Artificial Sequence: Synthetic oligonucleotide
<400> 155
   uccucuagac ccuuagac 18
<210> 156
   <211> 18
   <212> RNA
   <213> Artificial Sequence
<220>
   <223> Description of Artificial Sequence: Synthetic oligonucleotide
<400> 156
   uuuuguccuc uagacccu 18
<210> 157
   <211> 18
   <212> RNA
   <213> Artificial Sequence
<220>
   <223> Description of Artificial Sequence: Synthetic oligonucleotide
<400> 157
   agacuuuuug uccucuag 18
<210> 158
   <211> 18
   <212> RNA
   <213> Artificial Sequence
<220>
   <223> Description of Artificial Sequence: Synthetic oligonucleotide
<400> 158
   ccuacagacu uuuugucc 18
<210> 159
   <211> 18
   <212> RNA
   <213> Artificial Sequence
<220>
   <223> Description of Artificial Sequence: Synthetic oligonucleotide
<400> 159
   aaagaccuac agacuuuu 18
<210> 160
   <211> 18
   <212> RNA
   <213> Artificial Sequence
<220>
   <223> Description of Artificial Sequence: Synthetic oligonucleotide
<400> 160
   aauguaaaga ccuacaga 18
<210> 161
   <211> 18
   <212> RNA
   <213> Artificial Sequence
<220>
   <223> Description of Artificial Sequence: Synthetic oligonucleotide
<400> 161
   uugacaaugu aaagaccu 18
<210> 162
   <211> 18
   <212> RNA
   <213> Artificial Sequence
<220>
   <223> Description of Artificial Sequence: Synthetic oligonucleotide
<400> 162
   uguucuugac aauguaaa 18
<210> 163
   <211> 18
   <212> RNA
   <213> Artificial Sequence
<220>
   <223> Description of Artificial Sequence: Synthetic oligonucleotide
<400> 163
   cucauuguuc uugacaau 18
<210> 164
   <211> 18
   <212> RNA
   <213> Artificial Sequence
<220>
   <223> Description of Artificial Sequence: Synthetic oligonucleotide
<400> 164
   acaaucucau uguucuug 18
<210> 165
   <211> 18
   <212> RNA
   <213> Artificial Sequence
<220>
   <223> Description of Artificial Sequence: Synthetic oligonucleotide
<400> 165
   uacuuacaau cucauugu 18
<210> 166
   <211> 18
   <212> RNA
   <213> Artificial Sequence
<220>
   <223> Description of Artificial Sequence: Synthetic oligonucleotide
<400> 166
   aaaaauacuu acaaucuc 18
<210> 167
   <211> 18
   <212> RNA
   <213> Artificial Sequence
<220>
   <223> Description of Artificial Sequence: Synthetic oligonucleotide
<400> 167
   cauccaaaaa uacuuaca 18
<210> 168
   <211> 18
   <212> RNA
   <213> Artificial Sequence
<220>
   <223> Description of Artificial Sequence: Synthetic oligonucleotide
<400> 168
   acuaucaucc aaaaauac 18
<210> 169
   <211> 18
   <212> RNA
   <213> Artificial Sequence
<220>
   <223> Description of Artificial Sequence: Synthetic oligonucleotide
<400> 169
   uauuuacuau cauccaaa 18
<210> 170
   <211> 18
   <212> RNA
   <213> Artificial Sequence
<220>
   <223> Description of Artificial Sequence: Synthetic oligonucleotide
<400> 170
   agagguauuu acuaucau 18
<210> 171
   <211> 18
   <212> RNA
   <213> Artificial Sequence
<220>
   <223> Description of Artificial Sequence: Synthetic oligonucleotide
<400> 171
   cagggagagg uauuuacu 18
<210> 172
   <211> 18
   <212> RNA
   <213> Artificial Sequence
<220>
   <223> Description of Artificial Sequence: Synthetic oligonucleotide
<400> 172
   agagucaggg agagguau 18
<210> 173
   <211> 18
   <212> RNA
   <213> Artificial Sequence
<220>
   <223> Description of Artificial Sequence: Synthetic oligonucleotide
<400> 173
   cucuaagagu cagggaga 18
<210> 174
   <211> 18
   <212> RNA
   <213> Artificial Sequence
<220>
   <223> Description of Artificial Sequence: Synthetic oligonucleotide
<400> 174
   guuuacucua agagucag 18
<210> 175
   <211> 18
   <212> RNA
   <213> Artificial Sequence
<220>
   <223> Description of Artificial Sequence: Synthetic oligonucleotide
<400> 175
   ccaaaguuua cucuaaga 18
<210> 176
   <211> 18
   <212> RNA
   <213> Artificial Sequence
<220>
   <223> Description of Artificial Sequence: Synthetic oligonucleotide
<400> 176
   acuaaccaaa guuuacuc 18
<210> 177
   <211> 18
   <212> RNA
   <213> Artificial Sequence
<220>
   <223> Description of Artificial Sequence: Synthetic oligonucleotide
<400> 177
   acauuacuaa ccaaaguu 18
<210> 178
   <211> 18
   <212> RNA
   <213> Artificial Sequence
<220>
   <223> Description of Artificial Sequence: Synthetic oligonucleotide
<400> 178
   ccauuacauu acuaacca 18
<210> 179
   <211> 18
   <212> RNA
   <213> Artificial Sequence
<220>
   <223> Description of Artificial Sequence: Synthetic oligonucleotide
<400> 179
   auuaaccauu acauuacu 18
<210> 180
   <211> 18
   <212> RNA
   <213> Artificial Sequence
<220>
   <223> Description of Artificial Sequence: Synthetic oligonucleotide
<400> 180
   uuuacauuaa ccauuaca 18
<210> 181
   <211> 18
   <212> RNA
   <213> Artificial Sequence
<220>
   <223> Description of Artificial Sequence: Synthetic oligonucleotide
<400> 181
   agcacuuuac auuaacca 18
<210> 182
   <211> 18
   <212> RNA
   <213> Artificial Sequence
<220>
   <223> Description of Artificial Sequence: Synthetic oligonucleotide
<400> 182
   agucuagcac uuuacauu 18
<210> 183
   <211> 18
   <212> RNA
   <213> Artificial Sequence
<220>
   <223> Description of Artificial Sequence: Synthetic oligonucleotide
<400> 183
   uucaaagucu agcacuuu 18
<210> 184
   <211> 18
   <212> RNA
   <213> Artificial Sequence
<220>
   <223> Description of Artificial Sequence: Synthetic oligonucleotide
<400> 184
   auuucuucaa agucuagc 18
<210> 185
   <211> 18
   <212> RNA
   <213> Artificial Sequence
<220>
   <223> Description of Artificial Sequence: Synthetic oligonucleotide
<400> 185
   cuuacauuuc uucaaagu 18
<210> 186
   <211> 18
   <212> RNA
   <213> Artificial Sequence
<220>
   <223> Description of Artificial Sequence: Synthetic oligonucleotide
<400> 186
   acacucuuac auuucuuc 18
<210> 187
   <211> 18
   <212> RNA
   <213> Artificial Sequence
<220>
   <223> Description of Artificial Sequence: Synthetic oligonucleotide
<400> 187
   uuuccacacu cuuacauu 18
<210> 188
   <211> 18
   <212> RNA
   <213> Artificial Sequence
<220>
   <223> Description of Artificial Sequence: Synthetic oligonucleotide
<400> 188
   aaauauuucc acacucuu 18
<210> 189
   <211> 18
   <212> RNA
   <213> Artificial Sequence
<220>
   <223> Description of Artificial Sequence: Synthetic oligonucleotide
<400> 189
   ucuagaaaua uuuccaca 18
<210> 190
   <211> 18
   <212> RNA
   <213> Artificial Sequence
<220>
   <223> Description of Artificial Sequence: Synthetic oligonucleotide
<400> 190
   augagucuag aaauauuu 18
<210> 191
   <211> 18
   <212> RNA
   <213> Artificial Sequence
<220>
   <223> Description of Artificial Sequence: Synthetic oligonucleotide
<400> 191
   aguugaugag ucuagaaa 18
<210> 192
   <211> 18
   <212> RNA
   <213> Artificial Sequence
<220>
   <223> Description of Artificial Sequence: Synthetic oligonucleotide
<400> 192
   gguagaguug augagucu 18
<210> 193
   <211> 18
   <212> RNA
   <213> Artificial Sequence
<220>
   <223> Description of Artificial Sequence: Synthetic oligonucleotide
<400> 193
   uaaaagguag aguugaug 18
<210> 194
   <211> 18
   <212> RNA
   <213> Artificial Sequence
<220>
   <223> Description of Artificial Sequence: Synthetic oligonucleotide
<400> 194
   augcuuaaaa gguagagu 18
<210> 195
   <211> 18
   <212> RNA
   <213> Artificial Sequence
<220>
   <223> Description of Artificial Sequence: Synthetic oligonucleotide
<400> 195
   gaugaaugcu uaaaaggu 18
<210> 196
   <211> 18
   <212> RNA
   <213> Artificial Sequence
<220>
   <223> Description of Artificial Sequence: Synthetic oligonucleotide
<400> 196
   aaauagauga augcuuaa 18
<210> 197
   <211> 18
   <212> RNA
   <213> Artificial Sequence
<220>
   <223> Description of Artificial Sequence: Synthetic oligonucleotide
<400> 197
   uaaacaaaua gaugaaug 18
<210> 198
   <211> 18
   <212> RNA
   <213> Artificial Sequence
<220>
   <223> Description of Artificial Sequence: Synthetic oligonucleotide
<400> 198
   uggaguaaac aaauagau 18
<210> 199
   <211> 18
   <212> RNA
   <213> Artificial Sequence
<220>
   <223> Description of Artificial Sequence: Synthetic oligonucleotide
<400> 199
   gccucuggag uaaacaaa 18
<210> 200
   <211> 18
   <212> RNA
   <213> Artificial Sequence
<220>
   <223> Description of Artificial Sequence: Synthetic oligonucleotide
<400> 200
   caaaggccuc uggaguaa 18
<210> 201
   <211> 18
   <212> RNA
   <213> Artificial Sequence
<220>
   <223> Description of Artificial Sequence: Synthetic oligonucleotide
<400> 201
   auaaacaaag gccucugg 18
<210> 202
   <211> 18
   <212> RNA
   <213> Artificial Sequence
<220>
   <223> Description of Artificial Sequence: Synthetic oligonucleotide
<400> 202
   ucucuauaaa caaaggcc 18
<210> 203
   <211> 18
   <212> RNA
   <213> Artificial Sequence
<220>
   <223> Description of Artificial Sequence: Synthetic oligonucleotide
<400> 203
   uuuccucucu auaaacaa 18
<210> 204
   <211> 18
   <212> RNA
   <213> Artificial Sequence
<220>
   <223> Description of Artificial Sequence: Synthetic oligonucleotide
<400> 204
   aaugguuucc ucucuaua 18
<210> 205
   <211> 18
   <212> RNA
   <213> Artificial Sequence
<220>
   <223> Description of Artificial Sequence: Synthetic oligonucleotide
<400> 205
   uccaaaaugg uuuccucu 18
<210> 206
   <211> 18
   <212> RNA
   <213> Artificial Sequence
<220>
   <223> Description of Artificial Sequence: Synthetic oligonucleotide
<400> 206
   augaauccaa aaugguuu 18
<210> 207
   <211> 18
   <212> RNA
   <213> Artificial Sequence
<220>
   <223> Description of Artificial Sequence: Synthetic oligonucleotide
<400> 207
   aaaagaugaa uccaaaau 18
<210> 208
   <211> 18
   <212> RNA
   <213> Artificial Sequence
<220>
   <223> Description of Artificial Sequence: Synthetic oligonucleotide
<400> 208
   aaguaaaaag augaaucc 18
<210> 209
   <211> 18
   <212> RNA
   <213> Artificial Sequence
<220>
   <223> Description of Artificial Sequence: Synthetic oligonucleotide
<400> 209
   guggcaagua aaaagaug 18
<210> 210
   <211> 18
   <212> RNA
   <213> Artificial Sequence
<220>
   <223> Description of Artificial Sequence: Synthetic oligonucleotide
<400> 210
   auggaguggc aaguaaaa 18
<210> 211
   <211> 18
   <212> RNA
   <213> Artificial Sequence
<220>
   <223> Description of Artificial Sequence: Synthetic oligonucleotide
<400> 211
   ccugaaugga guggcaag 18
<210> 212
   <211> 18
   <212> RNA
   <213> Artificial Sequence
<220>
   <223> Description of Artificial Sequence: Synthetic oligonucleotide
<400> 212
   agcugccuga auggagug 18
<210> 213
   <211> 18
   <212> RNA
   <213> Artificial Sequence
<220>
   <223> Description of Artificial Sequence: Synthetic oligonucleotide
<400> 213
   ucagcagcug ccugaaug 18
<210> 214
   <211> 18
   <212> RNA
   <213> Artificial Sequence
<220>
   <223> Description of Artificial Sequence: Synthetic oligonucleotide
<400> 214
   uagcaucagc agcugccu 18
<210> 215
   <211> 18
   <212> RNA
   <213> Artificial Sequence
<220>
   <223> Description of Artificial Sequence: Synthetic oligonucleotide
<400> 215
   cauuuuagca ucagcagc 18
<210> 216
   <211> 18
   <212> RNA
   <213> Artificial Sequence
<220>
   <223> Description of Artificial Sequence: Synthetic oligonucleotide
<400> 216
   ucuuccauuu uagcauca 18
<210> 217
   <211> 18
   <212> RNA
   <213> Artificial Sequence
<220>
   <223> Description of Artificial Sequence: Synthetic oligonucleotide
<400> 217
   uuaugucuuc cauuuuag 18
<210> 218
   <211> 18
   <212> RNA
   <213> Artificial Sequence
<220>
   <223> Description of Artificial Sequence: Synthetic oligonucleotide
<400> 218
   uuuaguuaug ucuuccau 18
<210> 219
   <211> 18
   <212> RNA
   <213> Artificial Sequence
<220>
   <223> Description of Artificial Sequence: Synthetic oligonucleotide
<400> 219
   guugcucaaa uuucgaac 18
<210> 220
   <211> 18
   <212> RNA
   <213> Artificial Sequence
<220>
   <223> Description of Artificial Sequence: Synthetic oligonucleotide
<400> 220
   gcuggguugc ucaaauuu 18
<210> 221
   <211> 18
   <212> RNA
   <213> Artificial Sequence
<220>
   <223> Description of Artificial Sequence: Synthetic oligonucleotide
<400> 221
   ucuuggcugg guugcuca 18
<210> 222
   <211> 18
   <212> RNA
   <213> Artificial Sequence
<220>
   <223> Description of Artificial Sequence: Synthetic oligonucleotide
<400> 222
   gaacuucuug gcuggguu 18
<210> 223
   <211> 18
   <212> RNA
   <213> Artificial Sequence
<220>
   <223> Description of Artificial Sequence: Synthetic oligonucleotide
<400> 223
   aucuugaacu ucuuggcu 18
<210> 224
   <211> 18
   <212> RNA
   <213> Artificial Sequence
<220>
   <223> Description of Artificial Sequence: Synthetic oligonucleotide
<400> 224
   cuucaaucuu gaacuucu 18
<210> 225
   <211> 18
   <212> RNA
   <213> Artificial Sequence
<220>
   <223> Description of Artificial Sequence: Synthetic oligonucleotide
<400> 225
   auuggcuuca aucuugaa 18
<210> 226
   <211> 18
   <212> RNA
   <213> Artificial Sequence
<220>
   <223> Description of Artificial Sequence: Synthetic oligonucleotide
<400> 226
   ccagcauugg cuucaauc 18
<210> 227
   <211> 18
   <212> RNA
   <213> Artificial Sequence
<220>
   <223> Description of Artificial Sequence: Synthetic oligonucleotide
<400> 227
   guugcccagc auuggcuu 18
<210> 228
   <211> 18
   <212> RNA
   <213> Artificial Sequence
<220>
   <223> Description of Artificial Sequence: Synthetic oligonucleotide
<400> 228
   guacaguugc ccagcauu 18
<210> 229
   <211> 18
   <212> RNA
   <213> Artificial Sequence
<220>
   <223> Description of Artificial Sequence: Synthetic oligonucleotide
<400> 229
   gucagguaca guugccca 18
<210> 230
   <211> 18
   <212> RNA
   <213> Artificial Sequence
<220>
   <223> Description of Artificial Sequence: Synthetic oligonucleotide
<400> 230
   ccccugucag guacaguu 18
<210> 231
   <211> 18
   <212> RNA
   <213> Artificial Sequence
<220>
   <223> Description of Artificial Sequence: Synthetic oligonucleotide
<400> 231
   caccaccccu gucaggua 18
<210> 232
   <211> 18
   <212> RNA
   <213> Artificial Sequence
<220>
   <223> Description of Artificial Sequence: Synthetic oligonucleotide
<400> 232
   aguaccacca ccccuguc 18
<210> 233
   <211> 18
   <212> RNA
   <213> Artificial Sequence
<220>
   <223> Description of Artificial Sequence: Synthetic oligonucleotide
<400> 233
   ugugcaguac caccaccc 18
<210> 234
   <211> 18
   <212> RNA
   <213> Artificial Sequence
<220>
   <223> Description of Artificial Sequence: Synthetic oligonucleotide
<400> 234
   auccuugugc aguaccac 18
<210> 235
   <211> 18
   <212> RNA
   <213> Artificial Sequence
<220>
   <223> Description of Artificial Sequence: Synthetic oligonucleotide
<400> 235
   uugacauccu ugugcagu 18
<210> 236
   <211> 18
   <212> RNA
   <213> Artificial Sequence
<220>
   <223> Description of Artificial Sequence: Synthetic oligonucleotide
<400> 236
   ccacguugac auccuugu 18
<210> 237
   <211> 18
   <212> RNA
   <213> Artificial Sequence
<220>
   <223> Description of Artificial Sequence: Synthetic oligonucleotide
<400> 237
   uacuaccacg uugacauc 18
<210> 238
   <211> 18
   <212> RNA
   <213> Artificial Sequence
<220>
   <223> Description of Artificial Sequence: Synthetic oligonucleotide
<400> 238
   uccacuacua ccacguug 18
<210> 239
   <211> 18
   <212> RNA
   <213> Artificial Sequence
<220>
   <223> Description of Artificial Sequence: Synthetic oligonucleotide
<400> 239
   ccccuuccac uacuacca 18
<210> 240
   <211> 18
   <212> RNA
   <213> Artificial Sequence
<220>
   <223> Description of Artificial Sequence: Synthetic oligonucleotide
<400> 240
   ggguugcuca aauuucga 18
<210> 241
   <211> 18
   <212> RNA
   <213> Artificial Sequence
<220>
   <223> Description of Artificial Sequence: Synthetic oligonucleotide
<400> 241
   uggcuggguu gcucaaau 18
<210> 242
   <211> 18
   <212> RNA
   <213> Artificial Sequence
<220>
   <223> Description of Artificial Sequence: Synthetic oligonucleotide
<400> 242
   cuucuuggcu ggguugcu 18
<210> 243
   <211> 18
   <212> RNA
   <213> Artificial Sequence
<220>
   <223> Description of Artificial Sequence: Synthetic oligonucleotide
<400> 243
   uugaacuucu uggcuggg 18
<210> 244
   <211> 18
   <212> RNA
   <213> Artificial Sequence
<220>
   <223> Description of Artificial Sequence: Synthetic oligonucleotide
<400> 244
   caaucuugaa cuucuugg 18
<210> 245
   <211> 18
   <212> RNA
   <213> Artificial Sequence
<220>
   <223> Description of Artificial Sequence: Synthetic oligonucleotide
<400> 245
   ggcuucaauc uugaacuu 18
<210> 246
   <211> 18
   <212> RNA
   <213> Artificial Sequence
<220>
   <223> Description of Artificial Sequence: Synthetic oligonucleotide
<400> 246
   gcauuggcuu caaucuug 18
<210> 247
   <211> 18
   <212> RNA
   <213> Artificial Sequence
<220>
   <223> Description of Artificial Sequence: Synthetic oligonucleotide
<400> 247
   gcccagcauu ggcuucaa 18
<210> 248
   <211> 18
   <212> RNA
   <213> Artificial Sequence
<220>
   <223> Description of Artificial Sequence: Synthetic oligonucleotide
<400> 248
   caguugccca gcauuggc 18
<210> 249
   <211> 18
   <212> RNA
   <213> Artificial Sequence
<220>
   <223> Description of Artificial Sequence: Synthetic oligonucleotide
<400> 249
   agguacaguu gcccagca 18
<210> 250
   <211> 18
   <212> RNA
   <213> Artificial Sequence
<220>
   <223> Description of Artificial Sequence: Synthetic oligonucleotide
<400> 250
   cugucaggua caguugcc 18
<210> 251
   <211> 18
   <212> RNA
   <213> Artificial Sequence
<220>
   <223> Description of Artificial Sequence: Synthetic oligonucleotide
<400> 251
   caccccuguc agguacag 18
<210> 252
   <211> 18
   <212> RNA
   <213> Artificial Sequence
<220>
   <223> Description of Artificial Sequence: Synthetic oligonucleotide
<400> 252
   accaccaccc cugucagg 18
<210> 253
   <211> 18
   <212> RNA
   <213> Artificial Sequence
<220>
   <223> Description of Artificial Sequence: Synthetic oligonucleotide
<400> 253
   gcaguaccac caccccug 18
<210> 254
   <211> 18
   <212> RNA
   <213> Artificial Sequence
<220>
   <223> Description of Artificial Sequence: Synthetic oligonucleotide
<400> 254
   cuugugcagu accaccac 18
<210> 255
   <211> 18
   <212> RNA
   <213> Artificial Sequence
<220>
   <223> Description of Artificial Sequence: Synthetic oligonucleotide
<400> 255
   acauccuugu gcaguacc 18
<210> 256
   <211> 18
   <212> RNA
   <213> Artificial Sequence
<220>
   <223> Description of Artificial Sequence: Synthetic oligonucleotide
<400> 256
   cguugacauc cuugugca 18
<210> 257
   <211> 18
   <212> RNA
   <213> Artificial Sequence
<220>
   <223> Description of Artificial Sequence: Synthetic oligonucleotide
<400> 257
   uaccacguug acauccuu 18
<210> 258
   <211> 18
   <212> RNA
   <213> Artificial Sequence
<220>
   <223> Description of Artificial Sequence: Synthetic oligonucleotide
<400> 258
   acuacuacca cguugaca 18
<210> 259
   <211> 18
   <212> RNA
   <213> Artificial Sequence
<220>
   <223> Description of Artificial Sequence: Synthetic oligonucleotide
<400> 259
   cuuccacuac uaccacgu 18
<210> 260
   <211> 18
   <212> RNA
   <213> Artificial Sequence
<220>
   <223> Description of Artificial Sequence: Synthetic oligonucleotide
<400> 260
   ucccucaagu uuuucaga 18
<210> 261
   <211> 18
   <212> RNA
   <213> Artificial Sequence
<220>
   <223> Description of Artificial Sequence: Synthetic oligonucleotide
<400> 261
   cagucucccu caaguuuu 18
<210> 262
   <211> 18
   <212> RNA
   <213> Artificial Sequence
<220>
   <223> Description of Artificial Sequence: Synthetic oligonucleotide
<400> 262
   gggacagucu cccucaag 18
<210> 263
   <211> 18
   <212> RNA
   <213> Artificial Sequence
<220>
   <223> Description of Artificial Sequence: Synthetic oligonucleotide
<400> 263
   agccuagggu ugucuggg 18
<210> 264
   <211> 18
   <212> RNA
   <213> Artificial Sequence
<220>
   <223> Description of Artificial Sequence: Synthetic oligonucleotide
<400> 264
   cucuaagccu aggguugu 18
<210> 265
   <211> 18
   <212> RNA
   <213> Artificial Sequence
<220>
   <223> Description of Artificial Sequence: Synthetic oligonucleotide
<400> 265
   aaucacucua agccuagg 18
<210> 266
   <211> 18
   <212> RNA
   <213> Artificial Sequence
<220>
   <223> Description of Artificial Sequence: Synthetic oligonucleotide
<400> 266
   augcuaauca cucuaagc 18
<210> 267
   <211> 18
   <212> RNA
   <213> Artificial Sequence
<220>
   <223> Description of Artificial Sequence: Synthetic oligonucleotide
<400> 267
   ccacaaugcu aaucacuc 18
<210> 268
   <211> 18
   <212> RNA
   <213> Artificial Sequence
<220>
   <223> Description of Artificial Sequence: Synthetic oligonucleotide
<400> 268
   ucuccccaca augcuaau 18
<210> 269
   <211> 18
   <212> RNA
   <213> Artificial Sequence
<220>
   <223> Description of Artificial Sequence: Synthetic oligonucleotide
<400> 269
   uccuuucucc ccacaaug 18
<210> 270
   <211> 18
   <212> RNA
   <213> Artificial Sequence
<220>
   <223> Description of Artificial Sequence: Synthetic oligonucleotide
<400> 270
   uuuucuccuu ucucccca 18
<210> 271
   <211> 18
   <212> RNA
   <213> Artificial Sequence
<220>
   <223> Description of Artificial Sequence: Synthetic oligonucleotide
<400> 271
   gaucuuuuuc uccuuucu 18
<210> 272
   <211> 18
   <212> RNA
   <213> Artificial Sequence
<220>
   <223> Description of Artificial Sequence: Synthetic oligonucleotide
<400> 272
   aauacgaucu uuuucucc 18
<210> 273
   <211> 18
   <212> RNA
   <213> Artificial Sequence
<220>
   <223> Description of Artificial Sequence: Synthetic oligonucleotide
<400> 273
   acuaaaauac gaucuuuu 18
<210> 274
   <211> 18
   <212> RNA
   <213> Artificial Sequence
<220>
   <223> Description of Artificial Sequence: Synthetic oligonucleotide
<400> 274
   auggcacuaa aauacgau 18
<210> 275
   <211> 18
   <212> RNA
   <213> Artificial Sequence
<220>
   <223> Description of Artificial Sequence: Synthetic oligonucleotide
<400> 275
   cagguauggc acuaaaau 18
<210> 276
   <211> 18
   <212> RNA
   <213> Artificial Sequence
<220>
   <223> Description of Artificial Sequence: Synthetic oligonucleotide
<400> 276
   uauaucaggu auggcacu 18
<210> 277
   <211> 18
   <212> RNA
   <213> Artificial Sequence
<220>
   <223> Description of Artificial Sequence: Synthetic oligonucleotide
<400> 277
   cauauuauau cagguaug 18
<210> 278
   <211> 18
   <212> RNA
   <213> Artificial Sequence
<220>
   <223> Description of Artificial Sequence: Synthetic oligonucleotide
<400> 278
   ccuagcauau uauaucag 18
<210> 279
   <211> 18
   <212> RNA
   <213> Artificial Sequence
<220>
   <223> Description of Artificial Sequence: Synthetic oligonucleotide
<400> 279
   agagaccuag cauauuau 18
<210> 280
   <211> 18
   <212> RNA
   <213> Artificial Sequence
<220>
   <223> Description of Artificial Sequence: Synthetic oligonucleotide
<400> 280
   gaagcagaga ccuagcau 18
<210> 281
   <211> 18
   <212> RNA
   <213> Artificial Sequence
<220>
   <223> Description of Artificial Sequence: Synthetic oligonucleotide
<400> 281
   acacagaagc agagaccu 18
<210> 282
   <211> 18
   <212> RNA
   <213> Artificial Sequence
<220>
   <223> Description of Artificial Sequence: Synthetic oligonucleotide
<400> 282
   aaaaaacaca gaagcaga 18
<210> 283
   <211> 18
   <212> RNA
   <213> Artificial Sequence
<220>
   <223> Description of Artificial Sequence: Synthetic oligonucleotide
<400> 283
   acuccaaaaa acacagaa 18
<210> 284
   <211> 18
   <212> RNA
   <213> Artificial Sequence
<220>
   <223> Description of Artificial Sequence: Synthetic oligonucleotide
<400> 284
   cuucuacucc aaaaaaca 18
<210> 285
   <211> 18
   <212> RNA
   <213> Artificial Sequence
<220>
   <223> Description of Artificial Sequence: Synthetic oligonucleotide
<400> 285
   agggccuucu acuccaaa 18
<210> 286
   <211> 18
   <212> RNA
   <213> Artificial Sequence
<220>
   <223> Description of Artificial Sequence: Synthetic oligonucleotide
<400> 286
   aucuuagggc cuucuacu 18
<210> 287
   <211> 18
   <212> RNA
   <213> Artificial Sequence
<220>
   <223> Description of Artificial Sequence: Synthetic oligonucleotide
<400> 287
   agaccuagca uauuauau 18
<210> 288
   <211> 18
   <212> RNA
   <213> Artificial Sequence
<220>
   <223> Description of Artificial Sequence: Synthetic oligonucleotide
<400> 288
   agcagagacc uagcauau 18
<210> 289
   <211> 18
   <212> RNA
   <213> Artificial Sequence
<220>
   <223> Description of Artificial Sequence: Synthetic oligonucleotide
<400> 289
   acagaagcag agaccuag 18
<210> 290
   <211> 18
   <212> RNA
   <213> Artificial Sequence
<220>
   <223> Description of Artificial Sequence: Synthetic oligonucleotide
<400> 290
   aaaacacaga agcagaga 18
<210> 291
   <211> 18
   <212> RNA
   <213> Artificial Sequence
<220>
   <223> Description of Artificial Sequence: Synthetic oligonucleotide
<400> 291
   uccaaaaaac acagaagc 18
<210> 292
   <211> 18
   <212> RNA
   <213> Artificial Sequence
<220>
   <223> Description of Artificial Sequence: Synthetic oligonucleotide
<400> 292
   ucuacuccaa aaaacaca 18
<210> 293
   <211> 18
   <212> RNA
   <213> Artificial Sequence
<220>
   <223> Description of Artificial Sequence: Synthetic oligonucleotide
<400> 293
   ggccuucuac uccaaaaa 18
<210> 294
   <211> 18
   <212> RNA
   <213> Artificial Sequence
<220>
   <223> Description of Artificial Sequence: Synthetic oligonucleotide
<400> 294
   cuuagggccu ucuacucc 18
<210> 295
   <211> 18
   <212> RNA
   <213> Artificial Sequence
<220>
   <223> Description of Artificial Sequence: Synthetic oligonucleotide
<400> 295
   cacaucuuag ggccuucu 18
<210> 296
   <211> 18
   <212> RNA
   <213> Artificial Sequence
<220>
   <223> Description of Artificial Sequence: Synthetic oligonucleotide
<400> 296
   ggaaccacau cuuagggc 18
<210> 297
   <211> 18
   <212> RNA
   <213> Artificial Sequence
<220>
   <223> Description of Artificial Sequence: Synthetic oligonucleotide
<400> 297
   ucucaggaac cacaucuu 18
<210> 298
   <211> 18
   <212> RNA
   <213> Artificial Sequence
<220>
   <223> Description of Artificial Sequence: Synthetic oligonucleotide
<400> 298
   agcaaucuca ggaaccac 18
<210> 299
   <211> 18
   <212> RNA
   <213> Artificial Sequence
<220>
   <223> Description of Artificial Sequence: Synthetic oligonucleotide
<400> 299
   guugaagcaa ucucagga 18
<210> 300
   <211> 18
   <212> RNA
   <213> Artificial Sequence
<220>
   <223> Description of Artificial Sequence: Synthetic oligonucleotide
<400> 300
   ugguaguuga agcaaucu 18
<210> 301
   <211> 18
   <212> RNA
   <213> Artificial Sequence
<220>
   <223> Description of Artificial Sequence: Synthetic oligonucleotide
<400> 301
   uaauguggua guugaagc 18
<210> 302
   <211> 18
   <212> RNA
   <213> Artificial Sequence
<220>
   <223> Description of Artificial Sequence: Synthetic oligonucleotide
<400> 302
   gacauuaaug ugguaguu 18
<210> 303
   <211> 18
   <212> RNA
   <213> Artificial Sequence
<220>
   <223> Description of Artificial Sequence: Synthetic oligonucleotide
<400> 303
   gcucagacau uaaugugg 18
<210> 304
   <211> 18
   <212> RNA
   <213> Artificial Sequence
<220>
   <223> Description of Artificial Sequence: Synthetic oligonucleotide
<400> 304
   ucugagcuca gacauuaa 18
<210> 305
   <211> 18
   <212> RNA
   <213> Artificial Sequence
<220>
   <223> Description of Artificial Sequence: Synthetic oligonucleotide
<400> 305
   gaaccucuga gcucagac 18
<210> 306
   <211> 18
   <212> RNA
   <213> Artificial Sequence
<220>
   <223> Description of Artificial Sequence: Synthetic oligonucleotide
<400> 306
   agaaagaacc ucugagcu 18
<210> 307
   <211> 18
   <212> RNA
   <213> Artificial Sequence
<220>
   <223> Description of Artificial Sequence: Synthetic oligonucleotide
<400> 307
   acuguggaag augauggg 18
<210> 308
   <211> 18
   <212> RNA
   <213> Artificial Sequence
<220>
   <223> Description of Artificial Sequence: Synthetic oligonucleotide
<400> 308
   ccagaacugu ggaagaug 18
<210> 309
   <211> 18
   <212> RNA
   <213> Artificial Sequence
<220>
   <223> Description of Artificial Sequence: Synthetic oligonucleotide
<400> 309
   uuuugccaga acugugga 18
<210> 310
   <211> 18
   <212> RNA
   <213> Artificial Sequence
<220>
   <223> Description of Artificial Sequence: Synthetic oligonucleotide
<400> 310
   aauaauuuug ccagaacu 18
<210> 311
   <211> 18
   <212> RNA
   <213> Artificial Sequence
<220>
   <223> Description of Artificial Sequence: Synthetic oligonucleotide
<400> 311
   uaaauuucuu cugggccu 18
<210> 312
   <211> 18
   <212> RNA
   <213> Artificial Sequence
<220>
   <223> Description of Artificial Sequence: Synthetic oligonucleotide
<400> 312
   acgcuuaaau uucuucug 18
<210> 313
   <211> 18
   <212> RNA
   <213> Artificial Sequence
<220>
   <223> Description of Artificial Sequence: Synthetic oligonucleotide
<400> 313
   auaagacgcu uaaauuuc 18
<210> 314
   <211> 18
   <212> RNA
   <213> Artificial Sequence
<220>
   <223> Description of Artificial Sequence: Synthetic oligonucleotide
<400> 314
   gcagcauaag acgcuuaa 18
<210> 315
   <211> 18
   <212> RNA
   <213> Artificial Sequence
<220>
   <223> Description of Artificial Sequence: Synthetic oligonucleotide
<400> 315
   ccgaugcagc auaagacg 18
<210> 316
   <211> 18
   <212> RNA
   <213> Artificial Sequence
<220>
   <223> Description of Artificial Sequence: Synthetic oligonucleotide
<400> 316
   uuuauccgau gcagcaua 18
<210> 317
   <211> 18
   <212> RNA
   <213> Artificial Sequence
<220>
   <223> Description of Artificial Sequence: Synthetic oligonucleotide
<400> 317
   cccacuuuau ccgaugca 18
<210> 318
   <211> 18
   <212> RNA
   <213> Artificial Sequence
<220>
   <223> Description of Artificial Sequence: Synthetic oligonucleotide
<400> 318
   uucaucccac uuuauccg 18
<210> 319
   <211> 18
   <212> RNA
   <213> Artificial Sequence
<220>
   <223> Description of Artificial Sequence: Synthetic oligonucleotide
<400> 319
   gucuguucau cccacuuu 18
<210> 320
   <211> 18
   <212> RNA
   <213> Artificial Sequence
<220>
   <223> Description of Artificial Sequence: Synthetic oligonucleotide
<400> 320
   uagaugucug uucauccc 18
<210> 321
   <211> 18
   <212> RNA
   <213> Artificial Sequence
<220>
   <223> Description of Artificial Sequence: Synthetic oligonucleotide
<400> 321
   uguguuagau gucuguuc 18
<210> 322
   <211> 18
   <212> RNA
   <213> Artificial Sequence
<220>
   <223> Description of Artificial Sequence: Synthetic oligonucleotide
<400> 322
   cccuuugugu uagauguc 18
<210> 323
   <211> 18
   <212> RNA
   <213> Artificial Sequence
<220>
   <223> Description of Artificial Sequence: Synthetic oligonucleotide
<400> 323
   caucucccuu uguguuag 18
<210> 324
   <211> 18
   <212> RNA
   <213> Artificial Sequence
<220>
   <223> Description of Artificial Sequence: Synthetic oligonucleotide
<400> 324
   aaguuccugg ucaaacac 18
<210> 325
   <211> 18
   <212> RNA
   <213> Artificial Sequence
<220>
   <223> Description of Artificial Sequence: Synthetic oligonucleotide
<400> 325
   gcaucaaguu ccugguca 18
<210> 326
   <211> 18
   <212> RNA
   <213> Artificial Sequence
<220>
   <223> Description of Artificial Sequence: Synthetic oligonucleotide
<400> 326
   ccagugcauc aaguuccu 18
<210> 327
   <211> 18
   <212> RNA
   <213> Artificial Sequence
<220>
   <223> Description of Artificial Sequence: Synthetic oligonucleotide
<400> 327
   aauuuccagu gcaucaag 18
<210> 328
   <211> 18
   <212> RNA
   <213> Artificial Sequence
<220>
   <223> Description of Artificial Sequence: Synthetic oligonucleotide
<400> 328
   gucucaauuu ccagugca 18
<210> 329
   <211> 18
   <212> RNA
   <213> Artificial Sequence
<220>
   <223> Description of Artificial Sequence: Synthetic oligonucleotide
<400> 329
   guacugucuc aauuucca 18
<210> 330
   <211> 18
   <212> RNA
   <213> Artificial Sequence
<220>
   <223> Description of Artificial Sequence: Synthetic oligonucleotide
<400> 330
   cuuuuguacu gucucaau 18
<210> 331
   <211> 18
   <212> RNA
   <213> Artificial Sequence
<220>
   <223> Description of Artificial Sequence: Synthetic oligonucleotide
<400> 331
   gucuccuuuu guacuguc 18
<210> 332
   <211> 18
   <212> RNA
   <213> Artificial Sequence
<220>
   <223> Description of Artificial Sequence: Synthetic oligonucleotide
<400> 332
   ggauugucuc cuuuugua 18
<210> 333
   <211> 18
   <212> RNA
   <213> Artificial Sequence
<220>
   <223> Description of Artificial Sequence: Synthetic oligonucleotide
<400> 333
   gggauggauu gucuccuu 18
<210> 334
   <211> 18
   <212> RNA
   <213> Artificial Sequence
<220>
   <223> Description of Artificial Sequence: Synthetic oligonucleotide
<400> 334
   ucuuauauga cuuucggg 18
<210> 335
   <211> 18
   <212> RNA
   <213> Artificial Sequence
<220>
   <223> Description of Artificial Sequence: Synthetic oligonucleotide
<400> 335
   uucaucuuau augacuuu 18
<210> 336
   <211> 18
   <212> RNA
   <213> Artificial Sequence
<220>
   <223> Description of Artificial Sequence: Synthetic oligonucleotide
<400> 336
   aagaguucau cuuauaug 18
<210> 337
   <211> 18
   <212> RNA
   <213> Artificial Sequence
<220>
   <223> Description of Artificial Sequence: Synthetic oligonucleotide
<400> 337
   acaggaagag uucaucuu 18
<210> 338
   <211> 18
   <212> RNA
   <213> Artificial Sequence
<220>
   <223> Description of Artificial Sequence: Synthetic oligonucleotide
<400> 338
   ucugcacagg aagaguuc 18
<210> 339
   <211> 18
   <212> RNA
   <213> Artificial Sequence
<220>
   <223> Description of Artificial Sequence: Synthetic oligonucleotide
<400> 339
   ggauaucugc acaggaag 18
<210> 340
   <211> 18
   <212> RNA
   <213> Artificial Sequence
<220>
   <223> Description of Artificial Sequence: Synthetic oligonucleotide
<400> 340
   gagcaggaua ucugcaca 18
<210> 341
   <211> 18
   <212> RNA
   <213> Artificial Sequence
<220>
   <223> Description of Artificial Sequence: Synthetic oligonucleotide
<400> 341
   gcaaagagca ggauaucu 18
<210> 342
   <211> 18
   <212> RNA
   <213> Artificial Sequence
<220>
   <223> Description of Artificial Sequence: Synthetic oligonucleotide
<400> 342
   aggaggcaaa gagcagga 18
<210> 343
   <211> 18
   <212> RNA
   <213> Artificial Sequence
<220>
   <223> Description of Artificial Sequence: Synthetic oligonucleotide
<400> 343
   cuuauaggag gcaaagag 18
<210> 344
   <211> 18
   <212> RNA
   <213> Artificial Sequence
<220>
   <223> Description of Artificial Sequence: Synthetic oligonucleotide
<400> 344
   uuccacuuau aggaggca 18
<210> 345
   <211> 18
   <212> RNA
   <213> Artificial Sequence
<220>
   <223> Description of Artificial Sequence: Synthetic oligonucleotide
<400> 345
   agacauucca cuuauagg 18
<210> 346
   <211> 18
   <212> RNA
   <213> Artificial Sequence
<220>
   <223> Description of Artificial Sequence: Synthetic oligonucleotide
<400> 346
   ccgggagaca uuccacuu 18
<210> 347
   <211> 18
   <212> RNA
   <213> Artificial Sequence
<220>
   <223> Description of Artificial Sequence: Synthetic oligonucleotide
<400> 347
   gagggccggg agacauuc 18
<210> 348
   <211> 18
   <212> RNA
   <213> Artificial Sequence
<220>
   <223> Description of Artificial Sequence: Synthetic oligonucleotide
<400> 348
   gcaaugaggg ccgggaga 18
<210> 349
   <211> 18
   <212> RNA
   <213> Artificial Sequence
<220>
   <223> Description of Artificial Sequence: Synthetic oligonucleotide
<400> 349
   agccagcaau gagggccg 18
<210> 350
   <211> 18
   <212> RNA
   <213> Artificial Sequence
<220>
   <223> Description of Artificial Sequence: Synthetic oligonucleotide
<400> 350
   gagucagcca gcaaugag 18
<210> 351
   <211> 18
   <212> RNA
   <213> Artificial Sequence
<220>
   <223> Description of Artificial Sequence: Synthetic oligonucleotide
<400> 351
   gggcuggguc cugaggca 18
<210> 352
   <211> 18
   <212> RNA
   <213> Artificial Sequence
<220>
   <223> Description of Artificial Sequence: Synthetic oligonucleotide
<400> 352
   gccuagggcu ggguccug 18
<210> 353
   <211> 18
   <212> RNA
   <213> Artificial Sequence
<220>
   <223> Description of Artificial Sequence: Synthetic oligonucleotide
<400> 353
   uggcugccua gggcuggg 18
<210> 354
   <211> 18
   <212> RNA
   <213> Artificial Sequence
<220>
   <223> Description of Artificial Sequence: Synthetic oligonucleotide
<400> 354
   uguccuggcu gccuaggg 18
<210> 355
   <211> 18
   <212> RNA
   <213> Artificial Sequence
<220>
   <223> Description of Artificial Sequence: Synthetic oligonucleotide
<400> 355
   gaaagugucc uggcugcc 18
<210> 356
   <211> 18
   <212> RNA
   <213> Artificial Sequence
<220>
   <223> Description of Artificial Sequence: Synthetic oligonucleotide
<400> 356
   aaaacgaaag uguccugg 18
<210> 357
   <211> 18
   <212> RNA
   <213> Artificial Sequence
<220>
   <223> Description of Artificial Sequence: Synthetic oligonucleotide
<400> 357
   acaggaaaac gaaagugu 18
<210> 358
   <211> 18
   <212> RNA
   <213> Artificial Sequence
<220>
   <223> Description of Artificial Sequence: Synthetic oligonucleotide
<400> 358
   gaagaacagg aaaacgaa 18
<210> 359
   <211> 18
   <212> RNA
   <213> Artificial Sequence
<220>
   <223> Description of Artificial Sequence: Synthetic oligonucleotide
<400> 359
   ggcuagaaga acaggaaa 18
<210> 360
   <211> 18
   <212> RNA
   <213> Artificial Sequence
<220>
   <223> Description of Artificial Sequence: Synthetic oligonucleotide
<400> 360
   ugcagggcua gaagaaca 18
<210> 361
   <211> 18
   <212> RNA
   <213> Artificial Sequence
<220>
   <223> Description of Artificial Sequence: Synthetic oligonucleotide
<400> 361
   aaaguugcag ggcuagaa 18
<210> 362
   <211> 18
   <212> RNA
   <213> Artificial Sequence
<220>
   <223> Description of Artificial Sequence: Synthetic oligonucleotide
<400> 362
   uuccuaaagu ugcagggc 18
<210> 363
   <211> 18
   <212> RNA
   <213> Artificial Sequence
<220>
   <223> Description of Artificial Sequence: Synthetic oligonucleotide
<400> 363
   gacaauuccu aaaguugc 18
<210> 364
   <211> 18
   <212> RNA
   <213> Artificial Sequence
<220>
   <223> Description of Artificial Sequence: Synthetic oligonucleotide
<400> 364
   gacaggacaa uuccuaaa 18
<210> 365
   <211> 18
   <212> RNA
   <213> Artificial Sequence
<220>
   <223> Description of Artificial Sequence: Synthetic oligonucleotide
<400> 365
   aggcagacag gacaauuc 18
<210> 366
   <211> 18
   <212> RNA
   <213> Artificial Sequence
<220>
   <223> Description of Artificial Sequence: Synthetic oligonucleotide
<400> 366
   aacaaaggca gacaggac 18
<210> 367
   <211> 18
   <212> RNA
   <213> Artificial Sequence
<220>
   <223> Description of Artificial Sequence: Synthetic oligonucleotide
<400> 367
   uuugaaacaa aggcagac 18
<210> 368
   <211> 18
   <212> RNA
   <213> Artificial Sequence
<220>
   <223> Description of Artificial Sequence: Synthetic oligonucleotide
<400> 368
   ccaaguuuga aacaaagg 18
<210> 369
   <211> 18
   <212> RNA
   <213> Artificial Sequence
<220>
   <223> Description of Artificial Sequence: Synthetic oligonucleotide
<400> 369
   uggcuccaag uuugaaac 18
<210> 370
   <211> 18
   <212> RNA
   <213> Artificial Sequence
<220>
   <223> Description of Artificial Sequence: Synthetic oligonucleotide
<400> 370
   agcacuggcu ccaaguuu 18
<210> 371
   <211> 18
   <212> RNA
   <213> Artificial Sequence
<220>
   <223> Description of Artificial Sequence: Synthetic oligonucleotide
<400> 371
   agcguagcac uggcucca 18
<210> 372
   <211> 18
   <212> RNA
   <213> Artificial Sequence
<220>
   <223> Description of Artificial Sequence: Synthetic oligonucleotide
<400> 372
   cuccaagcgu agcacugg 18
<210> 373
   <211> 18
   <212> RNA
   <213> Artificial Sequence
<220>
   <223> Description of Artificial Sequence: Synthetic oligonucleotide
<400> 373
   acaggcucca agcguagc 18
<210> 374
   <211> 18
   <212> RNA
   <213> Artificial Sequence
<220>
   <223> Description of Artificial Sequence: Synthetic oligonucleotide
<400> 374
   uguugacagg cuccaagc 18
<210> 375
   <211> 18
   <212> RNA
   <213> Artificial Sequence
<220>
   <223> Description of Artificial Sequence: Synthetic oligonucleotide
<400> 375
   aaggguguug acaggcuc 18
<210> 376
   <211> 18
   <212> RNA
   <213> Artificial Sequence
<220>
   <223> Description of Artificial Sequence: Synthetic oligonucleotide
<400> 376
   ugacuaaggg uguugaca 18
<210> 377
   <211> 18
   <212> RNA
   <213> Artificial Sequence
<220>
   <223> Description of Artificial Sequence: Synthetic oligonucleotide
<400> 377
   agaucugacu aagggugu 18
<210> 378
   <211> 18
   <212> RNA
   <213> Artificial Sequence
<220>
   <223> Description of Artificial Sequence: Synthetic oligonucleotide
<400> 378
   ucagcagauc ugacuaag 18
<210> 379
   <211> 18
   <212> RNA
   <213> Artificial Sequence
<220>
   <223> Description of Artificial Sequence: Synthetic oligonucleotide
<400> 379
   gagaaucagc agaucuga 18
<210> 380
   <211> 18
   <212> RNA
   <213> Artificial Sequence
<220>
   <223> Description of Artificial Sequence: Synthetic oligonucleotide
<400> 380
   ccccagagaa ucagcaga 18
<210> 381
   <211> 18
   <212> RNA
   <213> Artificial Sequence
<220>
   <223> Description of Artificial Sequence: Synthetic oligonucleotide
<400> 381
   caggacccca gagaauca 18
<210> 382
   <211> 18
   <212> RNA
   <213> Artificial Sequence
<220>
   <223> Description of Artificial Sequence: Synthetic oligonucleotide
<400> 382
   guugacaggc uccaagcg 18
<210> 383
   <211> 18
   <212> RNA
   <213> Artificial Sequence
<220>
   <223> Description of Artificial Sequence: Synthetic oligonucleotide
<400> 383
   aggguguuga caggcucc 18
<210> 384
   <211> 18
   <212> RNA
   <213> Artificial Sequence
<220>
   <223> Description of Artificial Sequence: Synthetic oligonucleotide
<400> 384
   gacuaagggu guugacag 18
<210> 385
   <211> 18
   <212> RNA
   <213> Artificial Sequence
<220>
   <223> Description of Artificial Sequence: Synthetic oligonucleotide
<400> 385
   gaucugacua aggguguu 18
<210> 386
   <211> 18
   <212> RNA
   <213> Artificial Sequence
<220>
   <223> Description of Artificial Sequence: Synthetic oligonucleotide
<400> 386
   cagcagaucu gacuaagg 18
<210> 387
   <211> 18
   <212> RNA
   <213> Artificial Sequence
<220>
   <223> Description of Artificial Sequence: Synthetic oligonucleotide
<400> 387
   agaaucagca gaucugac 18
<210> 388
   <211> 18
   <212> RNA
   <213> Artificial Sequence
<220>
   <223> Description of Artificial Sequence: Synthetic oligonucleotide
<400> 388
   cccagagaau cagcagau 18
<210> 389
   <211> 18
   <212> RNA
   <213> Artificial Sequence
<220>
   <223> Description of Artificial Sequence: Synthetic oligonucleotide
<400> 389
   aggaccccag agaaucag 18
<210> 390
   <211> 18
   <212> RNA
   <213> Artificial Sequence
<220>
   <223> Description of Artificial Sequence: Synthetic oligonucleotide
<400> 390
   ucagcaggac cccagaga 18
<210> 391
   <211> 18
   <212> RNA
   <213> Artificial Sequence
<220>
   <223> Description of Artificial Sequence: Synthetic oligonucleotide
<400> 391
   ccaggucagc aggacccc 18
<210> 392
   <211> 18
   <212> RNA
   <213> Artificial Sequence
<220>
   <223> Description of Artificial Sequence: Synthetic oligonucleotide
<400> 392
   uuguuccagg ucagcagg 18
<210> 393
   <211> 18
   <212> RNA
   <213> Artificial Sequence
<220>
   <223> Description of Artificial Sequence: Synthetic oligonucleotide
<400> 393
   ccaacuuguu ccagguca 18
<210> 394
   <211> 18
   <212> RNA
   <213> Artificial Sequence
<220>
   <223> Description of Artificial Sequence: Synthetic oligonucleotide
<400> 394
   cuccaccaac uuguucca 18
<210> 395
   <211> 18
   <212> RNA
   <213> Artificial Sequence
<220>
   <223> Description of Artificial Sequence: Synthetic oligonucleotide
<400> 395
   acccacucca ccaacuug 18
<210> 396
   <211> 18
   <212> RNA
   <213> Artificial Sequence
<220>
   <223> Description of Artificial Sequence: Synthetic oligonucleotide
<400> 396
   aucccaccca cuccacca 18
<210> 397
   <211> 18
   <212> RNA
   <213> Artificial Sequence
<220>
   <223> Description of Artificial Sequence: Synthetic oligonucleotide
<400> 397
   aaaccauccc acccacuc 18
<210> 398
   <211> 18
   <212> RNA
   <213> Artificial Sequence
<220>
   <223> Description of Artificial Sequence: Synthetic oligonucleotide
<400> 398
   ucccaaaacc aucccacc 18
<210> 399
   <211> 18
   <212> RNA
   <213> Artificial Sequence
<220>
   <223> Description of Artificial Sequence: Synthetic oligonucleotide
<400> 399
   uuaaauccca aaaccauc 18
<210> 400
   <211> 18
   <212> RNA
   <213> Artificial Sequence
<220>
   <223> Description of Artificial Sequence: Synthetic oligonucleotide
<400> 400
   accacuuaaa ucccaaaa 18
<210> 401
   <211> 18
   <212> RNA
   <213> Artificial Sequence
<220>
   <223> Description of Artificial Sequence: Synthetic oligonucleotide
<400> 401
   ccagaaccac uuaaaucc 18
<210> 402
   <211> 18
   <212> RNA
   <213> Artificial Sequence
<220>
   <223> Description of Artificial Sequence: Synthetic oligonucleotide
<400> 402
   cagaaccaga accacuua 18
<210> 403
   <211> 18
   <212> RNA
   <213> Artificial Sequence
<220>
   <223> Description of Artificial Sequence: Synthetic oligonucleotide
<400> 403
   guccccagaa ccagaacc 18
<210> 404
   <211> 18
   <212> RNA
   <213> Artificial Sequence
<220>
   <223> Description of Artificial Sequence: Synthetic oligonucleotide
<400> 404
   ccaauguccc cagaacca 18
<210> 405
   <211> 18
   <212> RNA
   <213> Artificial Sequence
<220>
   <223> Description of Artificial Sequence: Synthetic oligonucleotide
<400> 405
   cauaaccaau guccccag 18
<210> 406
   <211> 18
   <212> RNA
   <213> Artificial Sequence
<220>
   <223> Description of Artificial Sequence: Synthetic oligonucleotide
<400> 406
   augggcauaa ccaauguc 18
<210> 407
   <211> 18
   <212> RNA
   <213> Artificial Sequence
<220>
   <223> Description of Artificial Sequence: Synthetic oligonucleotide
<400> 407
   aaaccauggg cauaacca 18
<210> 408
   <211> 18
   <212> RNA
   <213> Artificial Sequence
<220>
   <223> Description of Artificial Sequence: Synthetic oligonucleotide
<400> 408
   cuaagaaacc augggcau 18
<210> 409
   <211> 18
   <212> RNA
   <213> Artificial Sequence
<220>
   <223> Description of Artificial Sequence: Synthetic oligonucleotide
<400> 409
   agcuucuaag aaaccaug 18
<210> 410
   <211> 18
   <212> RNA
   <213> Artificial Sequence
<220>
   <223> Description of Artificial Sequence: Synthetic oligonucleotide
<400> 410
   guucaagcuu cuaagaaa 18
<210> 411
   <211> 18
   <212> RNA
   <213> Artificial Sequence
<220>
   <223> Description of Artificial Sequence: Synthetic oligonucleotide
<400> 411
   ggugcugucc aucacauc 18
<210> 412
   <211> 18
   <212> RNA
   <213> Artificial Sequence
<220>
   <223> Description of Artificial Sequence: Synthetic oligonucleotide
<400> 412
   gugguggugc uguccauc 18
<210> 413
   <211> 18
   <212> RNA
   <213> Artificial Sequence
<220>
   <223> Description of Artificial Sequence: Synthetic oligonucleotide
<400> 413
   ucuggguggu ggugcugu 18
<210> 414
   <211> 18
   <212> RNA
   <213> Artificial Sequence
<220>
   <223> Description of Artificial Sequence: Synthetic oligonucleotide
<400> 414
   guauuucugg gugguggu 18
<210> 415
   <211> 18
   <212> RNA
   <213> Artificial Sequence
<220>
   <223> Description of Artificial Sequence: Synthetic oligonucleotide
<400> 415
   auccaguauu ucugggug 18
<210> 416
   <211> 18
   <212> RNA
   <213> Artificial Sequence
<220>
   <223> Description of Artificial Sequence: Synthetic oligonucleotide
<400> 416
   ugucaaucca guauuucu 18
<210> 417
   <211> 18
   <212> RNA
   <213> Artificial Sequence
<220>
   <223> Description of Artificial Sequence: Synthetic oligonucleotide
<400> 417
   cuggauguca auccagua 18
<210> 418
   <211> 18
   <212> RNA
   <213> Artificial Sequence
<220>
   <223> Description of Artificial Sequence: Synthetic oligonucleotide
<400> 418
   cgcaacugga ugucaauc 18
<210> 419
   <211> 18
   <212> RNA
   <213> Artificial Sequence
<220>
   <223> Description of Artificial Sequence: Synthetic oligonucleotide
<400> 419
   cccagcgcaa cuggaugu 18
<210> 420
   <211> 18
   <212> RNA
   <213> Artificial Sequence
<220>
   <223> Description of Artificial Sequence: Synthetic oligonucleotide
<400> 420
   guccccccag cgcaacug 18
<210> 421
   <211> 18
   <212> RNA
   <213> Artificial Sequence
<220>
   <223> Description of Artificial Sequence: Synthetic oligonucleotide
<400> 421
   ucauaguccc cccagcgc 18
<210> 422
   <211> 18
   <212> RNA
   <213> Artificial Sequence
<220>
   <223> Description of Artificial Sequence: Synthetic oligonucleotide
<400> 422
   gggaaucaua gucccccc 18
<210> 423
   <211> 18
   <212> RNA
   <213> Artificial Sequence
<220>
   <223> Description of Artificial Sequence: Synthetic oligonucleotide
<400> 423
   gucgugggaa ucauaguc 18
<210> 424
   <211> 18
   <212> RNA
   <213> Artificial Sequence
<220>
   <223> Description of Artificial Sequence: Synthetic oligonucleotide
<400> 424
   ucaaugucgu gggaauca 18
<210> 425
   <211> 18
   <212> RNA
   <213> Artificial Sequence
<220>
   <223> Description of Artificial Sequence: Synthetic oligonucleotide
<400> 425
   agcgcucaau gucguggg 18
<210> 426
   <211> 18
   <212> RNA
   <213> Artificial Sequence
<220>
   <223> Description of Artificial Sequence: Synthetic oligonucleotide
<400> 426
   ggcguagcgc ucaauguc 18
<210> 427
   <211> 18
   <212> RNA
   <213> Artificial Sequence
<220>
   <223> Description of Artificial Sequence: Synthetic oligonucleotide
<400> 427
   gcccgggcgu agcgcuca 18
<210> 428
   <211> 18
   <212> RNA
   <213> Artificial Sequence
<220>
   <223> Description of Artificial Sequence: Synthetic oligonucleotide
<400> 428
   acuuggcccg ggcguagc 18
<210> 429
   <211> 18
   <212> RNA
   <213> Artificial Sequence
<220>
   <223> Description of Artificial Sequence: Synthetic oligonucleotide
<400> 429
   caggaacuug gcccgggc 18
<210> 430
   <211> 18
   <212> RNA
   <213> Artificial Sequence
<220>
   <223> Description of Artificial Sequence: Synthetic oligonucleotide
<400> 430
   uaguccagga acuuggcc 18
<210> 431
   <211> 18
   <212> RNA
   <213> Artificial Sequence
<220>
   <223> Description of Artificial Sequence: Synthetic oligonucleotide
<400> 431
   ugguguaguc caggaacu 18
<210> 432
   <211> 18
   <212> RNA
   <213> Artificial Sequence
<220>
   <223> Description of Artificial Sequence: Synthetic oligonucleotide
<400> 432
   gucgguggug uaguccag 18
<210> 433
   <211> 18
   <212> RNA
   <213> Artificial Sequence
<220>
   <223> Description of Artificial Sequence: Synthetic oligonucleotide
<400> 433
   auguugucgg ugguguag 18
<210> 434
   <211> 18
   <212> RNA
   <213> Artificial Sequence
<220>
   <223> Description of Artificial Sequence: Synthetic oligonucleotide
<400> 434
   uacucauguu gucggugg 18
<210> 435
   <211> 18
   <212> RNA
   <213> Artificial Sequence
<220>
   <223> Description of Artificial Sequence: Synthetic oligonucleotide
<400> 435
   guagauacuc auguuguc 18
<210> 436
   <211> 18
   <212> RNA
   <213> Artificial Sequence
<220>
   <223> Description of Artificial Sequence: Synthetic oligonucleotide
<400> 436
   gaaggguaga uacucaug 18
<210> 437
   <211> 18
   <212> RNA
   <213> Artificial Sequence
<220>
   <223> Description of Artificial Sequence: Synthetic oligonucleotide
<400> 437
   ugggcgaagg guagauac 18
<210> 438
   <211> 18
   <212> RNA
   <213> Artificial Sequence
<220>
   <223> Description of Artificial Sequence: Synthetic oligonucleotide
<400> 438
   accugugggc gaagggua 18
<210> 439
   <211> 18
   <212> RNA
   <213> Artificial Sequence
<220>
   <223> Description of Artificial Sequence: Synthetic oligonucleotide
<400> 439
   aguacaccug ugggcgaa 18
<210> 440
   <211> 18
   <212> RNA
   <213> Artificial Sequence
<220>
   <223> Description of Artificial Sequence: Synthetic oligonucleotide
<400> 440
   cgaugaguac accugugg 18
<210> 441
   <211> 18
   <212> RNA
   <213> Artificial Sequence
<220>
   <223> Description of Artificial Sequence: Synthetic oligonucleotide
<400> 441
   aauggcgaug aguacacc 18
<210> 442
   <211> 18
   <212> RNA
   <213> Artificial Sequence
<220>
   <223> Description of Artificial Sequence: Synthetic oligonucleotide
<400> 442
   aggucaaugg cgaugagu 18
<210> 443
   <211> 18
   <212> RNA
   <213> Artificial Sequence
<220>
   <223> Description of Artificial Sequence: Synthetic oligonucleotide
<400> 443
   aggccagguc aauggcga 18
<210> 444
   <211> 18
   <212> RNA
   <213> Artificial Sequence
<220>
   <223> Description of Artificial Sequence: Synthetic oligonucleotide
<400> 444
   guuauaggcc aggucaau 18
<210> 445
   <211> 18
   <212> RNA
   <213> Artificial Sequence
<220>
   <223> Description of Artificial Sequence: Synthetic oligonucleotide
<400> 445
   ugcaaguuau aggccagg 18
<210> 446
   <211> 474
   <212> DNA
   <213> Homo sapiens
<400> 446
   agttcgaaat ttgagcaacc cagccaagaa gttcaagatt gaagccaatg ctgggcaact    60
   gtacctgaca ggggtggtgg tactgcacaa ggatgtcaac gtggtagtag tggaaggggg   120
   tgagtctgaa aaacttgagg gagactgtcc ccagacaacc ctaggcttag agtgattagc   180
   attgtgggga gaaaggagaa aaagatcgta ttttagtgcc atacctgata taatatgcta   240
   ggtctctgct tctgtgtttt ttggagtaga aggccctaag atgtggttcc tgagattgct   300
   tcaactacca cattaatgtc tgagctcaga ggttctttct accccatcat cttccacagt   360
   tctggcaaaa ttattcttct ctgtttccag gccccaaggc ccagaagaaa tttaagcgtc   420
   ttatgctgca tcggataaag tgggatgaac agacatctaa cacaaaggga gatg         474
<210> 447
   <211> 1703
   <212> DNA
   <213> Homo sapiens
<400> 447
   agcgcatgaa gaggccaacg ctgcccgaaa actcacagca gaacagagaa aggtcaagaa 60
   aattaaaaag cttaaagaag acatttcaca gggggtacac atatctgtat ataggtaggt 120
   gtccatactg ggccaaactt ccccttagaa ttactaaaac attttccaag tgcttgaggc 180
   agaatcattg tggcctaaaa gcatacctaa caccagttcc tactctcttt taatgaccct 240
   agtgtggatc aagagctagt aaatcttgta aaatagcgag agttaatgac ttatgtttat 300
   ggttacccag ttcttccctc tcaagtcttc tttttttttt taccagaata ttagttctga 360
   acaagtcaat atacttgttc aggccaagat gtgaactaaa gattgtatgt cctttgagaa 420
   ctataaaata tcatcctatt tctttttttt ttcatctttt tttttttaaa tggtctgagt 480
   ttgcttccaa catccatgga agcattacaa attttctttc ctccgtagtc atatccttac 540
   cctgatgtat tgctgtgttt catcctcact ccaaaatgca ttttatatgt tgtaaatatt 600
   tttctacctt tgtgttgatt taatgttttt attcttttat gttgagtaca tgtgtatctt 660
   ctatttttaa aatgtgctta tttctattaa ctagttcttt attataaaag tggtacatta 720
   tcacaaaaat agtgaatgct tattaagatt taaaaaattg tctgacaaca cagaagaata 780
   cttactcaaa atgtctcctt tctattctct gatcccttag aaccactctc tttaggtaat 840
   tgctattaac agtttttcac atatcctcct agaaaatttt catgcctata tagtgatcct 900
   atatatatat tttatttatt tatttattta ttttgagacg gagtttcagt cttgttgccc 960
   aggcaggagt gcaatggcgc gatctcagcg cactgcaacc tctgcctccc aagttcaagc 1020
   aattctcctg cctcagcctc ctgagtagct aggattacag gcacgcaccc ccacgcccgg 1080
   ctaatttttg tatttttagt agagatgggg tttcaccatg ttggccaggc tggtcttaaa 1140
   ctcctgacct cgtgatctgc ccactttggc ctcccaaagt gctgggatta caggcatgag 1200
   ccaccgcacc cagccaagta atcctatatt ataagagtct aagggtctag aggacaaaaa 1260
   gtctgtaggt ctttacattg tcaagaacaa tgagattgta agtatttttg gatgatagta 1320
   aatacctctc cctgactctt agagtaaact ttggttagta atgtaatggt taatgtaaag 1380
   tgctagactt tgaagaaatg taagagtgtg gaaatatttc tagactcatc aactctacct 1440
   tttaagcatt catctatttg tttactccag aggcctttgt ttatagagag gaaaccattt 1500
   tggattcatc tttttacttg ccactccatt caggcagctg ctgatgctaa aatggaagac 1560
   ataactaaac tttcccttct ccagagttcg aaatttgagc aacccagcca agaagttcaa 1620
   gattgaagcc aatgctgggc aactgtacct gacaggggtg gtggtactgc acaaggatgt 1680
   caacgtggta gtagtggaag ggg 1703
<210> 448
   <211> 645
   <212> DNA
   <213> Homo sapiens
<400> 448
   gtgtttgacc aggaacttga tgcactggaa attgagacag tacaaaagga gacaatccat       60
   ccccgaaagt catataagat gaactcttcc tgtgcagata tcctgctctt tgcctcctat      120
   aagtggaatg tctcccggcc ctcattgctg gctgactcca agtaagtgcc tcaggaccca      180
   gccctaggca gccaggacac tttcgttttc ctgttcttct agccctgcaa ctttaggaat      240
   tgtcctgtct gcctttgttt caaacttgga gccagtgcta cgcttggagc ctgtcaacac      300
   ccttagtcag atctgctgat tctctggggt cctgctgacc tggaacaagt tggtggagtg      360
   ggtgggatgg ttttgggatt taagtggttc tggttctggg gacattggtt atgcccatgg      420
   tttcttagaa gcttgaaccc tcttcatcct cagggatgtg atggacagca ccaccaccca      480
   gaaatactgg attgacatcc agttgcgctg gggggactat gattcccacg acattgagcg      540
   ctacgcccgg gccaagttcc tggactacac caccgacaac atgagtatct acccttcgcc      600
   cacaggtgta ctcatcgcca ttgacctggc ctataacttg cacag                      645

## Description

The present invention related to pharmaceutical compositions comprising antisense oligonucleotides (ASOs) and to pharmaceutical compositions for use in treating Retinitis Pigmentosa 13 (RP13).

### BACKGROUND OF THE INVENTION

Retinitis pigmentosa (RP) describes a group of diseases that have similar clinical phenotypes and are associated with genetically heterogeneous causes. RP frequently manifests as a loss of night vision, and can progress to peripheral blindness, resulting in tunnel vision. As the disorder progresses, subjects may lose a significant portion of their photoreceptors before experiencing loss of visual acuity, and can eventually experience complete blindness. RP-associated genes PRPF3, PRPF8, PRPF31, and PAP1 are involved in the assembly of spliceosomes. Although the functional properties of several RP-associated genes have been extensively studied, genotype-phenotype correlations are incompletely understood. However, it is known that disease-causing mutations in the PRPF3, PRPF8, PRPF31 and PAP1 genes have a dominant pattern of inheritance (Berger et al., 2010, Progress in Retinal Eye Research 29, 335-375).

### SUMMARY

The invention is defined by the appended claims. The present invention relates to pharmaceutical compositions comprising ASOs as set out in the claims and to pharmaceutical compositions for use in treating retinitis pigmentosa as set out in the claims. Other material disclosed herein is for the assistance of the skilled person in understanding and practicing the invention. For the avoidance of doubt it is noted that the invention herein does not extend to methods of treatment of the human body. Also for the avoidance of doubt it is noted that the invention herein does not extend to the use of pharmaceutical compositions for the treatment of Retinitis pigmentosa 18 (RP18). Disclosed herein are methods of treating Retinitis Pigmentosa 18 (RP18) or Retinitis Pigmentosa 13 (RP13) in a subject in need thereof, by increasing the expression of a target protein or functional RNA by cells of the subject, wherein the cells have a retained-intron-containing pre-mRNA (RIC pre-mRNA), the RIC pre-mRNA comprising a retained intron, an exon flanking the 5' splice site, an exon flanking the 3' splice site, and wherein the RIC pre-mRNA encodes the target protein or functional RNA, the method comprising contacting the cells of the subject with an antisense oligomer (ASO) complementary to a targeted portion of the RIC pre-mRNA encoding the target protein or functional RNA, whereby the retained intron is constitutively spliced from the RIC pre-mRNA encoding the target protein or functional RNA, thereby increasing the level of mRNA encoding the target protein or functional RNA, and increasing the expression of the target protein or functional RNA in the cells of the subject.

Also disclosed herein are methods of increasing expression of a target protein, wherein the target protein is PRPF3 or PRPF8, by cells having a retained-intron-containing pre-mRNA (RIC pre-mRNA), the RIC pre-mRNA comprising a retained intron, an exon flanking the 5' splice site of the retained intron, an exon flanking the 3' splice site of the retained intron, and wherein the RIC pre-mRNA encodes PRPF3 or PRPF8protein, the method comprising contacting the cells with an antisense oligomer (ASO) complementary to a targeted portion of the RIC pre-mRNA encoding PRPF3 or PRPF8 protein, whereby the retained intron is constitutively spliced from the RIC pre-mRNA encoding PRPF3 or PRPF8 protein, thereby increasing the level of mRNA encoding PRPF3 or PRPF8 protein, and increasing the expression of PRPF3 or PRPF8 protein in the cells.

In some cases of any of the aforementioned methods, when the method is a method of treating RP18 the target protein is PRPF3. In some cases of any of the aforementioned methods, when the method is a method of treating RP13 the target protein is PRPF8. In some cases, the target protein or the functional RNA is a compensating protein or a compensating functional RNA that functionally augments or replaces a target protein or functional RNA that is deficient in amount or activity in the subject. In some cases, the cells are in or from a subject having a condition caused by a deficient amount or activity of PRPF3 or PRPF8 protein.

In some cases of any of the aforementioned methods, the deficient amount of the target protein is caused by haploinsufficiency of the target protein, wherein the subject has a first allele encoding a functional target protein, and a second allele from which the target protein is not produced, or a second allele encoding a nonfunctional target protein, and wherein the antisense oligomer binds to a targeted portion of a RIC pre-mRNA transcribed from the first allele. In some cases, the subject has a condition caused by a disorder resulting from a deficiency in the amount or function of the target protein, wherein the subject has (a) a first mutant allele from which (i) the target protein is produced at a reduced level compared to production from a wild-type allele, (ii) the target protein is produced in a form having reduced function compared to an equivalent wild-type protein, or (iii) the target protein is not produced, and (b) a second mutant allele from which (i) the target protein is produced at a reduced level compared to production from a wild-type allele, (ii) the target protein is produced in a form having reduced function compared to an equivalent wild-type protein, or (iii) the target protein is not produced, and wherein when the subject has a first mutant allele a(iii), the second mutant allele is b(i) or b(ii), and wherein when the subject has a second mutant allele b(iii), the first mutant allele is a(i) or a(ii), and wherein the RIC pre-mRNA is transcribed from either the first mutant allele that is a(i) or a(ii), and/or the second allele that is b(i) or b(ii). In some cases, the target protein is produced in a form having reduced function compared to the equivalent wild-type protein. In some cases, the target protein is produced in a form that is fully-functional compared to the equivalent wild-type protein.

In some cases of any of the aforementioned methods, the targeted portion of the RIC pre-mRNA is in the retained intron within the region +6 relative to the 5' splice site of the retained intron to -16 relative to the 3' splice site of the retained intron. In some cases, the targeted portion of the RIC pre-mRNA is in the retained intron within the region +498 relative to the 5' splice site of the retained intron to -496 relative to the 3' splice site of the retained intron. In some cases, the targeted portion of the RIC pre-mRNA is in the retained intron within the region +498 relative to the 5' splice site of the retained intron to -496 relative to the 3' splice site of the retained intron.

In some cases of any of the aforementioned methods, the target protein is PRPF3. In some cases, the targeted portion of the RIC pre-mRNA comprises a sequence that is at least about 80%, 85%, 90%, 95%, 97%, or 100% complimentary to any one of SEQ ID NOs 5-323. In some cases, the in the targeted portion of the RIC pre-mRNA comprises a sequence with at least 80%, 85%, 90%, 95%, 97%, or 100% sequence identity to a region comprising at least 8 contiguous nucleic acids of SEQ ID NO 447 or SEQ ID NO 446. In some cases, the ASO comprises a sequence with at least about 80%, 85%, 90%, 95%, 97%, or 100% sequence identity to any one of SEQ ID NOs 5-323. In some cases, the RIC pre-mRNA comprises a sequence with at least about 80%, 85%, 90%, 95%, 97%, or 100% sequence identity to SEQ ID NO 3. In some cases, the RIC pre-mRNA is encoded by a genetic sequence with at least about 80%, 85%, 90%, 95%, 97%, or 100% sequence identity to SEQ ID NO 1.

In some cases, the target protein is PRPF8. In some cases, the targeted portion of the RIC pre-mRNA is in the retained intron within the region +156 relative to the 5' splice site of the retained intron to -156 relative to the 3' splice site of the retained intron. In some cases, the targeted portion of the RIC pre-mRNA comprises a sequence that is at least about 80%, 85%, 90%, 95%, 97%, or 100% complimentary to any one of SEQ ID NOs 324-445. In some cases, the targeted portion of the RIC pre-mRNA comprises a sequence with at least 80%, 85%, 90%, 95%, 97%, or 100% sequence identity to a region comprising at least 8 contiguous nucleic acids of SEQ ID NO 448. In some cases, the ASO comprises a sequence with at least about 80%, 85%, 90%, 95%, 97%, or 100% sequence identity to any one of SEQ ID NOs 234-445. In some cases, the RIC pre-mRNA comprises a sequence with at least about 80%, 85%, 90%, 95%, 97%, or 100% sequence identity to SEQ ID NO 4. In some cases, the RIC pre-mRNA is encoded by a genetic sequence with at least about 80%, 85%, 90%, 95%, 97%, or 100% sequence identity to SEQ ID NO 2.

In some cases of any of the aforementioned methods, the targeted portion of the RIC pre-mRNA is in the retained intron within: (a) the region +6 to +100 relative to the 5' splice site of the retained intron; or (b) the region -16 to -100 relative to the 3' splice site of the retained intron. In some cases, the antisense oligomer targets a portion of the RIC pre-mRNA that is within the region about 100 nucleotides downstream of the 5' splice site of the at least one retained intron, to about 100 nucleotides upstream of the 3' splice site of the at least one retained intron. In some cases, the targeted portion of the RIC pre-mRNA is the retained intron within the: (a) the region +2e to -4e in the exon flanking the 5' splice site of the retained intron; or (b) the region +2e to - 4e in the exon flanking the 3' splice site of the retained intron.

In some cases of any of the aforementioned methods, the antisense oligomer does not increase the amount of the target protein or the functional RNA by modulating alternative splicing of pre-mRNA transcribed from a gene encoding the functional RNA or target protein. In some cases, the antisense oligomer does not increase the amount of the target protein or the functional RNA by modulating aberrant splicing resulting from mutation of the gene encoding the target protein or the functional RNA. In some cases, the RIC pre-mRNA was produced by partial splicing of a full-length pre-mRNA or partial splicing of a wild-type pre-mRNA. In some cases, the mRNA encoding the target protein or functional RNA is a full-length mature mRNA, or a wild-type mature mRNA. In some cases, the target protein produced is full-length protein, or wild-type protein. In some cases, the total amount of the mRNA encoding the target protein or functional RNA produced in the cell contacted with the antisense oligomer is increased about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1.1-fold, at least about 1.5-fold, at least about 2-fold, at least about 2.5-fold, at least about 3-fold, at least about 3.5-fold, at least about 4-fold, at least about 5-fold, or at least about 10-fold, compared to the total amount of the mRNA encoding the target protein or functional RNA produced in a control cell. In some cases, the total amount of target protein produced by the cell contacted with the antisense oligomer is increased about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1.1-fold, at least about 1.5-fold, at least about 2-fold, at least about 2.5-fold, at least about 3-fold, at least about 3.5-fold, at least about 4-fold, at least about 5-fold, or at least about 10-fold, compared to the total amount of target protein produced by a control cell.

In some cases of any of the aforementioned methods, the antisense oligomer comprises a backbone modification comprising a phosphorothioate linkage or a phosphorodiamidate linkage. In some cases, the antisense oligomer comprises a phosphorodiamidate morpholino, a locked nucleic acid, a peptide nucleic acid, a 2'-O-methyl, a 2'-Fluoro, or a 2'-O-methoxyethyl moiety. In some cases, the antisense oligomer comprises at least one modified sugar moiety. In some cases, each sugar moiety is a modified sugar moiety. In some cases, the antisense oligomer consists of from 8 to 50 nucleobases, 8 to 40 nucleobases, 8 to 35 nucleobases, 8 to 30 nucleobases, 8 to 25 nucleobases, 8 to 20 nucleobases, 8 to 15 nucleobases, 9 to 50 nucleobases, 9 to 40 nucleobases, 9 to 35 nucleobases, 9 to 30 nucleobases, 9 to 25 nucleobases, 9 to 20 nucleobases, 9 to 15 nucleobases, 10 to 50 nucleobases, 10 to 40 nucleobases, 10 to 35 nucleobases, 10 to 30 nucleobases, 10 to 25 nucleobases, 10 to 20 nucleobases, 10 to 15 nucleobases, 11 to 50 nucleobases, 11 to 40 nucleobases, 11 to 35 nucleobases, 11 to 30 nucleobases, 11 to 25 nucleobases, 11 to 20 nucleobases, 11 to 15 nucleobases, 12 to 50 nucleobases, 12 to 40 nucleobases, 12 to 35 nucleobases, 12 to 30 nucleobases, 12 to 25 nucleobases, 12 to 20 nucleobases, or 12 to 15 nucleobases. In some cases, the antisense oligomer is at least 80%, at least 85%, at least 90%, at least 95%, at least 98%, at least 99%, or 100%, complementary to the targeted portion of the RIC pre-mRNA encoding the protein.

In some cases of any of the aforementioned methods, the cell comprises a population of RIC pre-mRNAs transcribed from the gene encoding the target protein or functional RNA, wherein the population of RIC pre-mRNAs comprises two or more retained introns, and wherein the antisense oligomer binds to the most abundant retained intron in the population of RIC pre-mRNAs. In some cases, the binding of the antisense oligomer to the most abundant retained intron induces splicing out of the two or more retained introns from the population of RIC pre-mRNAs to produce mRNA encoding the target protein or functional RNA. In some cases, the cell comprises a population of RIC pre-mRNAs transcribed from the gene encoding the target protein or functional RNA, wherein the population of RIC pre-mRNAs comprises two or more retained introns, and wherein the antisense oligomer binds to the second most abundant retained intron in the population of RIC pre-mRNAs. In some cases, the binding of the antisense oligomer to the second most abundant retained intron induces splicing out of the two or more retained introns from the population of RIC pre-mRNAs to produce mRNA encoding the target protein or functional RNA. In some cases, the condition is a disease or disorder. In some cases, the disease or disorder is RP18 or RP13. In some cases, the target protein and the RIC pre-mRNA are encoded by the PRPF3 gene or PRPF8 gene. In some cases, the method further comprises assessing protein expression. In some cases, the subject is a human. In some cases, the subject is a non-human animal. In some cases, the subject is a fetus, an embryo, or a child. In some cases, the cells are ex vivo. In some cases, the antisense oligomer is administered by intravitreal injection, subretinal injection, topical application, implantation, intraperitoneal injection, intramuscular injection, subcutaneous injection, or intravenous injection of the subject. In some cases, the 9 nucleotides at -3e to -1e of the exon flanking the 5' splice site and +1 to +6 of the retained intron, are identical to the corresponding wild-type sequence. In some cases, the 16 nucleotides at -15 to -1 of the retained intron and +1e of the exon flanking the 3' splice site are identical to the corresponding wild-type sequence.

Disclosed herein, in some cases, are antisense oligomers as used in the methods described herein. In some cases, the antisense oligomer comprises a sequence with at least about 80%, 85%, 90%, 95%, 97%, or 100% sequence identity to any one of SEQ ID NOs 5-445.

Also disclosed herein, in some cases, are pharmaceutical compositions comprising any of the aforementioned antisense oligomers and an excipient.

Disclosed herein, in some cases, are methods of treating a subject in need thereof, by administering any of the aforementioned pharmaceutical compositions by intravitreal injection, subretinal injection, topical application, implantation, intraperitoneal injection, intramuscular injection, subcutaneous injection, or intravenous injection.

Disclosed herein, in some cases, are compositions comprising an antisense oligomer for use in a method of increasing expression of a target protein or a functional RNA by cells to treat RP18 or RP13 in a subject in need thereof, associated with a deficient protein or deficient functional RNA, wherein the deficient protein or deficient functional RNA is deficient in amount or activity in the subject, wherein the antisense oligomer enhances constitutive splicing of a retained intron-containing pre-mRNA (RIC pre-mRNA) encoding the target protein or the functional RNA, wherein the target protein is: the deficient protein; or a compensating protein which functionally augments or replaces the deficient protein or in the subject; and wherein the function RNA is: the deficient RNA; or a compensating functional RNA which functionally augments or replaces the deficient functional RNA in the subject; wherein the RIC pre-mRNA comprises a retained intron, an exon flanking the 5' splice site and an exon flanking the 3' splice site, and wherein the retained intron is spliced from the RIC pre-mRNA encoding the target protein or the functional RNA, thereby increasing production or activity of the target protein or the functional RNA in the subject.

Disclosed herein, in some cases, are compositions comprising an antisense oligomer for use in a method of treating a condition associated with PRPF3 or PRPF8 protein in a subject in need thereof, the method comprising the step of increasing expression of PRPF3 or PRPF8 protein by cells of the subject, wherein the cells have a retained-intron-containing pre-mRNA (RIC pre-mRNA) comprising a retained intron, an exon flanking the 5' splice site of the retained intron, an exon flanking the 3' splice site of the retained intron, and wherein the RIC pre-mRNA encodes the PRPF3 or PRPF8 protein, the method comprising contacting the cells with the antisense oligomer, whereby the retained intron is constitutively spliced from the RIC pre-mRNA transcripts encoding PRPF3 or PRPF8 protein, thereby increasing the level of mRNA encoding PRPF3 or PRPF8 protein, and increasing the expression of PRPF3 or PRPF8 protein, in the cells of the subject. In some cases, the target protein PRPF3 is encoded by the sequence at NM_004698 or PRPF8 or encoded by the sequence at NM_006445. In some cases, the condition is a disease or disorder. In some cases, the disease or disorder is RP18 or RP13. In some cases, the target protein and RIC pre-mRNA are encoded by the PRPF3 or PRPF8 gene. In some cases, the antisense oligomer targets a portion of the RIC pre-mRNA that is in the retained intron within the region +6 relative to the 5' splice site of the retained intron to -16 relative to the 3' splice site of the retained intron. In some cases, the targeted portion of the RIC pre-mRNA is in the retained intron within the region +498 relative to the 5' splice site of the retained intron to -496 relative to the 3' splice site of the retained intron. In some cases, the target protein in PRPF3. In some cases, the targeted portion of the RIC pre-mRNA is in the retained intron within the region +498 relative to the 5' splice site of the retained intron to -496 relative to the 3' splice site of the retained intron. In some cases, the targeted portion of the RIC pre-mRNA comprises a sequence that is at least about 80%, 85%, 90%, 95%, 97%, or 100% complimentary to any one of SEQ ID NOs 5-323. In some cases, the targeted portion of the RIC pre-mRNA comprises a sequence with at least 80%, 85%, 90%, 95%, 97%, or 100% sequence identity to a region comprising at least 8 contiguous nucleic acids of SEQ ID NO 447 or SEQ ID NO 446. In some cases, the ASO comprises a sequence with at least about 80%, 85%, 90%, 95%, 97%, or 100% sequence identity to any one of SEQ ID NOs 5-323. In some cases, the RIC pre-mRNA comprises a sequence with at least about 80%, 85%, 90%, 95%, 97%, or 100% sequence identity to SEQ ID NO 3. In some cases, the RIC pre-mRNA is encoded by a genetic sequence with at least about 80%, 85%, 90%, 95%, 97%, or 100% sequence identity to SEQ ID NO 1. In some cases, the target protein is PRPF8. In some cases, the targeted portion of the RIC pre-mRNA is in the retained intron within the region +156 relative to the 5' splice site of the retained intron to -156 relative to the 3' splice site of the retained intron. In some cases, the targeted portion of the RIC pre-mRNA comprises a sequence that is at least about 80%, 85%, 90%, 95%, 97%, or 100% complimentary to any one of SEQ ID NOs 324-445. In some cases, the targeted portion of the RIC pre-mRNA comprises a sequence with at least 80%, 85%, 90%, 95%, 97%, or 100% sequence identity to a region comprising at least 8 contiguous nucleic acids of SEQ ID NO 448. In some cases, the ASO comprises a sequence with at least about 80%, 85%, 90%, 95%, 97%, or 100% sequence identity to any one of SEQ ID NOs 234-445. In some cases, the RIC pre-mRNA comprises a sequence with at least about 80%, 85%, 90%, 95%, 97%, or 100% sequence identity to SEQ ID NO 4. In some cases, the RIC pre-mRNA is encoded by a genetic sequence with at least about 80%, 85%, 90%, 95%, 97%, or 100% sequence identity to SEQ ID NO 2. In some cases, the antisense oligomer targets a portion of the RIC pre-mRNA that is in the retained intron within: (a) the region +6 to +100 relative to the 5' splice site of the retained intron; or (b) the region -16 to -100 relative to the 3' splice site of the retained intron. In some cases, the antisense oligomer targets a portion of the RIC pre-mRNA that is within the region about 100 nucleotides downstream of the 5' splice site of the at least one retained intron, to about 100 nucleotides upstream of the 3' splice site of the at least one retained intron. In some cases, the targeted portion of the RIC pre-mRNA is within: (a) the region +2e to -4e in the exon flanking the 5' splice site of the retained intron; or (b) the region +2e to -4e in the exon flanking the 3' splice site of the retained intron.

In some cases of any of the aforementioned compositions, the antisense oligomer does not increase the amount of target protein or functional RNA by modulating alternative splicing of the pre-mRNA transcribed from a gene encoding the target protein or functional RNA. In some cases, the antisense oligomer does not increase the amount of the functional RNA or functional protein by modulating aberrant splicing resulting from mutation of the gene encoding the target protein or functional RNA. In some cases, the RIC pre-mRNA was produced by partial splicing from a full-length pre-mRNA or a wild-type pre-mRNA. In some cases, the mRNA encoding the target protein or functional RNA is a full-length mature mRNA, or a wild-type mature mRNA. In some cases, the target protein produced is full-length protein, or wild-type protein. In some cases, the retained intron is a rate-limiting intron. In some cases, the retained intron is the most abundant retained intron in the RIC pre-mRNA. In some cases, the retained intron is the second most abundant retained intron in the RIC pre-mRNA.

In some cases of any of the aforementioned compositions, the antisense oligomer comprises a backbone modification comprising a phosphorothioate linkage or a phosphorodiamidate linkage. In some cases, the antisense oligomer is an antisense oligonucleotide. In some cases, the antisense oligomer comprises a phosphorodiamidate morpholino, a locked nucleic acid, a peptide nucleic acid, a 2'-O-methyl, a 2'-Fluoro, or a 2'-O-methoxyethyl moiety. In some cases, the antisense oligomer comprises at least one modified sugar moiety. In some cases, each sugar moiety is a modified sugar moiety. In some cases, the antisense oligomer consists of from 8 to 50 nucleobases, 8 to 40 nucleobases, 8 to 35 nucleobases, 8 to 30 nucleobases, 8 to 25 nucleobases, 8 to 20 nucleobases, 8 to 15 nucleobases, 9 to 50 nucleobases, 9 to 40 nucleobases, 9 to 35 nucleobases, 9 to 30 nucleobases, 9 to 25 nucleobases, 9 to 20 nucleobases, 9 to 15 nucleobases, 10 to 50 nucleobases, 10 to 40 nucleobases, 10 to 35 nucleobases, 10 to 30 nucleobases, 10 to 25 nucleobases, 10 to 20 nucleobases, 10 to 15 nucleobases, 11 to 50 nucleobases, 11 to 40 nucleobases, 11 to 35 nucleobases, 11 to 30 nucleobases, 11 to 25 nucleobases, 11 to 20 nucleobases, 11 to 15 nucleobases, 12 to 50 nucleobases, 12 to 40 nucleobases, 12 to 35 nucleobases, 12 to 30 nucleobases, 12 to 25 nucleobases, 12 to 20 nucleobases, or 12 to 15 nucleobases. In some cases, n the antisense oligomer is at least 80%, at least 85%, at least 90%, at least 95%, at least 98%, at least 99%, or is 100% complementary to the targeted portion of the RIC pre-mRNA encoding the protein.

Disclosed herein, in some cases, are pharmaceutical compositions comprising the antisense oligomer of any of the aforementioned compositions and an excipient.

Disclosed herein, in some cases, are methods of treating a subject in need thereof, by administering any of the aforementioned pharmaceutical compositions by intravitreal injection, subretinal injection, topical application, implantation, intraperitoneal injection, intramuscular injection, subcutaneous injection, or intravenous injection of the subject.

Disclosed herein, in some cases, are pharmaceutical compositions comprising: an antisense oligomer that hybridizes to a target sequence of a deficient PRPF3 mRNA transcript, wherein the deficient PRPF3 mRNA transcript comprises a retained intron, wherein the antisense oligomer induces splicing out of the retained intron from the deficient PRPF3 mRNA transcript; and a pharmaceutical acceptable excipient. In some cases, the deficient PRPF3 mRNA transcript is a PRPF3 RIC pre-mRNA transcript. In some cases, the targeted portion of the PRPF3 RIC pre-mRNA transcript is in the retained intron within the region +500 relative to the 5' splice site of the retained intron to -500 relative to the 3' spliced site of the retained intron. In some cases, the PRPF3 RIC pre-mRNA transcript is encoded by a genetic sequence with at least about 80%, 85%, 90%, 95%, 96%, 97%, 98%, 99% or 100% sequence identity to SEQ ID NO: 1. In some cases, the PRPF3 RIC pre-mRNA transcript comprises a sequence with at least about 80%, 85%, 90%, 95%, 96%, 97%, 98%, 99% or 100% sequence identity to any one of SEQ ID NOs: 3-4. In some cases, the antisense oligomer comprises a backbone modification comprising a phosphorothioate linkage or a phosphorodiamidate linkage. In some cases, the antisense oligomer is an antisense oligonucleotide. In some cases, the antisense oligomer comprises a phosphorodiamidate morpholino, a locked nucleic acid, a peptide nucleic acid, a 2'-O-methyl, a 2'-Fluoro, or a 2'-O-methoxyethyl moiety. In some cases, the antisense oligomer comprises at least one modified sugar moiety. In some cases, the antisense oligomer comprises from 8 to 50 nucleobases, 8 to 40 nucleobases, 8 to 35 nucleobases, 8 to 30 nucleobases, 8 to 25 nucleobases, 8 to 20 nucleobases, 8 to 15 nucleobases, 9 to 50 nucleobases, 9 to 40 nucleobases, 9 to 35 nucleobases, 9 to 30 nucleobases, 9 to 25 nucleobases, 9 to 20 nucleobases, 9 to 15 nucleobases, 10 to 50 nucleobases, 10 to 40 nucleobases, 10 to 35 nucleobases, 10 to 30 nucleobases, 10 to 25 nucleobases, 10 to 20 nucleobases, 10 to 15 nucleobases, 11 to 50 nucleobases, 11 to 40 nucleobases, 11 to 35 nucleobases, 11 to 30 nucleobases, 11 to 25 nucleobases, 11 to 20 nucleobases, 11 to 15 nucleobases, 12 to 50 nucleobases, 12 to 40 nucleobases, 12 to 35 nucleobases, 12 to 30 nucleobases, 12 to 25 nucleobases, 12 to 20 nucleobases, or 12 to 15 nucleobases. In some cases, the antisense oligomer is at least 80%, at least 85%, at least 90%, at least 95%, at least 98%, at least 99%, or is 100% complementary to a targeted portion of the PRPF3 RIC pre-mRNA transcript. In some cases, the targeted portion of the PRPF3 RIC pre-mRNA transcript is within a sequence selected from SEQ ID NOs: 446-447. In some cases, the antisense oligomer comprises a nucleotide sequence that is at least about 80%, 85%, 90%, 91%, 92%, 93%, 94%, 95%, 96%, 97%, 98%, or 99% sequence identity to any one of SEQ ID NOs: 5-323. In some cases, the antisense oligomer comprises a nucleotide sequence selected from SEQ ID NOs: 5-323. In some cases, the pharmaceutical composition is formulated for intrathecal injection, intracerebroventricular injection, intraperitoneal injection, intramuscular injection, subcutaneous injection, or intravenous injection.

Disclosed herein, in some cases, are pharmaceutical compositions comprising: an antisense oligomer that hybridizes to a target sequence of a deficient PRPF8 mRNA transcript, wherein the deficient PRPF8 mRNA transcript comprises a retained intron, wherein the antisense oligomer induces splicing out of the retained intron from the deficient PRPF8 mRNA transcript; and a pharmaceutical acceptable excipient. In some cases, the deficient PRPF8 mRNA transcript is a PRPF8 RIC pre-mRNA transcript. In some cases, the targeted portion of the PRPF8 RIC pre-mRNA transcript is in the retained intron within the region +500 relative to the 5' splice site of the retained intron to -500 relative to the 3' spliced site of the retained intron. In some cases, the PRPF8 RIC pre-mRNA transcript is encoded by a genetic sequence with at least about 80%, 85%, 90%, 95%, 96%, 97%, 98%, 99% or 100% sequence identity to SEQ ID NO: 2. In some cases, the PRPF8 RIC pre-mRNA transcript comprises a sequence with at least about 80%, 85%, 90%, 95%, 96%, 97%, 98%, 99% or 100% sequence identity to any one of SEQ ID NOs: 3-4. In some cases, the antisense oligomer comprises a backbone modification comprising a phosphorothioate linkage or a phosphorodiamidate linkage. In some cases, the antisense oligomer is an antisense oligonucleotide. In some cases, the antisense oligomer comprises a phosphorodiamidate morpholino, a locked nucleic acid, a peptide nucleic acid, a 2'-O-methyl, a 2'-Fluoro, or a 2'-O-methoxyethyl moiety. In some cases, the antisense oligomer comprises at least one modified sugar moiety. In some cases, the antisense oligomer comprises from 8 to 50 nucleobases, 8 to 40 nucleobases, 8 to 35 nucleobases, 8 to 30 nucleobases, 8 to 25 nucleobases, 8 to 20 nucleobases, 8 to 15 nucleobases, 9 to 50 nucleobases, 9 to 40 nucleobases, 9 to 35 nucleobases, 9 to 30 nucleobases, 9 to 25 nucleobases, 9 to 20 nucleobases, 9 to 15 nucleobases, 10 to 50 nucleobases, 10 to 40 nucleobases, 10 to 35 nucleobases, 10 to 30 nucleobases, 10 to 25 nucleobases, 10 to 20 nucleobases, 10 to 15 nucleobases, 11 to 50 nucleobases, 11 to 40 nucleobases, 11 to 35 nucleobases, 11 to 30 nucleobases, 11 to 25 nucleobases, 11 to 20 nucleobases, 11 to 15 nucleobases, 12 to 50 nucleobases, 12 to 40 nucleobases, 12 to 35 nucleobases, 12 to 30 nucleobases, 12 to 25 nucleobases, 12 to 20 nucleobases, or 12 to 15 nucleobases. In some cases, the antisense oligomer is at least 80%, at least 85%, at least 90%, at least 95%, at least 98%, at least 99%, or is 100% complementary to a targeted portion of the PRPF8 RIC pre-mRNA transcript. In some cases, the targeted portion of the PRPF8 RIC pre-mRNA transcript is within a sequence selected from SEQ ID NOs: 448. In some cases, the antisense oligomer comprises a nucleotide sequence that is at least about 80%, 85%, 90%, 91%, 92%, 93%, 94%, 95%, 96%, 97%, 98%, or 99% sequence identity to any one of SEQ ID NOs: 324-445. In some cases, the antisense oligomer comprises a nucleotide sequence selected from SEQ ID NOs: 324-445. In some cases, the pharmaceutical composition is formulated for intravitreal injection, subretinal injection, topical application, implantation, intraperitoneal injection, intramuscular injection, subcutaneous injection, or intravenous injection.

Disclosed herein, in some cases, are methods of inducing processing of a deficient PRPF3 mRNA transcript to facilitate removal of a retained intron to produce a fully processed PRPF3 mRNA transcript that encodes a functional form of a PRPF3 protein, the method comprising: (a) contacting an antisense oligomer to a target cell of a subject; (b) hybridizing the antisense oligomer to the deficient PRPF3 mRNA transcript, wherein the deficient PRPF3 mRNA transcript is capable of encoding the functional form of a PRPF3 protein and comprises at least one retained intron; (c) removing the at least one retained intron from the deficient PRPF3 mRNA transcript to produce the fully processed PRPF3 mRNA transcript that encodes the functional form of PRPF3 protein; and (d) translating the functional form of PRPF3 protein from the fully processed PRPF3 mRNA transcript. In some cases, the retained intron is an entire retained intron. In some cases, the deficient PRPF3 mRNA transcript is a PRPF3 RIC pre-mRNA transcript.

Disclosed herein, in some cases, are methods of treating a subject having a condition caused by a deficient amount or activity of PRPF3 protein comprising administering to the subject an antisense oligomer comprising a nucleotide sequence with at least about 80%, 85%, 90%, 91%, 92%, 93%, 94%, 95%, 96%, 97%, 98%, or 99% sequence identity to any one of SEQ ID NOs: 5-323.

Disclosed herein, in some cases, are methods of inducing processing of a deficient PRPF8 mRNA transcript to facilitate removal of a retained intron to produce a fully processed PRPF8 mRNA transcript that encodes a functional form of a PRPF8 protein, the method comprising: (a) contacting an antisense oligomer to a target cell of a subject; (b) hybridizing the antisense oligomer to the deficient PRPF8 mRNA transcript, wherein the deficient PRPF8 mRNA transcript is capable of encoding the functional form of a PRPF8 protein and comprises at least one retained intron; (c) removing the at least one retained intron from the deficient PRPF8 mRNA transcript to produce the fully processed PRPF8 mRNA transcript that encodes the functional form of PRPF8 protein; and (d) translating the functional form of PRPF8 protein from the fully processed PRPF8 mRNA transcript. In some cases, the retained intron is an entire retained intron. In some cases, the deficient PRPF8 mRNA transcript is a PRPF8 RIC pre-mRNA transcript.

Disclosed herein, in some cases, are methods of treating a subject having a condition caused by a deficient amount or activity of PRPF8 protein comprising administering to the subject an antisense oligomer comprising a nucleotide sequence with at least about 80%, 85%, 90%, 91%, 92%, 93%, 94%, 95%, 96%, 97%, 98%, or 99% sequence identity to any one of SEQ ID NOs: 324-445.

### BRIEF DESCRIPTION OF THE DRAWINGS

The novel features of the invention are set forth with particularity in the appended claims. A better understanding of the features of the present disclosure will be obtained by reference to the following illustrative drawings.

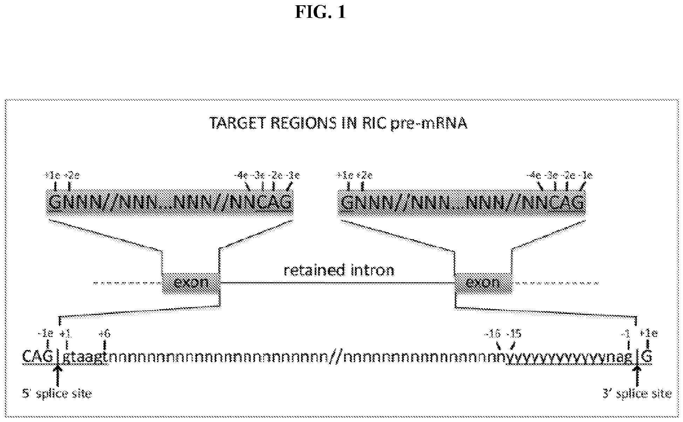

**FIG. 1** depicts a schematic representation of an exemplary retained-intron-containing (RIC) pre-mRNA transcript. The 5' splice site consensus sequence is indicated with underlined letters (letters are nucleotides; upper case: exonic portion and lower case: intronic portion) from - 3e to -1e and +1 to +6 (numbers labeled "e" are exonic and unlabeled numbers are intronic). The 3' splice site consensus sequence is indicated with underlined letters (letters are nucleotides; upper case: exonic portion and lower case: intronic portion) from -15 to -1 and +1e (numbers labeled "e" are exonic and unlabeled numbers are intronic). Intronic target regions for ASO screening comprise nucleotides +6 relative to the 5' splice site of the retained intron (arrow at left) to -16 relative to the 3' splice site of the retained intron (arrow at right). In cases, intronic target regions for ASO screening comprise nucleotides +6 to +100 relative to the 5' splice site of the retained intron and -16 to -100 relative to the 3' splice site of the retained intron. Exonic target regions comprise nucleotides +2e to -4e in the exon flanking the 5' splice site of the retained intron and +2e to -4e in the exon flanking the 3' splice site of the retained intron. "n" or "N" denote any nucleotide, "y" denotes pyrimidine. The sequences shown represent consensus sequences for mammalian splice sites and individual introns and exons need not match the consensus sequences at every position.

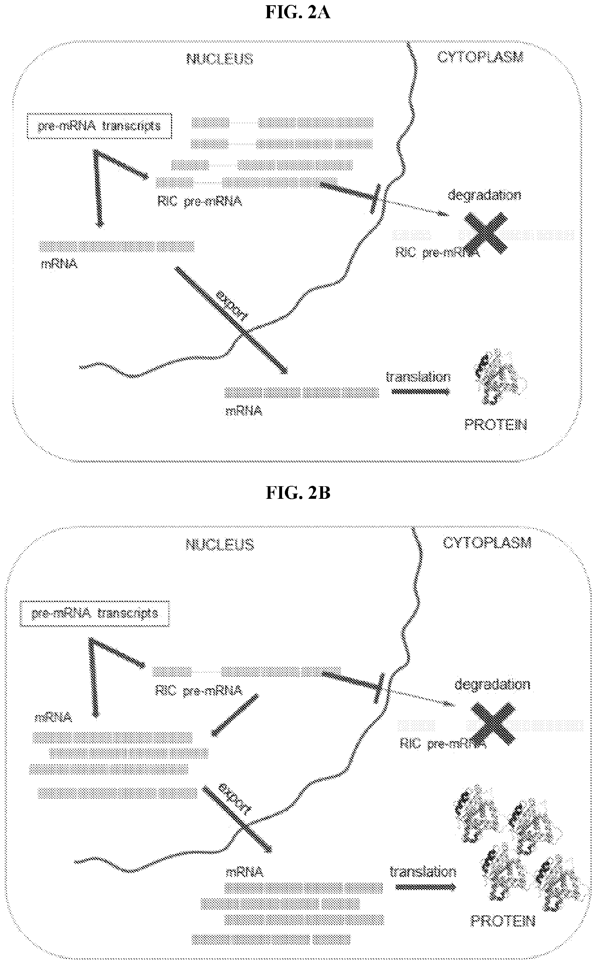

**FIG. 2A** depicts an exemplary representation of the Targeted Augmentation of Nuclear Gene Output (TANGO) approach. FIG. 2A shows a cell divided into nuclear and cytoplasmic compartments. In the nucleus, a pre-mRNA transcript of a target gene consisting of exons (rectangles) and introns (connecting lines) undergoes splicing to generate an mRNA, and this mRNA is exported to the cytoplasm and translated into target protein. For this target gene, the splicing of intron 1 is inefficient and a retained intron-containing (RIC) pre-mRNA accumulates primarily in the nucleus, and if exported to the cytoplasm, is degraded, leading to no target protein production.
**FIG. 2B** depicts an exemplary schematic representation of the Targeted Augmentation of Nuclear Gene Output (TANGO) approach. FIG. 2B shows an example of the same cell as in FIG. 2A divided into nuclear and cytoplasmic compartments. Treatment with an antisense oligomer (ASO) promotes the splicing of intron 1 and results in an increase in mRNA, which is in turn translated into higher levels of target protein

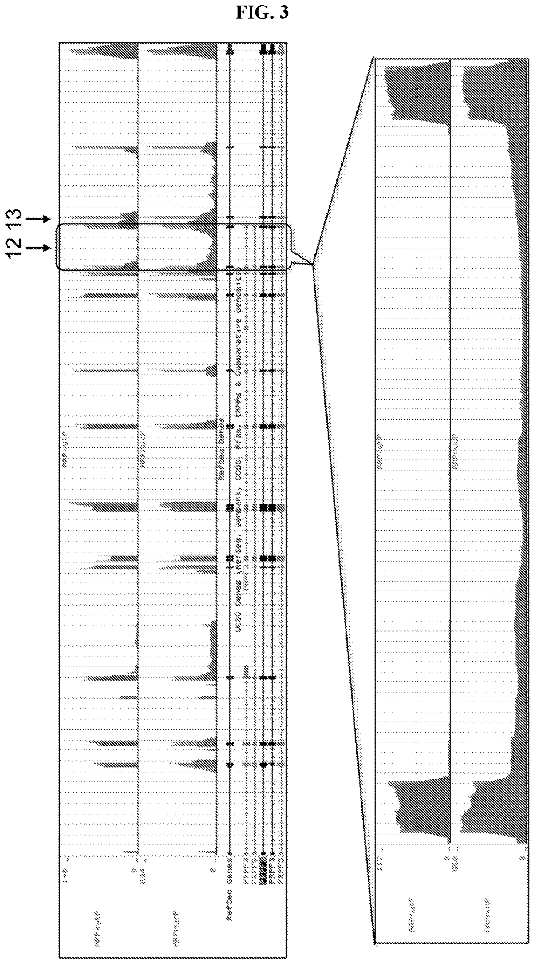

**FIG. 3** depicts intron-retention in the *PRPF3* gene with intron 12 detail. The identification of intron-retention events in the *PRPF3* gene using RNA sequencing (RNAseq) is shown, visualized in the UCSC genome browser. The upper panel shows the read density corresponding to the PRPF3 transcript expressed in ARPE-19 (retina epithelial) cells and localized in either the cytoplasmic (top) or nuclear fraction (bottom). At the bottom of this panel, a graphic representation of the *PRPF3* gene is shown to scale. The read density is shown as peaks. The highest read density corresponds to exons (black boxes), while no reads are observed for the majority of the introns (lines with arrow heads) in either cellular fraction. Higher read density is detected for intron 12 (indicated by the arrow) in the nuclear fraction compared to the cytoplasmic fraction indicating that splicing efficiency of intron 12 is low, resulting in intron retention. The retained-intron containing pre-mRNA transcripts are retained in the nucleus and are not exported out to the cytoplasm. The read density for intron 12 in renal epithelial cells is shown in detail in the lower panel.

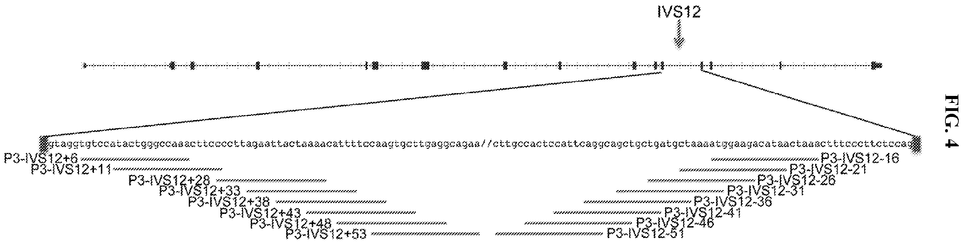

**FIG. 4** illustrates a graphic representation of the ASO walk performed for PRPF3 IVS 12 targeting sequences immediately downstream of the 5' splice site or upstream of the 3' splice site using 2'-O-Me ASOs, PS backbone, is shown. ASOs were designed to cover these regions by shifting 5 nucleotides at a time (with the exception of ASO P3-IVS12+28). The *PRPF3* exon-intron structure is drawn to scale.

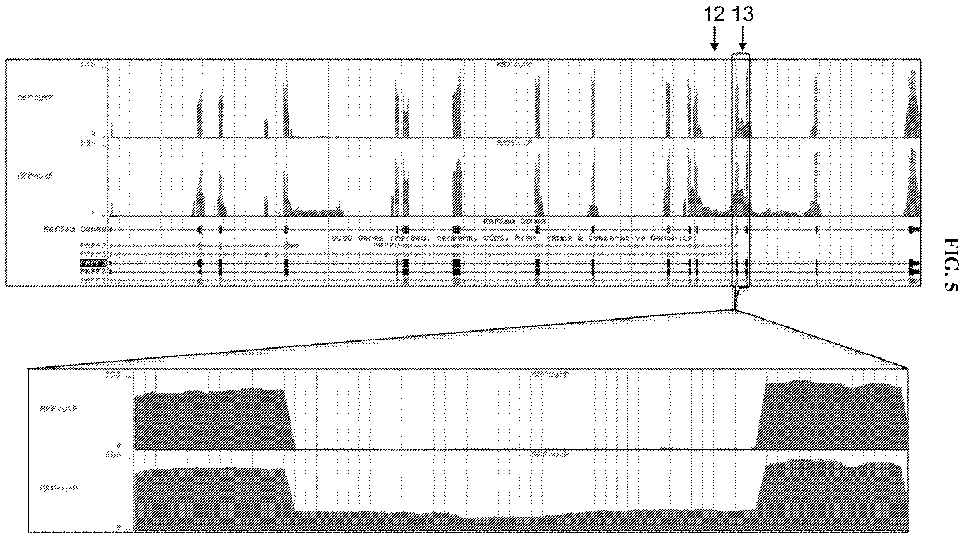

**FIG. 5** depicts intron-retention in the *PRPF3* gene with intron 13. Intron retention in the *PRPF3* gene was identified by RNA sequencing (RNAseq), visualized in the UCSC genome browser, as described herein in the Examples. The read density for intron 13 in ARPE-19 cells is shown in detail in the lower panel.

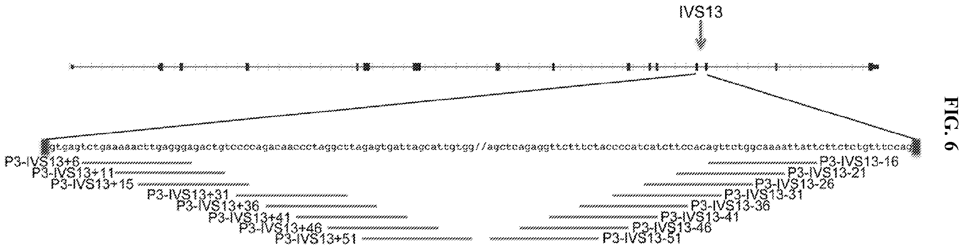

**FIG. 6** illustrates a graphic representation of the ASO walk performed for PRPF3 IVS 13 targeting sequences immediately downstream of the 5' splice site or upstream of the 3' splice site using 2'-O-Me ASOs, PS backbone, is shown. ASOs were designed to cover these regions by shifting 5 nucleotides at a time (with the exception of ASO P3-IVS13+15, P3-IVS13+31, and P3-IVS13-47). The *PRPF3* exon-intron structure is drawn to scale.

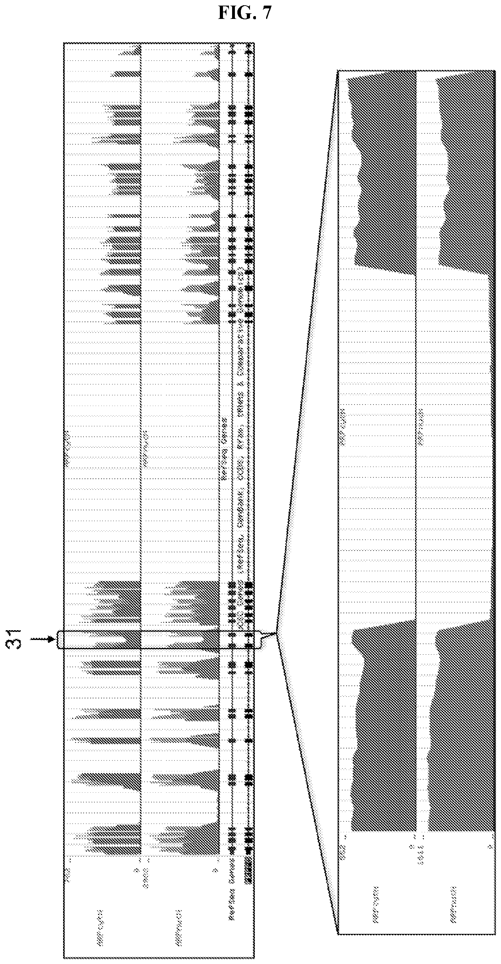

**FIG. 7** depicts intron-retention in the *PRPF8* gene with intron 31 detail. The identification of intron-retention events in the *PRPF8* gene using RNA sequencing (RNAseq) is shown, visualized in the UCSC genome browser. The upper panel shows the read density corresponding to the PRPF8 transcript expressed in ARPE-19 (retina epithelial) cells and localized in either the cytoplasmic (top) or nuclear fraction (bottom). At the bottom of this panel, a graphic representation of the *PRPF8* gene is shown to scale. The read density is shown as peaks. The highest read density corresponds to exons (black boxes), while no reads are observed for the majority of the introns (lines with arrow heads) in either cellular fraction. Higher read density is detected for intron 31 (indicated by the arrow) in the nuclear fraction compared to the cytoplasmic fraction indicating that splicing efficiency of intron 31 is low, resulting in intron retention. The retained-intron containing pre-mRNA transcripts are retained in the nucleus and are not exported out to the cytoplasm. The read density for intron 31 in renal epithelial cells is shown in detail in the lower panel.

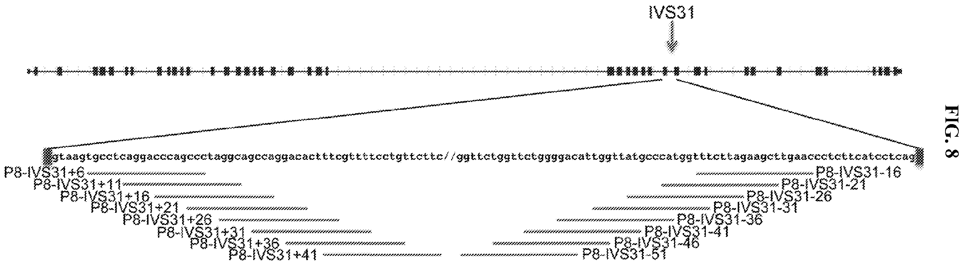

**FIG. 8** illustrates a graphic representation of the ASO walk performed for PRPF8 IVS 31 targeting sequences immediately downstream of the 5' splice site or upstream of the 3' splice site using 2'-O-Me ASOs, PS backbone, is shown. ASOs were designed to cover these regions by shifting 5 nucleotides at a time. The *PRPF8* exon-intron structure is drawn to scale.

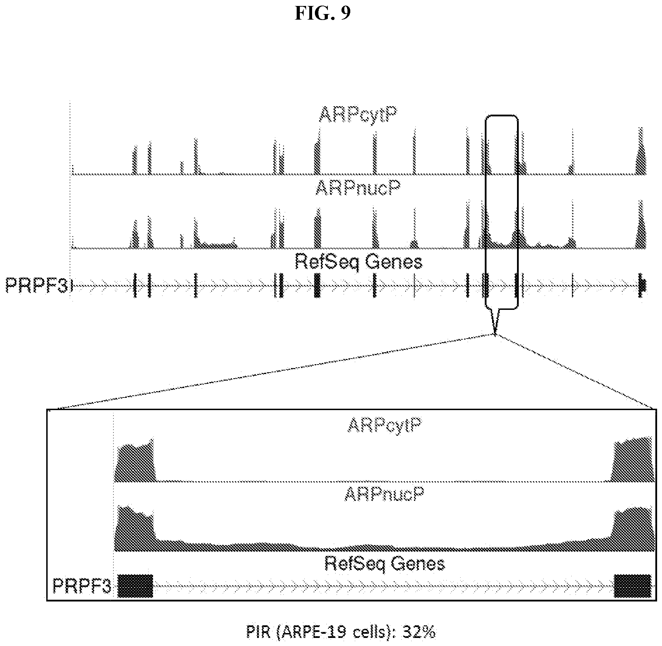

**FIG. 9** depicts a schematic of the RefSeq Genes for PRPF3 corresponding to NM_004698. The Percent Intron Retention (PIR) of the circled intron is shown.

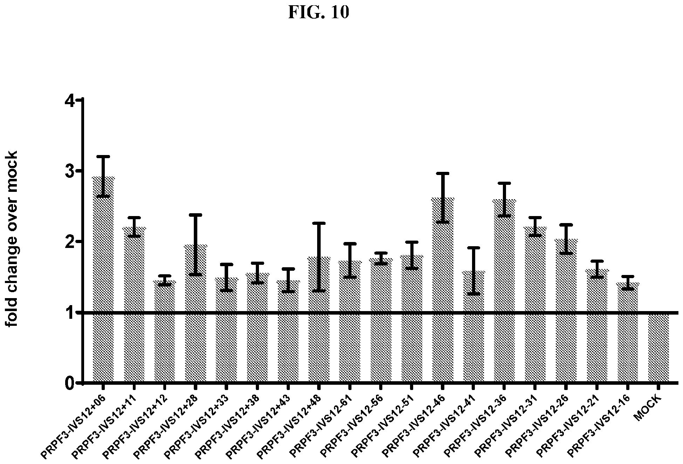

**FIG. 10** depicts an exemplary graph is depicted showing the average (n=3) fold change in expression levels of PRPF3 mRNA without intron 12 in ARPE-19 cells treated for 24 hrs with 80 nM of the indicated ASOs over mock treated cells. Data is normalized to RPL32 expression.

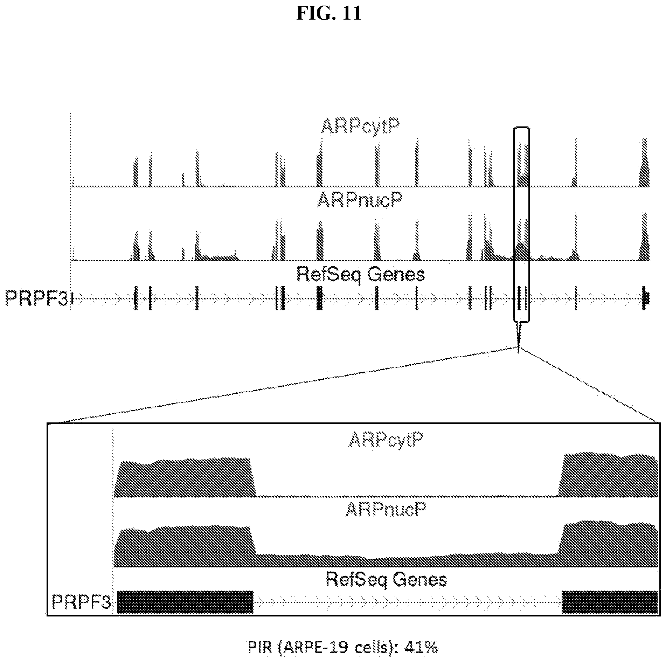

**FIG. 11** depicts a schematic of the RefSeq Genes for PRPF3 corresponding to NM_004698. The Percent Intron Retention (PIR) of the circled intron is shown.

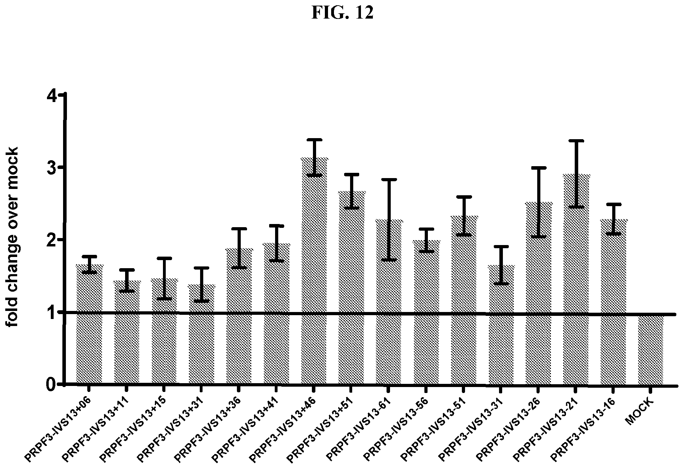

**FIG. 12** depicts an exemplary graph showing the average (n=3) fold change in expression levels of PRPF3 mRNA without intron 13 in ARPE-19 cells treated for 24 hrs with 80 nM of the indicated ASOs over mock treated cells. Data is normalized to RPL32 expression.

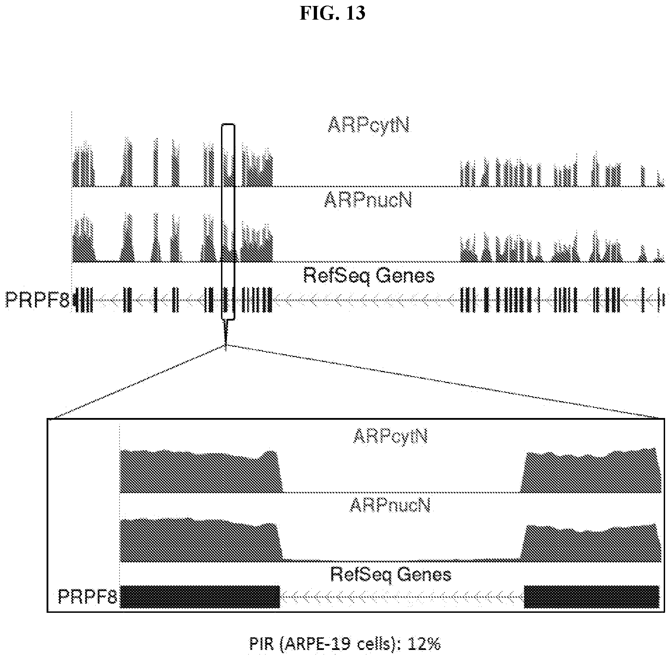

**FIG. 13** depicts a schematic of the RefSeq Genes for PRPF8 corresponding to NM_006445. The Percent Intron Retention (PIR) of the circled intron is shown.

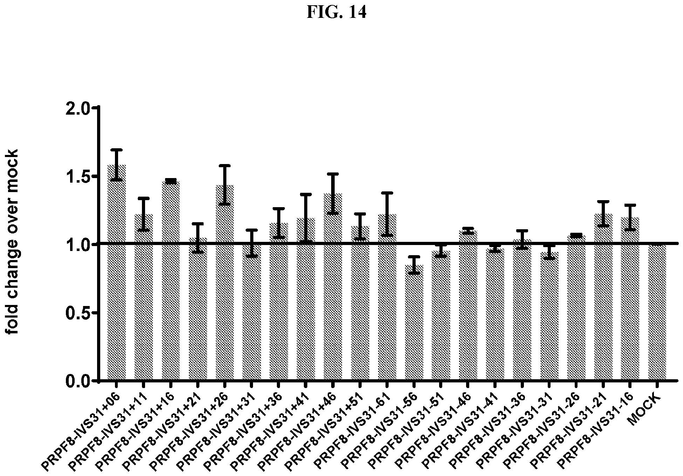

**FIG. 14** depicts an exemplary graph is depicted showing the average (n=3) fold change in expression levels of PRPF8 mRNA without intron 31 in ARPE-19 cells treated for 24 hrs with 80 nM of the indicated ASOs over mock treated cells. Data is normalized to RPL32 expression.

### DETAILED DESCRIPTION

Individual introns in primary transcripts of protein-coding genes having more than one intron are spliced from the primary transcript with different efficiencies. In most cases only the fully spliced mRNA is exported through nuclear pores for subsequent translation in the cytoplasm. Unspliced and partially spliced transcripts are detectable in the nucleus. It is generally thought that nuclear accumulation of transcripts that are not fully spliced is a mechanism to prevent the accumulation of potentially deleterious mRNAs in the cytoplasm that may be translated to protein. For some genes, splicing of the least efficient intron is a rate-limiting post-transcriptional step in gene expression, prior to translation in the cytoplasm.

Substantial levels of partially-spliced transcripts of the *PRPF3* and *PRPF8* genes, which encode the PRPF3 protein, deficient in RP18, and the PRPF8 protein, deficient in RP13, respectively, have been discovered in the nucleus of human cells. These *PRPF3* and *PRPF8* pre-mRNA species comprise at least one retained intron. Described herein are compositions and methods for upregulating splicing of one or more retained *PRPF3* or *PRPF8* introns that are rate-limiting for the nuclear stages of gene expression to increase steady-state production of fully-spliced, mature mRNA, and thus, translated PRPF3 or PRPF8 protein levels, respectively. These compositions and methods utilize antisense oligomers (ASOs) that promote constitutive splicing at an intron splice site of a retained-intron-containing *PRPF3* or *PRPF8* pre-mRNA that accumulates in the nucleus. Thus, PRPF3 or PRPF8 protein is increased using the methods of the disclosure to treat a condition caused by PRPF3 or PRPF8 protein deficiency. In other cases, the methods of the disclosure are used to increase PRPF3 or PRPF8 production to treat a condition in a subject in need thereof. In cases, the subject has condition in which PRPF3 or PRPF8 is not necessarily deficient relative to wild-type, but where an increase in PRPF3 or PRPF8 mitigates the condition nonetheless. In some cases, the condition is a caused by a PRPF3 or PRPF8 haploinsufficiency.

### Retinitis Pigmentosa

Retinitis pigmentosa (RP) describes a debilitating group of eye disorders , including RP18, caused by a deficiency in the PRPF3 protein, and RP13, caused by a deficiency in the PRPF8 protein. Subjects having RP experience loss of night vision in the early phase of the disorder. Over time, loss of vision begins to occur, which eventually leads to tunnel vision. This loss in vision can be attributed to a dysfunction in the rod photoreceptor system. As the disorder progresses, the afflicted patient may lose a significant portion of their cone photoreceptors before experiencing loss of visual acuity (Berger et al., 2010).

The overall prevalence of RP diseases is estimated to be about 1:3500. The mutation of over 40 genes has been correlated to incidence of RP disease through three patterns of inheritance: autosomal dominant, autosomal recessive, and X-linked. The genes that have been implicated in the progression of RP diseases can be grouped into five main categories: i) phototransduction, ii) retinal metabolism, iii) tissue development and maintenance, iv) cellular structure, and v) splicing. The genes that comprise the splicing category (PRPF3, PRPF8, PRP31 and PAP1) are responsible for the assembly of the spliceosome protein complex that is responsible for removing intronic sequences from pre-mRNA. Studies have suggested that the progression of at least some cases of RP is due to haploinsuffiency, meaning that the presence of a single dysfunctional allele results in diminished expression of the corresponding protein (Berger et al., 2010).

### PRPF3

The *PRPF3* gene, which spans 16 exons and is located at 1q21.1, encodes the pre-mRNA processing factor 3 (PRPF3) protein. The *PRPF3* canonical mRNA sequence is set forth at NCBI Reference Sequence: NM_004698. PRPF3 is a 77 kD, 682 amino acid protein that is involved in spliceosome assembly. Dysfunction of PRPF3 has been implicated in the progression of RP 18. It has been speculated that a retina-specific splicing element may interact with PRPF3 and generate the rod photoreceptor-specific phenotype.
Missense mutations P493S and T494M in PRPF3 have both been shown to display the RP18 phenotype, linking dysfunction of PRPF3 with the RP 18 phenotype (Berger et al., 2010). RP18 and the *PRPF3* gene are described by, e.g., OMIM #601414 (Online Mendelian Inheritance in Man, Johns Hopkins University, 1966-2015).

### PRPF8

The PRPF8 gene, which spans 42 exons and is located at 17p13.3, codes for the pre-mRNA processing factor 8 (PRPF8) protein. The *PRPF8* canonical mRNA sequence is set forth at NCBI Reference Sequence: NM_006445. PRPF8 is a 220 kD, 2,334 amino acid protein that is involved in spliceosome assembly. Dysfunction of PRPF8 has been implicated in the progression of RP13. PRPF8 mutants H2309P, H2309R, R2310K, P2301T, F2304L, R2310G and F2314L were found to result in the clinical phenotype of RP13, linking dysfunction of PRPF8 with RP13 (Berger et al., 2010). RP13 and the *RP8* gene are described by, e.g., OMIM #600059 (Online Mendelian Inheritance in Man, Johns Hopkins University, 1966-2015).

### Retained Intron Containing Pre-mRNA (RIC Pre-mRNA)

The methods of the present disclosure exploit the presence of retained-intron-containing pre-mRNA (RIC pre-mRNA) transcribed from the *PRPF3* or the *PRPF8* gene in the cell nucleus. Splicing of the identified RIC pre-mRNA species to produce mature, fully-spliced, mRNA, is induced using ASOs that stimulate splicing out of the retained introns. The resulting mature mRNA can be exported to the cytoplasm and translated, thereby increasing the amount of PRPF3 protein or PRPF8 protein in the patient's cells and alleviating symptoms of RP18 or RP13, respectively. This method, described further below, is known as Targeted Augmentation of Nuclear Gene Output (TANGO).

### PRPF3 Nuclear Transcripts

As described herein in the Examples, the *PRPF3* gene was analyzed for intron-retention events and retention of introns, *e.g*., introns 12 and 13, was observed. RNA sequencing (RNAseq), visualized in the UCSC genome browser, showed *PRPF3* transcripts expressed in ARPE-19 cells and localized in either the cytoplasmic or nuclear fraction. In both fractions, reads were not observed for the majority of the introns. However, higher read density was detected for introns 12 and 13 in the nuclear fraction compared to the cytoplasmic fraction indicating that splicing efficiency of introns 12 and 13 is low, resulting in intron retention. The retained-intron containing pre-mRNA transcripts accumulate primarily in the nucleus and are not translated into the PRPF3 protein. The read density for introns 12 and 13, indicated 26%, and 33% intron retention, respectively. The percent intron retention (PIR) value for intron 12 was obtained by averaging four values (37, 35, 16, and 15). The PIR value for intron 13 was obtained similarly, by averaging the four values 34, 33, 34, and 31. Each value was determined in ARPE-19 (retinal pigmented epithelial) cells using one of four different algorithms.

In some cases, the PRPF3 intron number corresponds to the mRNA sequence at NM_004698. In some cases, the targeted portion of the *PRPF3* RIC pre-mRNA is in intron 12 and/or 13. In cases, hybridization of an ASO to the targeted portion of a *PRPF3* RIC pre-mRNA results in enhanced splicing at the splice site (5' splice site or 3' splice site) of at least one of retained *PRPF3* introns 12 or 13 and subsequently increases PRPF3 protein production. It is understood that the intron numbering may change in reference to a different PRPF3 isoform sequence. One of skill in the art can determine the corresponding intron number in any PRPF3 isoform based on an intron sequence provided herein or using the intron number provided in reference to the mRNA sequence at NM_004698. One of skill in the art also can determine the sequences of flanking exons in any PRPF3 isoform for targeting using the methods of the disclosure, based on an intron sequence provided herein or using the intron number provided in reference to the mRNA sequence at NM_004698. In some cases, the compositions and methods of the present disclosure are used to increase the expression of any known PRPF3 isoform, e.g., as described in the NCBI Gene ID database at Gene ID 9129 (NCBI repository of biological and scientific information, operated by National Center for Biotechnology Information, National Library of Medicine, 8600 Rockville Pike, Bethesda, MD USA 20894).

In some cases, the PRPF8 intron numbering corresponds to the mRNA sequence at NM_006445. In some cases, the targeted portion of the *PRPF8* RIC pre-mRNA is in intron 31. Hybridization of an ASO to the targeted portion of the RIC pre-mRNA results in enhanced splicing at the splice site (5' splice site or 3' splice site) of at least one of retained *PRPF8* intron 31 and subsequently increases PRPF8 protein production. It is understood that the intron numbering may change in reference to a different PRPF8 isoform sequence. One of skill in the art can determine the corresponding intron number in any PRPF8 isoform based on an intron sequence provided herein or using the intron number provided in reference to the mRNA sequence at NM_006445. One of skill in the art also can determine the sequences of flanking exons in any PRPF8 isoform for targeting using the methods of the disclosure, based on an intron sequence provided herein or using the intron number provided in reference to the mRNA sequence at NM_006445. The compositions and methods of the present invention are used to increase the expression of any known PRPF8 isoform, e.g., as described in the NCBI Gene ID database at Gene ID 10594 (NCBI repository of biological and scientific information).

### PRPF3

In some cases, the ASOs disclosed herein target a RIC pre-mRNA transcribed from a PRPF3 genomic sequence (a PRPF3 RIC pre-mRNA). In some cases, the PRPF3 genomic sequence is SEQ ID NO: 1. In some cases, the PRPF3 RIC pre-mRNA is SEQ ID NO: 3.

### PRPF3: retained intron 12

In some cases, the PRPF3 RIC pre-mRNA transcript comprises retained intron 12. In some cases, when the PRPF3 RIC pre-mRNA transcript comprises retained intron 12, the ASOs disclosed herein target SEQ ID NO: 447. In some cases, when the PRPF3 RIC pre-mRNA transcript comprises retained intron 12, the ASO has a sequence according to any one of SEQ ID NOs: 5-239. In some cases, the ASOs target a PRPF3 RIC pre-mRNA sequence.

In some cases, the ASO targets exon 12 of PRPF3 RIC pre-mRNA comprising a retained intron 12. In some cases, the ASO targets an exon 12 sequence upstream (or 5') from the 5' splice site of a PRPF3 RIC pre-mRNA comprising a retained intron 12. In some cases, the ASO targets an exon sequence about 4 to about 94 nucleotides upstream (or 5') from the 5' splice site of a PRPF3 RIC pre-mRNA comprising a retained intron 12. In some cases, the ASO has a sequence according to any one of SEQ ID NOs: 5-23.

In some cases, the ASO targets intron 12 in a *PRPF3* RIC pre-mRNA comprising a retained intron 12. In some cases, the ASO targets an intron 12 sequence downstream (or 3') from the 5' splice site of a PRPF3 RIC pre-mRNA comprising a retained intron 12. In some cases, the ASO targets an intron 12 sequence about 6 to about 498 nucleotides downstream (or 3') from the 5' splice site of a PRPF3 RIC pre-mRNA comprising a retained intron 12. In some cases, the ASO has a sequence according to any one of SEQ ID NOs: 24-121.

In some cases, the ASO targets an intron 12 sequence upstream (or 5') from the 3' splice site of a PRPF3 RIC pre-mRNA comprising a retained intron 12. In some cases, the ASO targets an intron 12 sequence about 16 to about 496 nucleotides upstream (or 5') from the 3' splice site of a PRPF3 RIC pre-mRNA a comprising retained intron 12. In some cases, the ASO has a sequence according to any one of SEQ ID NOs: 122-218.

In some cases, the ASO targets exon 13 in a PRPF3 RIC pre-mRNA comprising a retained intron 12. In some cases, the ASO targets an exon 13 sequence downstream (or 3') from the 3' splice site of a PRPF3 RIC pre-mRNA comprising a retained intron 12. In some cases, the ASO targets an exon 13 sequence about 2 to about 102 nucleotides downstream (or 3') from the 3' splice site of a PRPF3 RIC pre-mRNA comprising a retained intron 12. In some cases, the ASO has a sequence according to any one of SEQ ID NOs: 219-239.

### PRPF3: retained intron 13

In some cases, the PRPF3 RIC pre-mRNA transcript comprises retained intron 13. In some cases, when the PRPF3 RIC pre-mRNA transcript comprises retained intron 13, the ASOs disclosed herein target SEQ ID NO: 446. In some cases, when the PRPF3 RIC pre-mRNA transcript comprises retained intron 13, the ASO has a sequence according to any one of SEQ ID NOs: 240-323. In some cases, the ASOs target a PRPF3 RIC pre-mRNA sequence.

In some cases, the ASO targets exon 13 of PRPF3 RIC pre-mRNA comprising a retained intron 13. In some cases, the ASO targets an exon 13 sequence upstream (or 5') from the 5' splice site of a PRPF3 RIC pre-mRNA comprising a retained intron 13. In some cases, the ASO targets an exon sequence about 4 to about 99 nucleotides upstream (or 5') from the 5' splice site of a PRPF3 RIC pre-mRNA comprising a retained intron 13. In some cases, the ASO has a sequence according to any one of SEQ ID NOs: 240-259.

In some cases, the ASO targets intron 13 in a *PRPF3* RIC pre-mRNA comprising a retained intron 13. In some cases, the ASO targets an intron 13 sequence downstream (or 3') from the 5' splice site of a PRPF3 RIC pre-mRNA comprising a retained intron 13. In some cases, the ASO targets an intron 13 sequence about 6 to about 146 nucleotides downstream (or 3') from the 5' splice site of a PRPF3 RIC pre-mRNA comprising a retained intron 13. In some cases, the ASO has a sequence according to any one of SEQ ID NOs: 260-286.

In some cases, the ASO targets an intron 13 sequence upstream (or 5') from the 3' splice site of a PRPF3 RIC pre-mRNA comprising a retained intron 13. In some cases, the ASO targets an intron 13 sequence about 16 to about 146 nucleotides upstream (or 5') from the 3' splice site of a PRPF3 RIC pre-mRNA a comprising retained intron 13. In some cases, the ASO has a sequence according to any one of SEQ ID NOs: 287-310.

In some cases, the ASO targets exon 14 in a PRPF3 RIC pre-mRNA comprising a retained intron 13. In some cases, the ASO targets an exon 14 sequence downstream (or 3') from the 3' splice site of a PRPF3 RIC pre-mRNA comprising a retained intron 13. In some cases, the ASO targets an exon 14 sequence about 2 to about 67 nucleotides downstream (or 3') from the 3' splice site of a PRPF3 RIC pre-mRNA comprising a retained intron 13. In some cases, the ASO has a sequence according to any one of SEQ ID NOs: 311-323.

### PRPF8

In some cases, the ASOs disclosed herein target a RIC pre-mRNA transcribed from a PRPF8 genomic sequence (a PRPF8 RIC pre-mRNA). In some cases, the PRPF8 genomic sequence is SEQ ID NO. 2. In some cases, the PRPF8 RIC pre-mRNA is SEQ ID NO. 4.

### PRPF8: retained intron 31

In some cases, the PRPF8 RIC pre-mRNA transcript comprises retained intron 31. In some cases, when the PRPF8 RIC pre-mRNA transcript comprises retained intron 31, the ASOs disclosed herein target SEQ ID NO: 448. In some cases, when the PRPF8 RIC pre-mRNA transcript comprises retained intron 31, the ASO has a sequence according to any one of SEQ ID NOs: 324-445. In some cases, the ASOs target a PRPF8 RIC pre-mRNA sequence.

In some cases, the ASO targets exon 31 of PRPF8 RIC pre-mRNA comprising a retained intron 31. In some cases, the ASO targets an exon 31 sequence upstream (or 5') from the 5' splice site of a PRPF8 RIC pre-mRNA comprising a retained intron 31. In some cases, the ASO targets an exon sequence about 4 to about 144 nucleotides upstream (or 5') from the 5' splice site of a PRPF8 RIC pre-mRNA comprising a retained intron 31. In some cases, the ASO has a sequence according to any one of SEQ ID NOs: 324-350.

In some cases, the ASO targets intron 31 in a *PRPF8* RIC pre-mRNA comprising a retained intron 31. In some cases, the ASO targets an intron 31 sequence downstream (or 3') from the 5' splice site of a PRPF8 RIC pre-mRNA comprising a retained intron 31. In some cases, the ASO targets an intron 31 sequence about 6 to about 156 nucleotides downstream (or 3') from the 5' splice site of a PRPF8 RIC pre-mRNA comprising a retained intron 31. In some cases, the ASO has a sequence according to any one of SEQ ID NOs: 351-381.

In some cases, the ASO targets an intron 31 sequence upstream (or 5') from the 3' splice site of a PRPF8 RIC pre-mRNA comprising a retained intron 31. In some cases, the ASO targets an intron 31 sequence about 16 to about 156 nucleotides upstream (or 5') from the 3' splice site of a PRPF8 RIC pre-mRNA a comprising retained intron 31. In some cases, the ASO has a sequence according to any one of SEQ ID NOs: 382-410.

In some cases, the ASO targets exon 32 in a PRPF8 RIC pre-mRNA comprising a retained intron 31. In some cases, the ASO targets an exon 32 sequence downstream (or 3') from the 3' splice site of a PRPF8 RIC pre-mRNA comprising a retained intron 31. In some cases, the ASO targets an exon 32 sequence about 2 to about 172 nucleotides downstream (or 3') from the 3' splice site of a PRPF8 RIC pre-mRNA comprising a retained intron 31. In some cases, the ASO has a sequence according to any one of SEQ ID NOs: 411-445.

### Protein Expression

As described above, *PRPF3* and *PRPF8* mutations in RP diseases are spread across the entire protein. In some cases, the methods described herein are used to increase the production of a functional PRPF3 protein. In other cases, the methods described herein are used to increase the production of a functional PRPF8 protein. In other cases, the methods described herein are used to increase the production of a functional PRPF3 protein or PRPF8 protein. As used herein, the term "functional" refers to the amount of activity or function of a PRPF3 or PRPF8 protein that is necessary to eliminate any one or more symptoms of an RP disease. In some cases, the methods are used to increase the production of a partially functional PRPF3 protein. In other cases, the methods are used to increase the production of a partially functional PRPF8 protein. In other cases, the methods are used to increase the production of a partially functional PRPF3 or PRPF8 protein. As used herein, the term "partially functional" refers to any amount of activity or function of the PRPF3 or PRPF8 protein that is less than the amount of activity or function that is necessary to eliminate or prevent any one or more symptoms of a disease. In some cases, a partially functional protein or RNA will have at least 10%, at least 20%, at least 30%, at least 40%, at least 50%, at least 60%, at least 70%, at least 75%, at least 80%, 85%, at least 90%, or at least 95% less activity relative to the fully functional protein or RNA.

In cases, the method is a method of increasing the expression of the PRPF3 protein by cells of a subject having a RIC pre-mRNA encoding the PRPF3 protein, wherein the subject has an RP disease caused by a deficient amount of activity of PRPF3 protein, and wherein the deficient amount of the PRPF3 protein is caused by haploinsufficiency of the PRPF3 protein. In such case, the subject has a first allele encoding a functional PRPF3 protein, and a second allele from which the PRPF3 protein is not produced. In another such case, the subject has a first allele encoding a functional PRPF3 protein, and a second allele encoding a nonfunctional PRPF3 protein. In another such case, the subject has a first allele encoding a functional PRPF3 protein, and a second allele encoding a partially functional PRPF3 protein. In any of these cases, the antisense oligomer binds to a targeted portion of the RIC pre-mRNA transcribed from the first allele (encoding functional PRPF3 protein), thereby inducing constitutive splicing of the retained intron from the RIC pre-mRNA, and causing an increase in the level of mature mRNA encoding functional PRPF3 protein, and an increase in the expression of the PRPF3 protein in the cells of the subject.

In some cases, the method is a method of increasing the expression of the PRPF8 protein by cells of a subject having a RIC pre-mRNA encoding the PRPF8 protein, wherein the subject has an RP disease caused by a deficient amount of activity of PRPF8 protein, and wherein the deficient amount of the PRPF8 protein is caused by haploinsufficiency of the PRPF8 protein. In such case, the subject has a first allele encoding a functional PRPF8 protein, and a second allele from which the PRPF8 protein is not produced. In another such case, the subject has a first allele encoding a functional PRPF8 protein, and a second allele encoding a nonfunctional PRPF8 protein. In another such case, the subject has a first allele encoding a functional PRPF8 protein, and a second allele encoding a partially functional PRPF8 protein. In any of these cases, the antisense oligomer binds to a targeted portion of the RIC pre-mRNA transcribed from the first allele (encoding functional PRPF8 protein), thereby inducing constitutive splicing of the retained intron from the RIC pre-mRNA, and causing an increase in the level of mature mRNA encoding functional PRPF8 protein, and an increase in the expression of the PRPF8 protein in the cells of the subject.

In some cases, the subject has a first allele encoding a functional PRPF3 protein, and a second allele encoding a partially functional PRPF3 protein, and the antisense oligomer binds to a targeted portion of the RIC pre-mRNA transcribed from the first allele (encoding functional PRPF3 protein) or a targeted portion of the RIC pre-mRNA transcribed from the second allele (encoding partially functional PRPF3 protein), thereby inducing constitutive splicing of the retained intron from the RIC pre-mRNA, and causing an increase in the level of mature mRNA encoding the PRPF3 protein, and an increase in the expression of functional or partially functional PRPF3 protein in the cells of the subject.

In other cases, the subject has a first allele encoding a functional PRPF8 protein, and a second allele encoding a partially functional PRPF8 protein, and the antisense oligomer binds to a targeted portion of the RIC pre-mRNA transcribed from the first allele (encoding functional PRPF8 protein) or a targeted portion of the RIC pre-mRNA transcribed from the second allele (encoding partially functional PRPF8 protein), thereby inducing constitutive splicing of the retained intron from the RIC pre-mRNA, and causing an increase in the level of mature mRNA encoding the PRPF8 protein, and an increase in the expression of functional or partially functional PRPF8 protein in the cells of the subject.

In related cases, the method is a method of using an ASO to increase the expression of a protein or functional RNA. In some cases, an ASO is used to increase the expression of PRPF3 protein in cells of a subject having a RIC pre-mRNA encoding PRPF3 protein, wherein the subject has a deficiency, e.g., RP18, in the amount or function of a PRPF3 protein.

In other related cases, the method is a method of using an ASO to increase the expression of a protein or functional RNA. In cases, an ASO is used to increase the expression of PRPF8 protein in cells of a subject having a RIC pre-mRNA encoding PRPF8 protein, wherein the subject has a deficiency, e.g., RP13, in the amount or function of a PRPF8 protein.

In cases, the RIC pre-mRNA transcript that encodes the protein that is causative of the disease is targeted by the ASOs described herein. In some cases, a RIC pre-mRNA transcript that encodes a protein that is not causative of the disease is targeted by the ASOs. For example, a disease that is the result of a mutation or deficiency of a first protein in a particular pathway may be ameliorated by targeting a RIC pre-mRNA that encodes a second protein, thereby increasing production of the second protein. In some cases, the function of the second protein is able to compensate for the mutation or deficiency of the first protein (which is causative of the disease or condition).

In cases, the subject has:
a. a first mutant allele from which
   i) the PRPF3 protein is produced at a reduced level compared to production from a wild-type allele,
   ii) the PRPF3 protein is produced in a form having reduced function compared to an equivalent wild-type protein, or
   iii) the PRPF3 protein or functional RNA is not produced; and
b.a second mutant allele from which
   i) the PRPF3 protein is produced at a reduced level compared to production from a wild-type allele,
   ii) the PRPF3 protein is produced in a form having reduced function compared to an equivalent wild-type protein, or
   iii) the PRPF3 protein is not produced, and
wherein the RIC pre-mRNA is transcribed from the first allele and/or the second allele. In these cases, the ASO binds to a targeted portion of the RIC pre-mRNA transcribed from the first allele or the second allele, thereby inducing constitutive splicing of the retained intron from the RIC pre-mRNA, and causing an increase in the level of mRNA encoding PRPF3 protein and an increase in the expression of the target protein or functional RNA in the cells of the subject. In these cases, the target protein or functional RNA having an increase in expression level resulting from the constitutive splicing of the retained intron from the RIC pre-mRNA is either in a form having reduced function compared to the equivalent wild-type protein (partially-functional), or having full function compared to the equivalent wild-type protein (fully-functional).

In cases, the subject has:
a. a first mutant allele from which
   i) the PRPF8 protein is produced at a reduced level compared to production from a wild-type allele,
   ii) the PRPF8 protein is produced in a form having reduced function compared to an equivalent wild-type protein, or
   iii) the PRPF8 protein or functional RNA is not produced; and
b.a second mutant allele from which
   iv) the PRPF8 protein is produced at a reduced level compared to production from a wild-type allele,
   v) the PRPF8 protein is produced in a form having reduced function compared to an equivalent wild-type protein, or
   vi) the PRPF8 protein is not produced, and
wherein the RIC pre-mRNA is transcribed from the first allele and/or the second allele. In these cases, the ASO binds to a targeted portion of the RIC pre-mRNA transcribed from the first allele or the second allele, thereby inducing constitutive splicing of the retained intron from the RIC pre-mRNA, and causing an increase in the level of mRNA encoding PRPF8 protein and an increase in the expression of the target protein or functional RNA in the cells of the subject. In these cases, the target protein or functional RNA having an increase in expression level resulting from the constitutive splicing of the retained intron from the RIC pre-mRNA is either in a form having reduced function compared to the equivalent wild-type protein (partially-functional), or having full function compared to the equivalent wild-type protein (fully-functional).

In cases, the level of mRNA encoding PRPF3 or PRPF8 protein is increased 1.1 to 10-fold, when compared to the amount of mRNA encoding PRPF3 or PRPF8 that is produced in a control cell, *e.g.,* one that is not treated with the antisense oligomer or one that is treated with an antisense oligomer that does not bind to the targeted portion of the PRPF3 or PRPF8 RIC pre-mRNA.

In cases, the condition caused by a deficient amount or activity of PRPF3 or PRPF8 protein is not a condition caused by alternative or aberrant splicing of the retained intron to which the ASO is targeted. In cases, the condition caused by a deficient amount or activity of the PRPF3 or PRPF8 protein is not a condition caused by alternative or aberrant splicing of any retained intron in a RIC pre-mRNA encoding the PRPF3 or PRPF8 protein.

In some cases, a subject treated using the methods of the disclosure expresses a partially functional PRPF3 or PRPF8 protein from one allele, wherein the partially functional PRPF3 or PRPF8 protein is caused by a frameshift mutation, a nonsense mutation, a missense mutation, or a partial gene deletion. In cases, a subject treated using the methods of the disclosure expresses a nonfunctional PRPF3 or PRPF8 protein from one allele, wherein the nonfunctional PRPF3 or PRPF8 protein is caused by a frameshift mutation, a nonsense mutation, a missense mutation, a partial gene deletion, in one allele. In cases, a subject treated using the methods of the disclosure has a PRPF3 or PRPF8 whole gene deletion, in one allele.

In some cases, the subject has a PRPF3 missense mutation selected from P493S and T494M. In other cases, the subject has a PRPF8 missense mutation selected from H2309P, H2309R, R2310K, P2301T, F2304L, R2310G and F2314L. In cases, a subject having any mutation known in the art and described in the literature, e.g., by McKie et al 2001 referenced above, is treated using the methods and compositions of the present disclosure.

### Use of TANGO for Increasing Protein Expression

As described above, in some cases, Targeted Augmentation of Nuclear Gene Output (TANGO) is used in the methods of the disclosure to increase expression of a PRPF3 or PRPF8 protein. In these cases, a retained-intron-containing pre-mRNA (RIC pre-mRNA) encoding PRPF3 or PRPF8 protein is present in the nucleus of a cell. Cells having a *PRPF3* or *PRPF8* RIC pre-mRNA that comprises a retained intron, an exon flanking the 5' splice site, and an exon flanking the 3' splice site, encoding the PRPF3 or PRPF8 protein, are contacted with antisense oligomers (ASOs) that are complementary to a targeted portion of the RIC pre-mRNA. Hybridization of the ASOs to the targeted portion of the RIC pre-mRNA results in enhanced splicing at the splice site (5' splice site or 3' splice site) of the retained intron and subsequently increases target protein production.

The terms "pre-mRNA," and "pre-mRNA transcript" may be used interchangeably and refer to any pre-mRNA species that contains at least one intron. In cases, pre-mRNA or pre-mRNA transcripts comprise a 5'-7-methylguanosine cap and/or a poly-A tail. In cases, pre-mRNA or pre-mRNA transcripts comprise both a 5'-7-methylguanosine cap and a poly-A tail. In some cases, the pre-mRNA transcript does not comprise a 5'-7-methylguanosine cap and/or a poly-A tail. A pre-mRNA transcript is a non-productive messenger RNA (mRNA) molecule if it is not translated into a protein (or transported into the cytoplasm from the nucleus).

As used herein, a "retained-intron-containing pre-mRNA" ("RIC pre-mRNA") is a pre-mRNA transcript that contains at least one retained intron. The RIC pre-mRNA contains a retained intron, an exon flanking the 5' splice site of the retained intron, an exon flanking the 3' splice site of the retained intron, and encodes the target protein. An "RIC pre-mRNA encoding a target protein" is understood to encode the target protein when fully spliced. A "retained intron" is any intron that is present in a pre-mRNA transcript when one or more other introns, such as an adjacent intron, encoded by the same gene have been spliced out of the same pre-mRNA transcript. In some cases, the retained intron is the most abundant intron in RIC pre-mRNA encoding the target protein. In cases, the retained intron is the most abundant intron in a population of RIC pre-mRNAs transcribed from the gene encoding the target protein in a cell, wherein the population of RIC pre-mRNAs comprises two or more retained introns. In cases, an antisense oligomer targeted to the most abundant intron in the population of RIC pre-mRNAs encoding the target protein induces splicing out of two or more retained introns in the population, including the retained intron to which the antisense oligomer is targeted or binds. In cases, a mature mRNA encoding the target protein is thereby produced. The terms "mature mRNA," and "fully-spliced mRNA," are used interchangeably herein to describe a fully processed mRNA encoding a target protein (*e.g*., mRNA that is exported from the nucleus into the cytoplasm and translated into target protein) or a fully processed functional RNA. The term "productive mRNA," also can be used to describe a fully processed mRNA encoding a target protein. In cases, the targeted region is in a retained intron that is the most abundant intron in a RIC pre-mRNA encoding the PRPF3 and/or PRPF8 protein. In cases, the most retained intron in a RIC pre-mRNA encoding the PRPF3 protein is intron 12. In cases, the most retained intron in a RIC pre-mRNA encoding the PRPF3 protein is intron 13. In some cases, the most retained intron in a RIC pre-mRNA encoding the PRPF8 protein is intron 31.

In cases, a retained intron is an intron that is identified as a retained intron based on a determination of at least about 5%, at least about 10%, at least about 15%, at least about 20%, at least about 25%, at least about 30%, at least about 35%, at least about 40%, at least about 45%, or at least about 50%, retention. In cases, a retained intron is an intron that is identified as a retained intron based on a determination of about 5% to about 100%, about 5% to about 95%, about 5% to about 90%, about 5% to about 85%, about 5% to about 80%, about 5% to about 75%, about 5% to about 70%, about 5% to about 65%, about 5% to about 60%, about 5% to about 65%, about 5% to about 60%, about 5% to about 55%, about 5% to about 50%, about 5% to about 45%, about 5% to about 40%, about 5% to about 35%, about 5% to about 30%, about 5% to about 25%, about 5% to about 20%, about 5% to about 15%, about 10% to about 100%, about 10% to about 95%, about 10% to about 90%, about 10% to about 85%, about 10% to about 80%, about 10% to about 75%, about 10% to about 70%, about 10% to about 65%, about 10% to about 60%, about 10% to about 65%, about 10% to about 60%, about 10% to about 55%, about 10% to about 50%, about 10% to about 45%, about 10% to about 40%, about 10% to about 35%, about 10% to about 30%, about 10% to about 25%, about 10% to about 20%, about 15% to about 100%, about 15% to about 95%, about 15% to about 90%, about 15% to about 85%, about 15% to about 80%, about 15% to about 75%, about 15% to about 70%, about 15% to about 65%, about 15% to about 60%, about 15% to about 65%, about 15% to about 60%, about 15% to about 55%, about 15% to about 50%, about 15% to about 45%, about 15% to about 40%, about 15% to about 35%, about 15% to about 30%, about 15% to about 25%, about 20% to about 100%, about 20% to about 95%, about 20% to about 90%, about 20% to about 85%, about 20% to about 80%, about 20% to about 75%, about 20% to about 70%, about 20% to about 65%, about 20% to about 60%, about 20% to about 65%, about 20% to about 60%, about 20% to about 55%, about 20% to about 50%, about 20% to about 45%, about 20% to about 40%, about 20% to about 35%, about 20% to about 30%, about 25% to about 100%, about 25% to about 95%, about 25% to about 90%, about 25% to about 85%, about 25% to about 80%, about 25% to about 75%, about 25% to about 70%, about 25% to about 65%, about 25% to about 60%, about 25% to about 65%, about 25% to about 60%, about 25% to about 55%, about 25% to about 50%, about 25% to about 45%, about 25% to about 40%, or about 25% to about 35%, retention. ENCODE data (described by, e.g., Tilgner, et al., 2012, "Deep sequencing of subcellular RNA fractions shows splicing to be predominantly co-transcriptional in the human genome but inefficient for IncRNAs," Genome Research 22(9): 1616-25) can be used to aid in identifying retained introns.

As used herein, the term "comprise" or variations thereof such as "comprises" or "comprising" are to be read to indicate the inclusion of any recited feature (*e.g.* in the case of an antisense oligomer, a defined nucleobase sequence) but not the exclusion of any other features. Thus, as used herein, the term "comprising" is inclusive and does not exclude additional, unrecited features (*e.g.* in the case of an antisense oligomer, the presence of additional, unrecited nucleobases).

In cases of any of the compositions and methods provided herein, "comprising" may be replaced with "consisting essentially of" or "consisting of." The phrase "consisting essentially of' is used herein to require the specified feature(s) (*e.g*. nucleobase sequence) as well as those which do not materially affect the character or function of the present disclosure. As used herein, the term "consisting" is used to indicate the presence of the recited feature (*e.g*. nucleobase sequence) alone (so that in the case of an antisense oligomer consisting of a specified nucleobase sequence, the presence of additional, unrecited nucleobases is excluded).

In cases, the targeted region is in a retained intron that is the second most abundant intron in a population of RIC pre-mRNA encoding the PRPF3 or PRPF8 protein. For example, the second most abundant retained intron may be targeted rather than the most abundant retained intron due to the uniqueness of the nucleotide sequence of the second most abundant retained intron, ease of ASO design to target a particular nucleotide sequence, and/or amount of increase in protein production resulting from targeting the intron with an ASO. In cases, the retained intron is the second most abundant intron in a population of RIC pre-mRNAs transcribed from the gene encoding the target protein in a cell, wherein the population of RIC pre-mRNAs comprises two or more retained introns. In cases, an antisense oligomer targeted to the second most abundant intron in the population of RIC pre-mRNAs encoding the target protein induces splicing out of two or more retained introns in the population, including the retained intron to which the antisense oligomer is targeted or binds. In cases, fully-spliced (mature) RNA encoding the target protein is thereby produced. In cases, the second-most abundant retained intron in a population of RIC pre-mRNA encoding the PRPF3 protein is intron 12. In cases, the second-most abundant retained intron in a population of RIC pre-mRNA encoding the PRPF3 protein is intron 13.

In cases, an ASO is complementary to a targeted region that is within a non-retained intron in a RIC pre-mRNA. In cases, the targeted portion of the RIC pre-mRNA is within: the region +6 to +100 relative to the 5' splice site of the non-retained intron; or the region -16 to -100 relative to the 3' splice site of the non-retained intron. In cases, the targeted portion of the RIC pre-mRNA is within the region +100 relative to the 5' splice site of the non-retained intron to - 100 relative to the 3' splice site of the non-retained intron. As used to identify the location of a region or sequence, "within" is understood to include the residues at the positions recited. For example, a region +6 to +100 includes the residues at positions +6 and +100. In cases, fully-spliced (mature) RNA encoding the target protein is thereby produced.

In cases, the retained intron of the RIC pre-mRNA is an inefficiently spliced intron. As used herein, "inefficiently spliced" may refer to a relatively low frequency of splicing at a splice site adjacent to the retained intron (5' splice site or 3' splice site) as compared to the frequency of splicing at another splice site in the RIC pre-mRNA. The term "inefficiently spliced" may also refer to the relative rate or kinetics of splicing at a splice site, in which an "inefficiently spliced" intron may be spliced or removed at a slower rate as compared to another intron in a RIC pre-mRNA.

In cases, the 9-nucleotide sequence at -3e to -1e of the exon flanking the 5' splice site and +1 to +6 of the retained intron is identical to the corresponding wild-type sequence. In cases, the 16 nucleotide sequence at -15 to -1 of the retained intron and +1e of the exon flanking the 3' splice site is identical to the corresponding wild-type sequence. As used herein, the "wild-type sequence" refers to the nucleotide sequence for the *PRPF3* or *PRPF8* gene in the published reference genome deposited in the NCBI repository of biological and scientific information. As used herein, the "wild-type *PRPF3* sequence" refers to the canonical sequence available at NCBI Gene ID 9129. As used herein, the "wild-type *PRPF8* sequence" refers to the canonical sequence available at NCBI Gene ID 10594. Also used herein, a nucleotide position denoted with an "e" indicates the nucleotide is present in the sequence of an exon *(e.g.,* the exon flanking the 5' splice site or the exon flanking the 3' splice site).

The methods involve contacting cells with an ASO that is complementary to a portion of a pre-mRNA encoding PRPF3 or PRPF8 protein, resulting in increased expression of the PRPF3 or PRPF8 protein, respectively. As used herein, "contacting" or administering to cells refers to any method of providing an ASO in immediate proximity with the cells such that the ASO and the cells interact. A cell that is contacted with an ASO will take up or transport the ASO into the cell. The method involves contacting a condition or disease-associated or condition or disease-relevant cell with any of the ASOs described herein. In some cases, the ASO may be further modified or attached (*e.g*., covalently attached) to another molecule to target the ASO to a cell type, enhance contact between the ASO and the condition or disease-associated or condition or disease-relevant cell, or enhance uptake of the ASO.

As used herein, the term "increasing protein production" or "increasing expression of a target protein" means enhancing the amount of protein that is translated from an mRNA in a cell. A "target protein" may be any protein for which increased expression/production is desired.

In cases, contacting a cell that expresses a *PRPF3* or *PRPF8* RIC pre-mRNA with an ASO that is complementary to a targeted portion of the *PRPF3* or *PRPF8* RIC pre-mRNA transcript results in a measurable increase in the amount of the PRPF3 or PRPF8 protein *(e.g.,* a target protein) encoded by the pre-mRNA. Methods of measuring or detecting production of a protein will be evident to one of skill in the art and include any known method, for example, Western blotting, flow cytometry, immunofluorescence microscopy, and ELISA.

In cases, contacting cells with an ASO that is complementary to a targeted portion of a *PRPF3* and/or *PRPF8* RIC pre-mRNA transcript results in an increase in the amount of PRPF3 and/or PRPF8 protein produced by at least 10, 20, 30, 40, 50, 60, 80, 100, 150, 200, 250, 300, 350, 400, 450, 500, or 1000%, compared to the amount of the protein produced by a cell in the absence of the ASO/absence of treatment. In cases, the total amount of PRPF3 or PRPF8 protein produced by the cell to which the antisense oligomer was contacted is increased about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1.1-fold, at least about 1.5-fold, at least about 2-fold, at least about 2.5-fold, at least about 3-fold, at least about 3.5-fold, at least about 4-fold, at least about 5-fold, or at least about 10-fold, compared to the amount of target protein produced by an control compound. A control compound can be, for example, an oligonucleotide that is not complementary to the targeted portion of the RIC pre-mRNA.

In some cases, contacting cells with an ASO that is complementary to a targeted portion of a *PRPF3* or *PRPF8* RIC pre-mRNA transcript results in an increase in the amount of mRNA encoding PRPF3 or PRPF8, including the mature mRNA encoding the target protein. In some cases, the amount of mRNA encoding PRPF3 or PRPF8 protein, or the mature mRNA encoding the PRPF3 or PRPF8 protein, is increased by at least 10, 20, 30, 40, 50, 60, 80, 100, 150, 200, 250, 300, 350, 400, 450, 500, or 1000%, compared to the amount of the protein produced by a cell in the absence of the ASO/absence of treatment. In cases, the total amount of the mRNA encoding PRPF3 or PRPF8 protein, or the mature mRNA encoding PRPF3 or PRPF8 protein produced in the cell to which the antisense oligomer was contacted is increased about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1.1-fold, at least about 1.5-fold, at least about 2-fold, at least about 2.5-fold, at least about 3-fold, at least about 3.5-fold, at least about 4-fold, at least about 5-fold, or at least about 10-fold compared to the amount of mature RNA produced in an untreated cell, *e.g.,* an untreated cell or a cell treated with a control compound. A control compound can be, for example, an oligonucleotide that is not complementary to the targeted portion of the *PRPF3* or *PRPF8* RIC pre-mRNA.

### Constitutive Splicing of a Retained Intron from a RIC pre-mRNA

The methods and antisense oligonucleotide compositions provided herein are useful for increasing the expression of PRPF3 or PRPF8 protein in cells, for example, in a subject having RP caused by a deficiency in the amount or activity of PRPF3 or PRPF8 protein, by increasing the level of mRNA encoding PRPF3 or PRPF8 protein, or the mature mRNA encoding PRPF3 or PRPF8 protein. In particular, the methods and compositions as described herein induce the constitutive splicing of a retained intron from a PRPF3 or PRPF8 RIC pre-mRNA transcript encoding PRPF3 or PRPF8 protein, thereby increasing the level of mRNA encoding PRPF3 or PRPF8 protein, or the mature mRNA encoding PRPF3 or PRPF8 protein and increasing the expression of PRPF3 or PRPF8 protein.

Constitutive splicing of a retained intron from a RIC pre-mRNA correctly removes the retained intron from the RIC pre-mRNA, wherein the retained intron has wild-type splice sequences. Constitutive splicing, as used herein, does not encompass splicing of a retained intron from a RIC pre-mRNA transcribed from a gene or allele having a mutation that causes alternative splicing or aberrant splicing of a pre-mRNA transcribed from the gene or allele. For example, constitutive splicing of a retained intron, as induced using the methods and antisense oligonucleotides provided herein, does not correct aberrant splicing in or influence alternative splicing of a pre-mRNA to result in an increased expression of a target protein or functional RNA.

In some cases, constitutive splicing correctly removes a retained intron from a *PRPF3* or *PRPF8* RIC pre-mRNA, wherein the *PRPF3* or *PRPF8* RIC pre-mRNA is transcribed from a wild-type gene or allele, or a polymorphic gene or allele, that encodes a fully-functional target protein or functional RNA, and wherein the gene or allele does not have a mutation that causes alternative splicing or aberrant splicing of the retained intron.

In some cases, constitutive splicing of a retained intron from a *PRPF3* or *PRPF8* RIC pre-mRNA encoding PRPF3 or PRPF8 protein correctly removes a retained intron from a *PRPF3* or *PRPF8* RIC pre-mRNA encoding PRPF3 or PRPF8 protein, wherein the *PRPF3* or *PRPF8* RIC pre-mRNA is transcribed from a gene or allele from which the target gene or functional RNA is produced at a reduced level compared to production from a wild-type allele, and wherein the gene or allele does not have a mutation that causes alternative splicing or aberrant splicing of the retained intron. In these cases, the correct removal of the constitutively spliced retained intron results in production of target protein or functional RNA that is functional when compared to an equivalent wild-type protein or functional RNA.

In other cases, constitutive splicing correctly removes a retained intron from a *PRPF3* or *PRPF8* RIC pre-mRNA, wherein the *PRPF3* or *PRPF8* RIC pre-mRNA is transcribed from a gene or allele that encodes a target protein or functional RNA produced in a form having reduced function compared to an equivalent wild-type protein or functional RNA, and wherein the gene or allele does not have a mutation that causes alternative splicing or aberrant splicing of the retained intron. In these cases, the correct removal of the constitutively spliced retained intron results in production of partially functional target protein, or functional RNA that is partially functional when compared to an equivalent wild-type protein or functional RNA.

"Correct removal" of the retained intron by constitutive splicing refers to removal of the entire intron, without removal of any part of an exon.

In some cases, an antisense oligomer as described herein or used in any method described herein does not increase the amount of mRNA encoding PRPF3 or PRPF8 protein or the amount of PRPF3 or PRPF8 protein by modulating alternative splicing or aberrant splicing of a pre-mRNA transcribed from the PRPF3 or PRPF8 gene. Modulation of alternative splicing or aberrant splicing can be measured using any known method for analyzing the sequence and length of RNA species, *e.g*., by RT-PCR and using methods described elsewhere herein and in the literature. In some cases, modulation of alternative or aberrant splicing is determined based on an increase or decrease in the amount of the spliced species of interest of at least 10% or 1.1-fold. In some cases, modulation is determined based on an increase or decrease at a level that is at least 10% to 100% or 1.1 to 10-fold, as described herein regarding determining an increase in mRNA encoding PRPF3 or PRPF8 protein in the methods of the disclosure.

In cases, the method is a method wherein the *PRPF3* or *PRPF8* RIC pre-mRNA was produced by partial splicing of a wild-type *PRPF3* or *PRPF8* pre-mRNA. In cases, the method is a method wherein the *PRPF3* or *PRPF8* RIC pre-mRNA was produced by partial splicing of a full-length wild-type *PRPF3* or *PRPF8* pre-mRNA. In cases, the *PRPF3* or *PRPF8* RIC pre-mRNA was produced by partial splicing of a full-length *PRPF3* or *PRPF8* pre-mRNA. In these cases, a full-length *PRPF3* or *PRPF8* pre-mRNA may have a polymorphism in a splice site of the retained intron that does not impair correct splicing of the retained intron as compared to splicing of the retained intron having the wild-type splice site sequence.

In cases, the mRNA encoding PRPF3 or PRPF8 protein is a full-length mature mRNA, or a wild-type mature mRNA. In these cases, a full-length mature mRNA may have a polymorphism that does not affect the activity of the target protein or the functional RNA encoded by the mature mRNA, as compared to the activity of PRPF3 or PRPF8 protein encoded by the wild-type mature mRNA.

### Antisense Oligomers

One aspect of the present disclosure is a composition comprising antisense oligomers that enhances splicing by binding to a targeted portion of a *PRPF3* or *PRPF8* RIC pre-mRNA. As used herein, the terms "ASO" and "antisense oligomer" are used interchangeably and refer to an oligomer such as a polynucleotide, comprising nucleobases, that hybridizes to a target nucleic acid *(e.g.,* a *PRPF3* or *PRPF8* RIC pre-mRNA) sequence by Watson-Crick base pairing or wobble base pairing (G-U). The ASO may have exact sequence complementary to the target sequence or near complementarity (*e.g*., sufficient complementarity to bind the target sequence and enhancing splicing at a splice site). ASOs are designed so that they bind (hybridize) to a target nucleic acid *(e.g.,* a targeted portion of a pre-mRNA transcript) and remain hybridized under physiological conditions. Typically, if they hybridize to a site other than the intended (targeted) nucleic acid sequence, they hybridize to a limited number of sequences that are not a target nucleic acid (to a few sites other than a target nucleic acid). Design of an ASO can take into consideration the occurrence of the nucleic acid sequence of the targeted portion of the pre-mRNA transcript or a sufficiently similar nucleic acid sequence in other locations in the genome or cellular pre-mRNA or transcriptome, such that the likelihood the ASO will bind other sites and cause "off-target" effects is limited. Any antisense oligomers known in the art, for example in PCT Application No. PCT/US2014/054151, published as WO 2015/035091, titled "Reducing Nonsense-Mediated mRNA Decay," can be used to practice the methods described herein.

In some cases, ASOs "specifically hybridize" to or are "specific" to a target nucleic acid or a targeted portion of a RIC pre-mRNA. Typically such hybridization occurs with a Tm substantially greater than 37°C, preferably at least 50°C, and typically between 60°C to approximately 90°C. Such hybridization preferably corresponds to stringent hybridization conditions. At a given ionic strength and pH, the Tm is the temperature at which 50% of a target sequence hybridizes to a complementary oligonucleotide.

Oligomers, such as oligonucleotides, are "complementary" to one another when hybridization occurs in an antiparallel configuration between two single-stranded polynucleotides. A double-stranded polynucleotide can be "complementary" to another polynucleotide, if hybridization can occur between one of the strands of the first polynucleotide and the second. Complementarity (the degree to which one polynucleotide is complementary with another) is quantifiable in terms of the proportion (e.g., the percentage) of bases in opposing strands that are expected to form hydrogen bonds with each other, according to generally accepted base-pairing rules. The sequence of an antisense oligomer (ASO) need not be 100% complementary to that of its target nucleic acid to hybridize. In certain cases, ASOs can comprise at least 70%, at least 75%, at least 80%, at least 85%, at least 90%, at least 95%, at least 96%, at least 97%, at least 98%, or at least 99% sequence complementarity to a target region within the target nucleic acid sequence to which they are targeted. For example, an ASO in which 18 of 20 nucleobases of the oligomeric compound are complementary to a target region, and would therefore specifically hybridize, would represent 90 percent complementarity. In this example, the remaining noncomplementary nucleobases may be clustered together or interspersed with complementary nucleobases and need not be contiguous to each other or to complementary nucleobases. Percent complementarity of an ASO with a region of a target nucleic acid can be determined routinely using BLAST programs (basic local alignment search tools) and PowerBLAST programs known in the art (Altschul et al., J. Mol. Biol., 1990, 215, 403-410; Zhang and Madden, Genome Res., 1997, 7, 649-656).

An ASO need not hybridize to all nucleobases in a target sequence and the nucleobases to which it does hybridize may be contiguous or noncontiguous. ASOs may hybridize over one or more segments of a pre-mRNA transcript, such that intervening or adjacent segments are not involved in the hybridization event *(e.g.,* a loop structure or hairpin structure may be formed). In certain cases, an ASO hybridizes to noncontiguous nucleobases in a target pre-mRNA transcript. For example, an ASO can hybridize to nucleobases in a pre-mRNA transcript that are separated by one or more nucleobase(s) to which the ASO does not hybridize.

The ASOs described herein comprise nucleobases that are complementary to nucleobases present in a target portion of a RIC pre-mRNA. The term ASO embodies oligonucleotides and any other oligomeric molecule that comprises nucleobases capable of hybridizing to a complementary nucleobase on a target mRNA but does not comprise a sugar moiety, such as a peptide nucleic acid (PNA). The ASOs may comprise naturally-occurring nucleotides, nucleotide analogs, modified nucleotides, or any combination of two or three of the preceding. The term "naturally occurring nucleotides" includes deoxyribonucleotides and ribonucleotides. The term "modified nucleotides" includes nucleotides with modified or substituted sugar groups and/or having a modified backbone. In some cases, all of the nucleotides of the ASO are modified nucleotides. Chemical modifications of ASOs or components of ASOs that are compatible with the methods and compositions described herein will be evident to one of skill in the art and can be found, for example, in U.S. Patent No. 8,258,109 B2, U.S. Patent No. 5,656,612, U.S. Patent Publication No. 2012/0190728, and Dias and Stein, Mol. Cancer Ther. 2002, 1 , 347-355.

The nucleobase of an ASO may be any naturally occurring, unmodified nucleobase such as adenine, guanine, cytosine, thymine and uracil, or any synthetic or modified nucleobase that is sufficiently similar to an unmodified nucleobase such that it is capable of hydrogen bonding with a nucleobase present on a target pre-mRNA. Examples of modified nucleobases include, without limitation, hypoxanthine, xanthine, 7-methylguanine, 5,6-dihydrouracil, 5-methylcytosine, and 5-hydroxymethoylcytosine.

The ASOs described herein also comprise a backbone structure that connects the components of an oligomer. The term "backbone structure" and "oligomer linkages" may be used interchangeably and refer to the connection between monomers of the ASO. In naturally occurring oligonucleotides, the backbone comprises a 3'-5' phosphodiester linkage connecting sugar moieties of the oligomer. The backbone structure or oligomer linkages of the ASOs described herein may include (but are not limited to) phosphorothioate, phosphorodithioate, phosphoroselenoate, phosphorodiselenoate, phosphoroanilothioate, phosphoraniladate, phosphoramidate, and the like. See e.g., LaPlanche et al., Nucleic Acids Res. 14:9081 (1986); Stec et al., J. Am. Chem. Soc. 106:6077 (1984), Stein et al., Nucleic Acids Res. 16:3209 (1988), Zon et al., Anti Cancer Drug Design 6:539 (1991); Zon et al., Oligonucleotides and Analogues: A Practical Approach, pp. 87-108 (F. Eckstein, Ed., Oxford University Press, Oxford England (1991)); Stec et al., U.S. Pat. No. 5,151,510; Uhlmann and Peyman, Chemical Reviews 90:543 (1990). In some cases, the backbone structure of the ASO does not contain phosphorous but rather contains peptide bonds, for example in a peptide nucleic acid (PNA), or linking groups including carbamate, amides, and linear and cyclic hydrocarbon groups. In some cases, the backbone modification is a phosphothioate linkage. In some cases, the backbone modification is a phosphoramidate linkage.

In cases, the stereochemistry at each of the phosphorus internucleotide linkages of the ASO backbone is random. In cases, the stereochemistry at each of the phosphorus internucleotide linkages of the ASO backbone is controlled and is not random. For example, U.S. Pat. App. Pub. No. 2014/0194610, "Methods for the Synthesis of Functionalized Nucleic Acids," incorporated herein by reference, describes methods for independently selecting the handedness of chirality at each phosphorous atom in a nucleic acid oligomer. An ASO used in the methods of the disclosure, including, but not limited to, any of the ASOs set forth herein in Table 1, comprises an ASO having phosphorus internucleotide linkages that are not random. A composition used in the methods of the disclosure comprises a pure diastereomeric ASO. In cases, a composition used in the methods of the disclosure comprises an ASO that has diastereomeric purity of at least about 90%, at least about 91%, at least about 92%, at least about 93%, at least about 94%, at least about 95%, at least about 96%, at least about 97%, at least about 98%, at least about 99%, about 100%, about 90% to about 100%, about 91% to about 100%, about 92% to about 100%, about 93% to about 100%, about 94% to about 100%, about 95% to about 100%, about 96% to about 100%, about 97% to about 100%, about 98% to about 100% , or about 99% to about 100%.

In cases, the ASO has a nonrandom mixture of Rp and Sp configurations at its phosphorus internucleotide linkages. For example, it has been suggested that a mix of Rp and Sp is required in antisense oligonucleotides to achieve a balance between good activity and nuclease stability (Wan, et al., 2014, "Synthesis, biophysical properties and biological activity of second generation antisense oligonucleotides containing chiral phosphorothioate linkages," Nucleic Acids Research 42(22): 13456-13468, incorporated herein by reference). In cases, an ASO used in the methods of the disclosure, including, but not limited to, any of the ASOs set forth herein in Table 1, comprises about 5-100% Rp, at least about 5% Rp, at least about 10% Rp, at least about 15% Rp, at least about 20% Rp, at least about 25% Rp, at least about 30% Rp, at least about 35% Rp, at least about 40% Rp, at least about 45% Rp, at least about 50% Rp, at least about 55% Rp, at least about 60% Rp, at least about 65% Rp, at least about 70% Rp, at least about 75% Rp, at least about 80% Rp, at least about 85% Rp, at least about 90% Rp, or at least about 95% Rp, with the remainder Sp, or about 100% Rp. In cases, an ASO used in the methods of the disclosure, including, but not limited to, any of the ASOs set forth herein in Table 1, comprises about 10% to about 100% Rp, about 15% to about 100% Rp, about 20% to about 100% Rp, about 25% to about 100% Rp, about 30% to about 100% Rp, about 35% to about 100% Rp, about 40% to about 100% Rp, about 45% to about 100% Rp, about 50% to about 100% Rp, about 55% to about 100% Rp, about 60% to about 100% Rp, about 65% to about 100% Rp, about 70% to about 100% Rp, about 75% to about 100% Rp, about 80% to about 100% Rp, about 85% to about 100% Rp, about 90% to about 100% Rp, or about 95% to about 100% Rp, about 20% to about 80% Rp, about 25% to about 75% Rp, about 30% to about 70% Rp, about 40% to about 60% Rp, or about 45% to about 55% Rp, with the remainder Sp.

In cases, an ASO used in the methods of the disclosure, including, but not limited to, any of the ASOs set forth herein in Table 1, comprises about 5-100% Sp, at least about 5% Sp, at least about 10% Sp, at least about 15% Sp, at least about 20% Sp, at least about 25% Sp, at least about 30% Sp, at least about 35% Sp, at least about 40% Sp, at least about 45% Sp, at least about 50% Sp, at least about 55% Sp, at least about 60% Sp, at least about 65% Sp, at least about 70% Sp, at least about 75% Sp, at least about 80% Sp, at least about 85% Sp, at least about 90% Sp, or at least about 95% Sp, with the remainder Rp, or about 100% Sp. In cases, an ASO used in the methods of the disclosure, including, but not limited to, any of the ASOs set forth herein in Table 1, comprises about 10% to about 100% Sp, about 15% to about 100% Sp, about 20% to about 100% Sp, about 25% to about 100% Sp, about 30% to about 100% Sp, about 35% to about 100% Sp, about 40% to about 100% Sp, about 45% to about 100% Sp, about 50% to about 100% Sp, about 55% to about 100% Sp, about 60% to about 100% Sp, about 65% to about 100% Sp, about 70% to about 100% Sp, about 75% to about 100% Sp, about 80% to about 100% Sp, about 85% to about 100% Sp, about 90% to about 100% Sp, or about 95% to about 100% Sp, about 20% to about 80% Sp, about 25% to about 75% Sp, about 30% to about 70% Sp, about 40% to about 60% Sp, or about 45% to about 55% Sp, with the remainder Rp.

Any of the ASOs described herein may contain a sugar moiety that comprises ribose or deoxyribose, as present in naturally occurring nucleotides, or a modified sugar moiety or sugar analog, including a morpholine ring. Non-limiting examples of modified sugar moieties include 2' substitutions such as 2'-O-methyl (2'-O-Me), 2'-O-methoxyethyl (2'MOE), 2'-O-aminoethyl, 2'F; N3'->P5' phosphoramidate, 2'dimethylaminooxyethoxy, 2'dimethylaminoethoxyethoxy, 2'-guanidinidium, 2'-O-guanidinium ethyl, carbamate modified sugars, and bicyclic modified sugars. In some cases, the sugar moiety modification is selected from 2'-O-Me, 2'F, and 2'MOE. In some cases, the sugar moiety modification is an extra bridge bond, such as in a locked nucleic acid (LNA). In some cases the sugar analog contains a morpholine ring, such as phosphorodiamidate morpholino (PMO). In some cases, the sugar moiety comprises a ribofuransyl or 2'deoxyribofuransyl modification. In some cases, the sugar moiety comprises 2'4'-constrained 2'O-methyloxyethyl (cMOE) modifications. In some cases, the sugar moiety comprises cEt 2', 4' constrained 2'-O ethyl BNA modifications. In some cases, the sugar moiety comprises tricycloDNA (tcDNA) modifications. In some cases, the sugar moiety comprises ethylene nucleic acid (ENA) modifications. In some cases, the sugar moiety comprises MCE modifications. Modifications are known in the art and described in the literature, *e.g*., by Jarver, et al., 2014, "A Chemical View of Oligonucleotides for Exon Skipping and Related Drug Applications," Nucleic Acid Therapeutics 24(1): 37-47.

In some examples, each monomer of the ASO is modified in the same way, for example each linkage of the backbone of the ASO comprises a phosphorothioate linkage or each ribose sugar moiety comprises a 2'O-methyl modification. Such modifications that are present on each of the monomer components of an ASO are referred to as "uniform modifications." In some examples, a combination of different modifications may be desired, for example, an ASO may comprise a combination of phosphorodiamidate linkages and sugar moieties comprising morpholine rings (morpholinos). Combinations of different modifications to an ASO are referred to as "mixed modifications" or "mixed chemistries."

In some cases, the ASO comprises one or more backbone modification. In some cases, the ASO comprises one or more sugar moiety modification. In some cases, the ASO comprises one or more backbone modification and one or more sugar moiety modification. In some cases, the ASO comprises 2'MOE modifications and a phosphorothioate backbone. In some cases, the ASO comprises a phosphorodiamidate morpholino (PMO). In some cases, the ASO comprises a peptide nucleic acid (PNA). Any of the ASOs or any component of an ASO *(e.g.,* a nucleobase, sugar moiety, backbone) described herein may be modified in order to achieve desired properties or activities of the ASO or reduce undesired properties or activities of the ASO. For example, an ASO or one or more component of any ASO may be modified to enhance binding affinity to a target sequence on a pre-mRNA transcript; reduce binding to any non-target sequence; reduce degradation by cellular nucleases (i.e., RNase H); improve uptake of the ASO into a cell and/or into the nucleus of a cell; alter the pharmacokinetics or pharmacodynamics of the ASO; and modulate the half-life of the ASO.

In some cases, the ASOs are comprised of 2'-O-(2-methoxyethyl) (MOE) phosphorothioate-modified nucleotides. ASOs comprised of such nucleotides are especially well-suited to the methods disclosed herein; oligomers having such modifications have been shown to have significantly enhanced resistance to nuclease degradation and increased bioavailability, making them suitable, for example, for oral delivery in some cases described herein. See *e.g.,* Geary et al., J Pharmacol Exp Ther. 2001; 296(3):890-7; Geary et al., J Pharmacol Exp Ther. 2001; 296(3):898-904.

Methods of synthesizing ASOs will be known to one of skill in the art. Alternatively or in addition, ASOs may be obtained from a commercial source.

Unless specified otherwise, the left-hand end of single-stranded nucleic acid (*e.g.,* pre-mRNA transcript, oligonucleotide, ASO, etc.) sequences is the 5' end and the left-hand direction of single or double-stranded nucleic acid sequences is referred to as the 5' direction. Similarly, the right-hand end or direction of a nucleic acid sequence (single or double stranded) is the 3' end or direction. Generally, a region or sequence that is 5' to a reference point in a nucleic acid is referred to as "upstream," and a region or sequence that is 3' to a reference point in a nucleic acid is referred to as "downstream." Generally, the 5' direction or end of an mRNA is where the initiation or start codon is located, while the 3' end or direction is where the termination codon is located. In some aspects, nucleotides that are upstream of a reference point in a nucleic acid may be designated by a negative number, while nucleotides that are downstream of a reference point may be designated by a positive number. For example, a reference point *(e.g.,* an exon-exon junction in mRNA) may be designated as the "zero" site, and a nucleotide that is directly adjacent and upstream of the reference point is designated "minus one," *e.g.,* "-1," while a nucleotide that is directly adjacent and downstream of the reference point is designated "plus one," *e.g.,* "+1."

In other cases, the ASOs are complementary to (and bind to) a targeted portion of a *PRPF3* or *PRPF8* RIC pre-mRNA that is downstream (in the 3' direction) of the 5' splice site of the retained intron in a *PRPF3* or *PRPF8* RIC pre-mRNA *(e.g.,* the direction designated by positive numbers relative to the 5' splice site) (Figure 1). In some cases, the ASOs are complementary to a targeted portion of the *PRPF3* or *PRPF8* RIC pre-mRNA that is within the region +6 to +100 relative to the 5' splice site of the retained intron. In some cases, the ASO is not complementary to nucleotides +1 to +5 relative to the 5' splice site (the first five nucleotides located downstream of the 5' splice site). In some cases, the ASOs may be complementary to a targeted portion of a *PRPF3* or *PRPF8* RIC pre-mRNA that is within the region between nucleotides +6 and +50 relative to the 5' splice site of the retained intron. In some aspects, the ASOs are complementary to a targeted portion that is within the region +6 to +90, +6 to +80, +6 to +70, +6 to +60, +6 to +50, +6 to +40, +6 to +30, or +6 to +20 relative to 5' splice site of the retained intron.

In some cases, the ASOs are complementary to a targeted region of a *PRPF3* or *PRPF8* RIC pre-mRNA that is upstream (5' relative) of the 3' splice site of the retained intron in a *PRPF3* or *PRPF8* RIC pre-mRNA *(e.g.,* in the direction designated by negative numbers) (Figure 1). In some cases, the ASOs are complementary to a targeted portion of the *PRPF3* or *PRPF8* RIC pre-mRNA that is within the region -16 to -100 relative to the 3' splice site of the retained intron. In some cases, the ASO is not complementary to nucleotides -1 to -15 relative to the 3' splice site (the first 15 nucleotides located upstream of the 3' splice site). In some cases, the ASOs are complementary to a targeted portion of the *PRPF3* or *PRPF8* RIC pre-mRNA that is within the region -16 to -50 relative to the 3' splice site of the retained intron. In some aspects, the ASOs are complementary to a targeted portion that is within the region -16 to -90, -16 to -80, -16 to -70, -16 to -60, -16 to -50, -16 to -40, or -16 to -30 relative to 3' splice site of the retained intron.

In cases, the targeted portion of the *PRPF3* or *PRPF8* RIC pre-mRNA is within the region +100 relative to the 5' splice site of the retained intron to -100 relative to the 3' splice site of the retained intron.

In some cases, the ASOs are complementary to a targeted portion of a *PRPF3* or *PRPF8* RIC pre-mRNA that is within the exon flanking the 5' splice site (upstream) of the retained intron (Figure 1). In some cases, the ASOs are complementary to a targeted portion of the *PRPF3* or *PRPF8* RIC pre-mRNA that is within the region +2e to -4e in the exon flanking the 5' splice site of the retained intron. In some cases, the ASOs are not complementary to nucleotides -1e to -3e relative to the 5' splice site of the retained intron. In some cases, the ASOs are complementary to a targeted portion of the *PRPF3* or *PRPF8* RIC pre-mRNA that is within the region -4e to -100e, -4e to -90e, -4e to -80e, -4e to -70e, -4e to -60e, -4e to -50e, -4 to -40e, - 4e to -30e, or -4e to -20e relative to the 5' splice site of the retained intron.

In some cases, the ASOs are complementary to a targeted portion of a *PRPF3* or *PRPF8* RIC pre-mRNA that is within the exon flanking the 3' splice site (downstream) of the retained intron (Figure 1). In some cases, the ASOs are complementary to a targeted portion to the *PRPF3* or *PRPF8* RIC pre-mRNA that is within the region +2e to -4e in the exon flanking the 3' splice site of the retained intron. In some cases, the ASOs are not complementary to nucleotide +1e relative to the 3' splice site of the retained intron. In some cases, the ASOs are complementary to a targeted portion of the *PRPF3* or *PRPF8* RIC pre-mRNA that is within the region+2e to +100e, +2e to +90e, +2e to +80e, +2e to +70e, +2e to +60e, +2e to +50e, +2e to +40e, +2e to +30e, or +2 to +20e relative to the 3' splice site of the retained intron. The ASOs may be of any length suitable for specific binding and effective enhancement of splicing. In some cases, the ASOs consist of 8 to 50 nucleobases. For example, the ASO may be 8, 9, 10, 11, 12, 13, 14, 15, 16, 17, 18, 19, 20, 21, 22, 23, 24, 25, 26, 27, 28, 29, 30, 31, 32, 33, 34, 35, 40, 45, or 50 nucleobases in length. In some cases, the ASOs consist of more than 50 nucleobases. In some cases, the ASO is from 8 to 50 nucleobases, 8 to 40 nucleobases, 8 to 35 nucleobases, 8 to 30 nucleobases, 8 to 25 nucleobases, 8 to 20 nucleobases, 8 to 15 nucleobases, 9 to 50 nucleobases, 9 to 40 nucleobases, 9 to 35 nucleobases, 9 to 30 nucleobases, 9 to 25 nucleobases, 9 to 20 nucleobases, 9 to 15 nucleobases, 10 to 50 nucleobases, 10 to 40 nucleobases, 10 to 35 nucleobases, 10 to 30 nucleobases, 10 to 25 nucleobases, 10 to 20 nucleobases, 10 to 15 nucleobases, 11 to 50 nucleobases, 11 to 40 nucleobases, 11 to 35 nucleobases, 11 to 30 nucleobases, 11 to 25 nucleobases, 11 to 20 nucleobases, 11 to 15 nucleobases, 12 to 50 nucleobases, 12 to 40 nucleobases, 12 to 35 nucleobases, 12 to 30 nucleobases, 12 to 25 nucleobases, 12 to 20 nucleobases, 12 to 15 nucleobases, 13 to 50 nucleobases, 13 to 40 nucleobases, 13 to 35 nucleobases, 13 to 30 nucleobases, 13 to 25 nucleobases, 13 to 20 nucleobases, 14 to 50 nucleobases, 14 to 40 nucleobases, 14 to 35 nucleobases, 14 to 30 nucleobases, 14 to 25 nucleobases, 14 to 20 nucleobases, 15 to 50 nucleobases, 15 to 40 nucleobases, 15 to 35 nucleobases, 15 to 30 nucleobases, 15 to 25 nucleobases, 15 to 20 nucleobases, 20 to 50 nucleobases, 20 to 40 nucleobases, 20 to 35 nucleobases, 20 to 30 nucleobases, 20 to 25 nucleobases, 25 to 50 nucleobases, 25 to 40 nucleobases, 25 to 35 nucleobases, or 25 to 30 nucleobases in length. In some cases, the ASOs are 18 nucleotides in length. In some cases, the ASOs are 15 nucleotides in length. In some cases, the ASOs are 25 nucleotides in length.

In some cases, two or more ASOs with different chemistries but complementary to the same targeted portion of the RIC pre-mRNA are used. In some cases, two or more ASOs that are complementary to different targeted portions of the RIC pre-mRNA are used.

In cases, the antisense oligonucleotides of the disclosure are chemically linked to one or more moieties or conjugates, e.g., a targeting moiety or other conjugate that enhances the activity or cellular uptake of the oligonucleotide. Such moieties include, but are not limited to, a lipid moiety, *e.g.,* as a cholesterol moiety, a cholesteryl moiety, an aliphatic chain, *e.g.,* dodecandiol or undecyl residues, a polyamine or a polyethylene glycol chain, or adamantane acetic acid. Oligonucleotides comprising lipophilic moieties, and preparation methods have been described in the published literature. In cases, the antisense oligonucleotide is conjugated with a moiety including, but not limited to, an abasic nucleotide, a polyether, a polyamine, a polyamide, a peptides, a carbohydrate, *e.g*., N-acetylgalactosamine (GalNAc), N-Ac-Glucosamine (GluNAc), or mannose (*e.g*., mannose-6-phosphate), a lipid, or a polyhydrocarbon compound. Conjugates can be linked to one or more of any nucleotides comprising the antisense oligonucleotide at any of several positions on the sugar, base or phosphate group, as understood in the art and described in the literature, e.g., using a linker. Linkers can include a bivalent or trivalent branched linker. In cases, the conjugate is attached to the 3' end of the antisense oligonucleotide. Methods of preparing oligonucleotide conjugates are described, *e.g*., in U.S. Pat. No. 8,450,467, "Carbohydrate conjugates as delivery agents for oligonucleotides,".

In some cases, the nucleic acid to be targeted by an ASO is a *PRPF3* or *PRPF8* RIC pre-mRNA expressed in a cell, such as a eukaryotic cell. In some cases, the term "cell" may refer to a population of cells. In some cases, the cell is in a subject. In some cases, the cell is isolated from a subject. In some cases, the cell is *ex vivo.* In some cases, the cell is a condition or disease-relevant cell or a cell line. In some cases, the cell is *in vitro* (*e.g.,* in cell culture).

### Pharmaceutical Compositions

Pharmaceutical compositions or formulations comprising the antisense oligonucleotide of the described compositions and for use in any of the described methods can be prepared according to conventional techniques well known in the pharmaceutical industry and described in the published literature. In cases, a pharmaceutical composition or formulation for treating a subject comprises an effective amount of any antisense oligomer as described above, or a pharmaceutically acceptable salt, solvate, hydrate or ester thereof, and a pharmaceutically acceptable diluent. The antisense oligomer of a pharmaceutical formulation may further comprise a pharmaceutically acceptable excipient, diluent or carrier.

Pharmaceutically acceptable salts are suitable for use in contact with the tissues of humans and lower animals without undue toxicity, irritation, allergic response, etc., and are commensurate with a reasonable benefit/risk ratio. (See, e.g., S. M. Berge, et al., J. Pharmaceutical Sciences, 66: 1-19 (1977), incorporated herein by reference for this purpose. The salts can be prepared in situ during the final isolation and purification of the compounds, or separately by reacting the free base function with a suitable organic acid. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other documented methodologies such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, loweralkyl sulfonate and aryl sulfonate.

In cases, the compositions are formulated into any of many possible dosage forms such as, but not limited to, tablets, capsules, gel capsules, liquid syrups, soft gels, suppositories, and enemas. In cases, the compositions are formulated as suspensions in aqueous, non-aqueous or mixed media. Aqueous suspensions may further contain substances that increase the viscosity of the suspension including, for example, sodium carboxymethylcellulose, sorbitol and/or dextran. The suspension may also contain stabilizers. In cases, a pharmaceutical formulation or composition of the present disclosure includes, but is not limited to, a solution, emulsion, microemulsion, foam or liposome-containing formulation (e.g., cationic or noncationic liposomes).

The pharmaceutical composition or formulation of the present disclosure may comprise one or more penetration enhancer, carrier, excipients or other active or inactive ingredients as appropriate and well known to those of skill in the art or described in the published literature. In cases, liposomes also include sterically stabilized liposomes, e.g., liposomes comprising one or more specialized lipids. These specialized lipids result in liposomes with enhanced circulation lifetimes. In cases, a sterically stabilized liposome comprises one or more glycolipids or is derivatized with one or more hydrophilic polymers, such as a polyethylene glycol (PEG) moiety. In cases, a surfactant is included in the pharmaceutical formulation or compositions. The use of surfactants in drug products, formulations and emulsions is well known in the art. In cases, the present disclosure employs a penetration enhancer to effect the efficient delivery of the antisense oligonucleotide, e.g., to aid diffusion across cell membranes and /or enhance the permeability of a lipophilic drug. In cases, the penetration enhancer is a surfactant, fatty acid, bile salt, chelating agent, or non-chelating nonsurfactant.

In cases, the pharmaceutical formulation comprises multiple antisense oligonucleotides. In cases, the antisense oligonucleotide is administered in combination with another drug or therapeutic agent. In cases, the antisense oligonucleotide is administered with one or more agents capable of promoting penetration of the subject antisense oligonucleotide across the blood-brain barrier by any method known in the art. For example, delivery of agents by administration of an adenovirus vector to motor neurons in muscle tissue is described in U.S. Pat. No. 6,632,427, "Adenoviral-vector-mediated gene transfer into medullary motor neurons," incorporated herein by reference. Delivery of vectors directly to the brain, e.g., the striatum, the thalamus, the hippocampus, or the substantia nigra, is described, e.g., in U.S. Pat. No. 6,756,523, "Adenovirus vectors for the transfer of foreign genes into cells of the central nervous system particularly in brain," incorporated herein by reference.

In cases, the antisense oligonucleotides are linked or conjugated with agents that provide desirable pharmaceutical or pharmacodynamic properties. In cases, the antisense oligonucleotide is coupled to a substance, known in the art to promote penetration or transport across the blood-brain barrier, e.g., an antibody to the transferrin receptor. In cases, the antisense oligonucleotide is linked with a viral vector, e.g., to render the antisense compound more effective or increase transport across the blood-brain barrier. In cases, osmotic blood brain barrier disruption is assisted by infusion of sugars, e.g.,, meso erythritol, xylitol, D(+) galactose, D(+) lactose, D(+) xylose, dulcitol, myo-inositol, L(-) fructose, D(-) mannitol, D(+) glucose, D(+) arabinose, D(-) arabinose, cellobiose, D(+) maltose, D(+) raffinose, L(+) rhamnose, D(+) melibiose, D(-) ribose, adonitol, D(+) arabitol, L(-) arabitol, D(+) fucose, L(-) fucose, D(-) lyxose, L(+) lyxose, and L(-) lyxose, or amino acids, e.g., glutamine, lysine, arginine, asparagine, aspartic acid, cysteine, glutamic acid, glycine, histidine, leucine, methionine, phenylalanine, proline, serine, threonine, tyrosine, valine, and taurine. Methods and materials for enhancing blood brain barrier penetration are described, e.g., in U.S. Pat. No. 4,866,042, "Method for the delivery of genetic material across the blood brain barrier," U.S. Pat. No. 6,294,520, "Material for passage through the blood-brain barrier," and U.S. Pat. No. 6,936,589, "Parenteral delivery systems," each incorporated herein by reference.

In cases, the antisense oligonucleotides of the disclosure are chemically linked to one or more moieties or conjugates, e.g., a targeting moiety or other conjugate that enhances the activity or cellular uptake of the oligonucleotide. Such moieties include, but are not limited to, a lipid moiety, e.g., as a cholesterol moiety, a cholesteryl moiety, an aliphatic chain, e.g., dodecandiol or undecyl residues, a polyamine or a polyethylene glycol chain, or adamantane acetic acid. Oligonucleotides comprising lipophilic moieties, and preparation methods have been described in the published literature. In cases, the antisense oligonucleotide is conjugated with a moiety including, but not limited to, an abasic nucleotide, a polyether, a polyamine, a polyamide, a peptides, a carbohydrate, e.g., N-acetylgalactosamine (GalNAc), N-Ac-Glucosamine (GluNAc), or mannose (e.g., mannose-6-phosphate), a lipid, or a polyhydrocarbon compound. Conjugates can be linked to one or more of any nucleotides comprising the antisense oligonucleotide at any of several positions on the sugar, base or phosphate group, as understood in the art and described in the literature, e.g., using a linker. Linkers can include a bivalent or trivalent branched linker. In cases, the conjugate is attached to the 3' end of the antisense oligonucleotide. Methods of preparing oligonucleotide conjugates are described, e.g., in U.S. Pat. No. 8,450,467, "Carbohydrate conjugates as delivery agents for oligonucleotides,".

### Treatment of Subjects

Any of the compositions provided herein may be administered to an individual. "Individual" may be used interchangeably with "subject" or "patient." An individual may be a mammal, for example a human or animal such as a non-human primate, a rodent, a rabbit, a rat, a mouse, a horse, a donkey, a goat, a cat, a dog, a cow, a pig, or a sheep. In cases, the individual is a human. In cases, the individual is a fetus, an embryo, or a child. In other cases, the individual may be another eukaryotic organism, such as a plant. In some cases, the compositions provided herein are administered to a cell *ex vivo.*

In some cases, the compositions provided herein are administered to an individual as a method of treating a disease or disorder. In some cases, the individual has a genetic disease, such as any of the diseases described herein. In some cases, the individual is at risk of having the disease, such as any of the diseases described herein. In some cases, the individual is at increased risk of having a disease or disorder caused by insufficient amount of a protein or insufficient activity of a protein. If an individual is "at an increased risk" of having a disease or disorder caused insufficient amount of a protein or insufficient activity of a protein, the method involves preventative or prophylactic treatment. For example, an individual may be at an increased risk of having such a disease or disorder because of family history of the disease. Typically, individuals at an increased risk of having such a disease or disorder benefit from prophylactic treatment (*e.g*., by preventing or delaying the onset or progression of the disease or disorder).

Suitable routes for administration of ASOs of the present disclosure may vary depending on cell type to which delivery of the ASOs is desired. The eye is the most significantly affected tissue in RP13 and RP18. An ASO of the present disclosure may therefore be administered to a subject by intravitreal injection, by subretinal injection, or by topical application to the eye. In cases, an ASO of the present disclosure administered to a subject via an implant, e.g., an encapsulated drug implanted in the eye. In cases, RPE cells treated ex vivo with an ASO of the present disclosure are implanted in the eye, e.g., in encapsulated form. In cases, therapy is administered in conjunction with existing treatments for RP, e.g., oral acetazolamide, calcium channel blockers, lutein or zeaxanthin, oral valproic acid, or an immunosuppressive agent. In cases, ASOs are administered parenterally. In cases, ASOs of the present disclosure are administered to a subject orally, by intraperitoneal injection, intramuscular injection, subcutaneous injection, or intravenous injection. In cases, a fetus is treated in utero, e.g., by administering the ASO composition to the fetus directly or indirectly (e.g., via the mother).

In cases, subjects treated using the methods and compositions are evaluated for improvement in condition using any methods known and described in the art.

### Methods of identifying additional ASOs that enhance splicing

Also within the scope of the present disclosure are methods for identifying (determining) additional ASOs that enhance splicing of a PRPF3 or PRPF8 RIC pre-mRNA, specifically at the target intron. ASOs that specifically hybridize to different nucleotides within the target region of the pre-mRNA may be screened to identify (determine) ASOs that improve the rate and/or extent of splicing of the target intron. In some cases, the ASO may block or interfere with the binding site(s) of a splicing repressor(s)/silencer. Any method known in the art may be used to identify (determine) an ASO that when hybridized to the target region of the intron results in the desired effect (*e.g*., enhanced splicing, protein or functional RNA production). These methods also can be used for identifying ASOs that enhance splicing of the retained intron by binding to a targeted region in an exon flanking the retained intron, or in a non-retained intron. An example of a method that may be used is provided below.

A round of screening, referred to as an ASO "walk" may be performed using ASOs that have been designed to hybridize to a target region of a pre-mRNA. For example, the ASOs used in the ASO walk can be tiled every 5 nucleotides from approximately 100 nucleotides upstream of the 5' splice site of the retained intron *(e.g.,* a portion of sequence of the exon located upstream of the target/retained intron) to approximately 100 nucleotides downstream of the 5' splice site of the target/retained intron and/or from approximately 100 nucleotides upstream of the 3' splice site of the retained intron to approximately 100 nucleotides downstream of the 3' splice site of the target/retained intron *(e.g.,* a portion of sequence of the exon located downstream of the target/retained intron). For example, a first ASO of 15 nucleotides in length may be designed to specifically hybridize to nucleotides +6 to +20 relative to the 5' splice site of the target/retained intron. A second ASO is designed to specifically hybridize to nucleotides +11 to +25 relative to the 5' splice site of the target/retained intron. ASOs are designed as such spanning the target region of the pre-mRNA. In cases, the ASOs can be tiled more closely, *e.g.,* every 1, 2, 3, or 4 nucleotides. Further, the ASOs can be tiled from 100 nucleotides downstream of the 5' splice site, to 100 nucleotides upstream of the 3' splice site.

One or more ASOs, or a control ASO (an ASO with a scrambled sequence, sequence that is not expected to hybridize to the target region) are delivered, for example by transfection, into a disease-relevant cell line that expresses the target pre-mRNA (e.g., the RIC pre-mRNA described elsewhere herein). The splicing-inducing effects of each of the ASOs may be assessed by any method known in the art, for example by reverse transcriptase (RT)-PCR using primers that span the splice junction, as described herein (see "Identification of intron-retention events"). A reduction or absence of the RT-PCR product produced using the primers spanning the splice junction in ASO-treated cells as compared to in control ASO-treated cells indicates that splicing of the target intron has been enhanced. In some cases, the splicing efficiency, the ratio of spliced to unspliced pre-mRNA, the rate of splicing, or the extent of splicing may be improved using the ASOs described herein. The amount of protein or functional RNA that is encoded by the target pre-mRNA can also be assessed to determine whether each ASO achieved the desired effect (*e.g*., enhanced protein production). Any method known in the art for assessing and/or quantifying protein production, such as Western blotting, flow cytometry, immunofluorescence microscopy, and ELISA, can be used.

A second round of screening, referred to as an ASO "micro-walk" may be performed using ASOs that have been designed to hybridize to a target region of a pre-mRNA. The ASOs used in the ASO micro-walk are tiled every 1 nucleotide to further refine the nucleotide acid sequence of the pre-mRNA that when hybridized with an ASO results in enhanced splicing.

Regions defined by ASOs that promote splicing of the target intron are explored in greater detail by means of an ASO "micro-walk", involving ASOs spaced in 1-nt steps, as well as longer ASOs, typically 18-25 nt.

As described for the ASO walk above, the ASO micro-walk is performed by delivering one or more ASOs, or a control ASO (an ASO with a scrambled sequence, sequence that is not expected to hybridize to the target region), for example by transfection, into a disease-relevant cell line that expresses the target pre-mRNA. The splicing-inducing effects of each of the ASOs may be assessed by any method known in the art, for example by reverse transcriptase (RT)-PCR using primers that span the splice junction, as described herein (see "Identification of intron-retention events"). A reduction or absence of the RT-PCR product produced using the primers spanning the splice junction in ASO-treated cells as compared to in control ASO-treated cells indicates that splicing of the target intron has been enhanced. In some cases, the splicing efficiency, the ratio of spliced to unspliced pre-mRNA, the rate of splicing, or the extent of splicing may be improved using the ASOs described herein. The amount of protein or functional RNA that is encoded by the target pre-mRNA can also be assessed to determine whether each ASO achieved the desired effect (*e.g*., enhanced protein production). Any method known in the art for assessing and/or quantifying protein production, such as Western blotting, flow cytometry, immunofluorescence microscopy, and ELISA, can be used.

ASOs that when hybridized to a region of a pre-mRNA result in enhanced splicing and increased protein production may be tested *in vivo* using animal models, for example transgenic mouse models in which the full-length human gene has been knocked-in or in humanized mouse models of disease. Suitable routes for administration of ASOs may vary depending on the disease and/or the cell types to which delivery of the ASOs is desired. ASOs may be administered, for example, by intravitreal injection, by subretinal injection, by topical application to the eye, or by an encapsulated drug implanted in the eye. Following administration, the cells, tissues, and/or organs of the model animals may be assessed to determine the effect of the ASO treatment by for example evaluating splicing (efficiency, rate, extent) and protein production by methods known in the art and described herein. The animal models may also be any phenotypic or behavioral indication of the disease or disease severity.

### EXAMPLES

The present disclosure will be more specifically illustrated by the following Examples.

### Example 1: Identification of intron retention events in PRPF3 transcripts by RNAseq using next generation sequencing

Whole transcriptome shotgun sequencing was carried out using next generation sequencing to reveal a snapshot of transcripts produced by the *PRPF3* gene to identify intron-retention events. For this purpose, polyA+ RNA from nuclear and cytoplasmic fractions of ARPE-19 (retinal epithelial) cells was isolated and cDNA libraries constructed using Illumina's TruSeq Stranded mRNA library Prep Kit. The libraries were pair-end sequenced resulting in 100-nucleotide reads that were mapped to the human genome (Feb. 2009, GRCh37/hg19 assembly). The sequencing results for *PRPF3* are shown in Figure 3. Briefly, Figure 3 shows the mapped reads visualized using the UCSC genome browser (operated by the UCSC Genome Informatics Group (Center for Biomolecular Science & Engineering, University of California, Santa Cruz, 1156 High Street, Santa Cruz, CA 95064) and described by, e.g., Rosenbloom, et al., 2015, "The UCSC Genome Browser database: 2015 update," Nucleic Acids Research 43, Database Issue, doi: 10.1093/nar/gku1177) and the coverage and number of reads can be inferred by the peak signals. The height of the peaks indicates the level of expression given by the density of the reads in a particular region. A schematic representation of *PRPF3* (drawn to scale) is provided by the UCSC genome browser (below the read signals) so that peaks can be matched to *PRPF3* exonic and intronic regions. Based on this display, we identified 2 introns in *PRPF3* (12 and 13, indicated by arrows, corresponding to NM_004698: intron 12, and NM_004698; intron 13, respectively) and 1 intron in *PRPF8* (31, indicated by the arrow, corresponding to NM_006445, intron 31) (Figure 7) that have high read density in the nuclear fraction of ARPE-19 cells, but have very low to no reads in the cytoplasmic fraction of these cells (as shown for intron 12 in the bottom diagram of Figure 3, 13 in the bottom diagram of Figure 5, and for introns 31 in the bottom diagram of Figure 5). This indicates that these introns are retained and that the intron-12 and intron-13, and, intron-31 containing transcripts remain in the nucleus, and suggests that these retained *PRPF3* and *PRPF8* RIC pre-mRNAs, respectively are non-productive, as they are not exported out to the cytoplasm.

### Example 2: Design of ASO-walk targeting a retained intron

An ASO walk was designed to target intron 12 of *PRPF3* using the method described herein (FIG. 4). A region immediately downstream of the intron 12 5' splice site spanning nucleotides +6 to +498 and a region immediately upstream of intron 12 3' splice site spanning nucleotides -16 to -496 of the intron were targeted with 2'-O-Me RNA, PS backbone, 18-mer ASOs shifted by 5-nucleotide intervals (with the exception of ASO P3-IVS12+28).

An ASO walk was designed to target intron 13 of *PRPF3* using the method described herein (FIG. 6). A region immediately downstream of the intron 13 5' splice site spanning nucleotides +6 to +146 and a region immediately upstream of intron 13 3' splice site spanning nucleotides -16 to -146 of the intron were targeted with 2'-O-Me RNA, PS backbone, 18-mer ASOs shifted by 5-nucleotide intervals (with the exception of ASOs P3-IVS13+15, P3-IVS13+31, and P3-IVS13-47).

An ASO walk was designed to target intron 31 of *PRPF8* using the method described herein (FIG. 8). A region immediately downstream of the intron 31 5' splice site spanning nucleotides +6 to +156 and a region immediately upstream of intron 31 3' splice site spanning nucleotides -16 to -156 were targeted with 2'-O-Me RNA, PS backbone, 18-mer ASOs shifted by 5-nucleotide intervals.

Table 1 lists exemplary ASOs that were designed and their target sequences.

**Table 1**

| Gene SEQ ID NO. | Pre-mRNA SEQ ID NO. | ASOs | Retained Intron | Target Sequence SEQ ID NO. |
|---|---|---|---|---|
| PRPF3 SEQ ID NO. 1 | PRPF3:NM_004698 SEQ ID NO. 3 | 5-239 | 12 | 447 |
| | | 240-323 | 13 | 446 |
| PRPF8 SEQ ID NO. 2 | PRPF8:NM_006445 SEQ ID NO. 4 | 324-445 | 31 | 448 |

### Example 3: Improved splicing efficiency via ASO-targeting of PRPF3 intron 12 increases transcript levels

To determine whether an increase in target gene intron splicing efficiency could be achieved with ASOs, the method described herein was used. ARPE-19 cells, a human retinal epithelium cell line (American Type Culture Collection (ATCC), USA) were mock-transfected, or transfected with the targeting ASOs described in Table 1. Cells were transfected using Lipofectamine RNAiMax transfection reagent (Thermo Fisher) according to vendor's specifications. Briefly, ASOs were plated in 96-well tissue culture plates and combined with RNAiMax diluted in Opti-MEM. Cells were detached using trypsin and resuspended in full medium, and approximately 25,000 cells were added the ASO-transfection mixture. Transfection experiments were carried out in triplicate plate replicates. Final ASO concentration was 80 nM. Media was changed 6h post-transfection, and cells harvested at 24h, using the Cells-to-Ct lysis reagent, supplemented with DNAse (Thermo Fisher), according to vendor's specifications. cDNA was generated with Cells-to-Ct RT reagents (Thermo Fisher) according to vendor's specifications. To quantify the amount of splicing at the intron of interest, quantitative PCR was carried out using Taqman assays with probes spanning the corresponding exon-exon junction (Thermo Fisher). Taqman assays were carried out according to vendor's specifications, on a QuantStudio 7 Flex Real-Time PCR system (Thermo Fisher). Target gene assay values were normalized to RPL32 (deltaCt) and plate-matched mock transfected samples (delta-delta Ct), generating fold-change over mock quantitation (2^-(delta-deltaCt). Average fold-change over mock of the three plate replicates was plotted (FIG. 10). Several ASOs were identified that increased the target gene expression, as shown in FIG. 10, implying an increase in splicing at that target intron. Together with whole transcriptome data confirming retention of the target intron (FIG. 9), these results confirm that ASOs can improve the splicing efficiency of a rate limiting intron.

### Example 4: Improved splicing efficiency via ASO-targeting of PRPF3 intron 13 increases transcript levels

To determine whether an increase in target gene intron splicing efficiency could be achieved with ASOs, the method described herein was used. ARPE-19 cells, a human retinal epithelium cell line (American Type Culture Collection (ATCC), USA) were mock-transfected, or transfected with the targeting ASOs described in Table 1. Cells were transfected using Lipofectamine RNAiMax transfection reagent (Thermo Fisher) according to vendor's specifications. Briefly, ASOs were plated in 96-well tissue culture plates and combined with RNAiMax diluted in Opti-MEM. Cells were detached using trypsin and resuspended in full medium, and approximately 25,000 cells were added the ASO-transfection mixture. Transfection experiments were carried out in triplicate plate replicates. Final ASO concentration was 80 nM. Media was changed 6h post-transfection, and cells harvested at 24h, using the Cells-to-Ct lysis reagent, supplemented with DNAse (Thermo Fisher), according to vendor's specifications. cDNA was generated with Cells-to-Ct RT reagents (Thermo Fisher) according to vendor's specifications. To quantify the amount of splicing at the intron of interest, quantitative PCR was carried out using Taqman assays with probes spanning the corresponding exon-exon junction (Thermo Fisher). Taqman assays were carried out according to vendor's specifications, on a QuantStudio 7 Flex Real-Time PCR system (Thermo Fisher). Target gene assay values were normalized to RPL32 (deltaCt) and plate-matched mock transfected samples (delta-delta Ct), generating fold-change over mock quantitation (2^-(delta-deltaCt). Average fold-change over mock of the three plate replicates was plotted (FIG. 10, FIG. 12). Several ASOs were identified that increased the target gene expression, as shown in FIG. 12, implying an increase in splicing at that target intron. Together with whole transcriptome data confirming retention of the target intron (FIG. 11), these results confirm that ASOs can improve the splicing efficiency of a rate limiting intron.

### Example 5: Improved splicing efficiency via ASO-targeting of PRPF8 intron 31 increases transcript levels

To determine whether an increase in target gene intron splicing efficiency could be achieved with ASOs, the method described herein was used. ARPE-19 cells, a human retinal epithelium cell line (American Type Culture Collection (ATCC), USA) were mock-transfected, or transfected with the targeting ASOs described in Table 1. Cells were transfected using Lipofectamine RNAiMax transfection reagent (Thermo Fisher) according to vendor's specifications. Briefly, ASOs were plated in 96-well tissue culture plates and combined with RNAiMax diluted in Opti-MEM. Cells were detached using trypsin and resuspended in full medium, and approximately 25,000 cells were added the ASO-transfection mixture. Transfection experiments were carried out in triplicate plate replicates. Final ASO concentration was 80 nM. Media was changed 6h post-transfection, and cells harvested at 24h, using the Cells-to-Ct lysis reagent, supplemented with DNAse (Thermo Fisher), according to vendor's specifications. cDNA was generated with Cells-to-Ct RT reagents (Thermo Fisher) according to vendor's specifications. To quantify the amount of splicing at the intron of interest, quantitative PCR was carried out using Taqman assays with probes spanning the corresponding exon-exon junction (Thermo Fisher). Taqman assays were carried out according to vendor's specifications, on a QuantStudio 7 Flex Real-Time PCR system (Thermo Fisher). Target gene assay values were normalized to RPL32 (deltaCt) and plate-matched mock transfected samples (delta-delta Ct), generating fold-change over mock quantitation (2^-(delta-deltaCt). Average fold-change over mock of the three plate replicates is plotted (FIG. 14). Several ASOs were identified that increased the target gene expression, as shown in FIG. 14, implying an increase in splicing at that target intron. Together with whole transcriptome data confirming retention of the target intron (FIG. 13), these results confirm that ASOs can improve the splicing efficiency of a rate limiting intron.

## Claims

1. A pharmaceutical composition comprising an antisense oligomer (ASO) or a viral vector encoding an ASO, wherein the ASO comprises a sequence selected from SEQ ID NOs: 351-353, 355, 357-360, 404, 409 and 410.

2. A pharmaceutical composition according to claim 1 for use in treating Retinitis pigmentosa 13 (RP13) in a subject.

3. A pharmaceutical composition for use in treating Retinitis pigmentosa 13 (RP13) in a subject, wherein the pharmaceutical composition comprises an ASO or a viral vector encoding an ASO, wherein the ASO is complementary to at least 8 contiguous nucleic acids of SEQ ID NO: 448, or of a sequence with at least 90% sequence identity to SEQ ID NO: 448, located within a retained-intron-containing pre-mRNA (RIC pre-mRNA) that encodes PRPF8 protein, and wherein the ASO increases the level of PRPF8 protein expression in the cells.

4. A pharmaceutical composition for use according to claim 3, wherein the targeted portion of the RIC pre-mRNA is within the region +6 relative to the 5' splice site of the retained intron to -16 relative to the 3' splice site of the retained intron.

5. A pharmaceutical composition for use according to any of claims 3 to 4, wherein the RIC pre-mRNA comprises a sequence with at least 80% sequence identity to SEQ ID NO: 4.

6. A pharmaceutical composition for use according to any of claims 3 to 5, wherein the ASO comprises a sequence selected from SEQ ID NOs: 351-353, 355, 357-360, 404, 409 and 410.

7. A pharmaceutical composition according to claim 1 or a pharmaceutical composition for use according to any of claims 2 to 6, wherein the ASO comprises a backbone modification comprising a phosphorothioate linkage or a phosphorodiamidate linkage; or a modified sugar moiety or a sugar analog, optionally comprising a phosphorodiamidate morpholino, a locked nucleic acid, a peptide nucleic acid, a 2'-O-methyl moiety, a 2'-Fluoro moiety, or a 2'-O-methoxyethyl moiety.

8. A pharmaceutical composition according to claim 1 or a pharmaceutical composition for use according to any of claims 2 to 7, wherein the ASO consists of from 8 to 50 nucleobases.

9. A pharmaceutical composition for use according to any of claims 2 to 8, wherein the PRPF8 protein is deficient in amount in the subject to be treated.

10. A pharmaceutical composition for use according to any of claims 2 to 8, wherein the PRPF8 protein is deficient in activity in the subject to be treated.

11. A pharmaceutical composition for use according to any of claims 2 to 10, wherein the PRPF8 protein is deficient in amount or activity in the subject to be treated, and the deficient amount of the PRPF8 protein is caused by haploinsufficiency of the PRPF8 protein, wherein the subject has a first allele encoding a functional PRPF8 protein, and a second allele from which the PRPF8 protein is not produced, or a second allele encoding a nonfunctional PRPF8 protein, and wherein the ASO binds to a targeted portion of a RIC pre-mRNA transcribed from the first allele.

12. A pharmaceutical composition for use according to any of claims 2 to 11, wherein the ASO is administered by intrathecal injection, intracerebroventricular injection, intraperitoneal injection, intramuscular injection, subcutaneous injection, or intravenous injection of the subject.

## Patentansprüche

1. Eine pharmazeutische Zusammensetzung umfassend ein Antisense-Oligomer (ASO) oder einen viralen Vektor, der ein ASO codiert, wobei das ASO eine Sequenz umfasst ausgewählt aus den SEQ ID NOs: 351-353, 355, 357-360, 404, 409 und 410.

2. Eine pharmazeutische Zusammensetzung nach Anspruch 1 für die Verwendung bei der Behandlung einer Retinitis pigmentosa 13 (RP13) in einem Patienten.

3. Eine pharmazeutische Zusammensetzung für die Verwendung bei der Behandlung einer Retinitis pigmentosa 13 (RP13) in einem Patienten, wobei die pharmazeutische Zusammensetzung ein ASO oder einen viralen Vektor umfasst, der ein ASO codiert, wobei das ASO komplementär zu mindestens 8 angrenzenden Nukleinsäuren der SEQ ID NO: 448 ist, oder einer Sequenz mit mindestens 90 % Sequenzidentität zu SEQ ID NO: 448, gelegen innerhalb einer beibehaltenen intronenthaltenden Pre-mRNA (RIC Pre-mRNA), die das PRPF8-Protein codiert, und wobei das ASO den Grad der PRPF8-Proteinexpression in den Zellen erhöht.

4. Eine pharmazeutische Zusammensetzung für die Verwendung nach Anspruch 3, wobei der Zielabschnitt der RIC Pre-mRNA in der Region +6 im Verhältnis zu der 5' Spleißstelle des beibehaltenen Introns bis -16 im Verhältnis zu der 3' Spleißstelle des beibehaltenen Introns liegt.

5. Eine pharmazeutische Zusammensetzung für die Verwendung nach Anspruch 3 bis 4, wobei die RIC Pre-mRNA eine Sequenz umfasst mit mindestens 80 % Sequenzidentität zu SEQ ID NO: 4.

6. Eine pharmazeutische Zusammensetzung für die Verwendung nach Anspruch 3 bis 5, wobei das ASO eine Sequenz umfasst ausgewählt aus den SEQ ID NOs: 351-353, 355, 357-360, 404, 409 und 410.

7. Eine pharmazeutische Zusammensetzung nach Anspruch 1 oder eine pharmazeutische Zusammensetzung für die Verwendung nach einem der Ansprüche 2 bis 6, wobei das ASO eine Rückgrat-Modifikation mit einer Phosphorothioatbindung oder einer Phosphordiamidatbindung umfasst; oder einen modifizierten Zuckerrest oder ein Zuckeranalog, optional umfassend ein Phosphordiamidat-Morpholin, eine verbrückte Nukleinsäure, eine Peptid-Nukleinsäure, einen 2'-O-Methyl-Rest, einen 2'-Fluor-Rest oder einen 2'-O-Methoxyethyl-Rest.

8. Eine pharmazeutische Zusammensetzung nach Anspruch 1 oder eine pharmazeutische Zusammensetzung für die Verwendung nach einem der Ansprüche 2 bis 7, wobei das ASO aus 8 bis 50 Nukleinbasen besteht.

9. Eine pharmazeutische Zusammensetzung für die Verwendung nach einem der Ansprüche 2 bis 8, wobei das PRPF8-Protein in der Menge oder der Aktivität bei dem zu behandelnden Patienten defizient ist.

10. Eine pharmazeutische Zusammensetzung für die Verwendung nach einem der Ansprüche 2 bis 8, wobei das PRPF8-Protein in der Aktivität bei dem zu behandelnden Patienten defizient ist.

11. Eine pharmazeutische Zusammensetzung für die Verwendung nach einem der Ansprüche 2 bis 10, wobei das PRPF8-Protein in der Menge oder der Aktivität bei dem zu behandelnden Patienten defizient ist und die defiziente Menge des PRPF8-Proteins durch eine Haploinsuffizienz des PRPF8-Proteins verursacht wird, wobei der Patient ein erstes Allel hat, das ein funktionales PRPF8-Protein codiert, und ein zweites Allel, aus dem das PRPF8-Protein nicht erzeugt wird, oder ein zweites Allel, das ein nichtfunktionales PRPF8-Protein codiert, und wobei sich das ASO an einen Zielabschnitt einer RIC Pre-mRNA bindet, der von dem ersten Allel transkribiert ist.

12. Eine pharmazeutische Zusammensetzung für die Verwendung nach einem der Ansprüche 2 bis 11, wobei das ASO dem Patienten durch eine intrathekale Injektion, intracerebroventrikuläre Injektion, intraperitonale Injektion, intramuskuläre Injektion, subkutane Injektion oder intravenöse Injektion verabreicht wird.

## Revendications

1. Composition pharmaceutique comprenant un oligomère antisens (ASO) ou un vecteur viral codant pour un ASO, dans laquelle l'ASO comprend une séquence sélectionnée parmi les SEQ ID n° : 351-353, 355, 357-360, 404, 409 et 410.

2. Composition pharmaceutique selon la revendication 1 destinée à être utilisée dans le traitement de la rétinite pigmentaire 13 (RP13) chez un sujet.

3. Composition pharmaceutique destinée à être utilisée dans le traitement de la rétinite pigmentaire 13 (RP13) chez un sujet, dans laquelle la composition pharmaceutique comprend un ASO ou un vecteur viral codant pour un ASO, dans laquelle l'ASO est complémentaire à au moins 8 acides nucléiques contigus de la SEQ ID n° : 448, ou d'une séquence présentant une identité de séquence d'au moins 90 % avec la SEQ ID n° : 448, située à l'intérieur d'un pré-ARNm contenant un intron retenu (pré-ARNm RIC) qui code pour la protéine PRPF8 et dans laquelle l'ASO augmente le niveau d'expression de la protéine PRPF8 dans les cellules.

4. Composition pharmaceutique destinée à être utilisée selon la revendication 3, dans laquelle la partie ciblée du pré-ARNm RIC est à l'intérieur de la région +6 par rapport au site d'épissage 5' de l'intron retenu à -16 par rapport au site d'épissage 3' de l'intron retenu.

5. Composition pharmaceutique destinée à être utilisée selon l'une quelconque des revendications 3 à 4, dans laquelle le pré-ARNm RIC comprend une séquence présentant une identité de séquence d'au moins 80 % avec la SEQ ID n° : 4.

6. Composition pharmaceutique destinée à être utilisée selon l'une quelconque des revendications 3 à 5, dans laquelle l'ASO comprend une séquence sélectionnée parmi les SEQ ID n° : 351-353, 355, 357-360, 404, 409 et 410.

7. Composition pharmaceutique selon la revendication 1 ou composition pharmaceutique destinée à être utilisée selon l'une quelconque des revendications 2 à 6, dans laquelle l'ASO comprend une modification de l'ossature comprenant une liaison phosphorothioate ou une liaison phosphorodiamidate ; ou une fraction sucre modifiée ou un analogue de sucre, comprenant éventuellement une morpholino phosphorodiamidate, un acide nucléique verrouillé, un acide nucléique peptidique, une fraction 2'-O-méthyle, une fraction 2'-fluor ou une fraction 2'-O- méthoxyéthyle.

8. Composition pharmaceutique selon la revendication 1 ou composition pharmaceutique destinée à être utilisée selon l'une quelconque des revendications 2 à 7, dans laquelle l'ASO est constituée de 8 à 50 nucléobases.

9. Composition pharmaceutique destinée à être utilisée selon l'une quelconque des revendications 2 à 8, dans laquelle la protéine PRPF8 est déficiente en quantité chez le sujet à traiter.

10. Composition pharmaceutique destinée à être utilisée selon l'une quelconque des revendications 2 à 8, dans laquelle la protéine PRPF8 est déficiente en activité chez le sujet à traiter.

11. Composition pharmaceutique destinée à être utilisée selon l'une quelconque des revendications 2 à 10, dans laquelle la protéine PRPF8 est déficiente en quantité ou en activité chez le sujet à traiter et la quantité déficiente de la protéine PRPF8 est causée par une haploinsuffisance de la protéine PRPF8, dans laquelle le sujet présente un premier allèle codant pour une protéine PRPF8 fonctionnelle et un second allèle à partir duquel la protéine PRPF8 n'est pas produite, ou un second allèle codant pour une protéine PRPF8 non fonctionnelle et dans laquelle l'ASO se lie à une partie ciblée d'un pré-ARNm RIC transcrit à partir du premier allèle.

12. Composition pharmaceutique destinée à être utilisée selon l'une quelconque des revendications 2 à 11, dans laquelle l'ASO est administré par injection intrathécale, injection intracérébroventriculaire, injection intrapéritonéale, injection intramusculaire, injection sous-cutanée ou injection intraveineuse du sujet.
